(19) Europäisches Patentamt
European Patent Office
Office européen des brevets

(11) EP 0 353 983 B1

(12) EUROPEAN PATENT SPECIFICATION

(45) Date of publication and mention of the grant of the patent: **29.05.1996 Bulletin 1996/22**

(21) Application number: **89307788.3**

(22) Date of filing: **01.08.1989**

(51) Int Cl.[6]: **C07D 471/04**, C07D 487/04, A61K 31/435, A61K 31/55 // (C07D471/04, 221:00, 209:00), (C07D487/04, 223:00, 209:00)

# (54) Lactam derivatives

Lactamderivate

Dérivés lactamiques

(84) Designated Contracting States:
**AT BE CH DE ES FR GB GR IT LI LU NL SE**

(30) Priority: **02.08.1988 GB 8818393**
**23.02.1989 GB 8904195**
**28.02.1989 GB 8904550**

(43) Date of publication of application:
**07.02.1990 Bulletin 1990/06**

(73) Proprietor: **GLAXO GROUP LIMITED**
**London W1Y 8DH (GB)**

(72) Inventors:
- **Coates, Ian Harold**
  **GB-Hertfordshire (GB)**
- **Oxford, Alexander William**
  **GB-Hertfordshire (GB)**
- **North, Peter Charles**
  **GB-Hertfordshire (GB)**
- **Price, Barry John**
  **GB-Hertfordshire (GB)**

(74) Representative: **Marchant, James Ian et al**
**Elkington and Fife**
**Prospect House**
**8 Pembroke Road**
**Sevenoaks, Kent TN13 1XR (GB)**

(56) References cited:
**EP-A- 0 306 323** **DE-A- 3 740 352**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 353 983 B1

Printed by Jouve, 75001 PARIS (FR)

## Description

This invention relates to lactam derivatives, to processes for their preparation, to pharmaceutical compositions containing them and to their medical use.

In particular the invention relates to compounds which are potent and selective antagonists of 5-hydroxytryptamine (5-HT) at 5-HT receptors of the type located on terminals of primary afferent nerves. Receptors of this type are now designated as 5-$HT_3$ receptors and are also present in the central nervous system. 5-HT occurs widely in the neuronal pathways in the central nervous system and disturbance of these 5-HT containing pathways is known to alter behavioural syndromes such as mood, psychomotor activity, appetite and memory.

Compounds having antagonist activity at 5-$HT_3$ receptors have been described previously.

Thus for example German Offenlegungsschrift No. 3740352 discloses compounds which may be represented by the general formula:

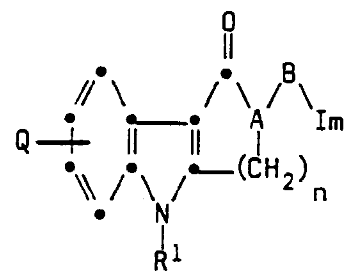

wherein Im represents an imidazolyl group of the formula:

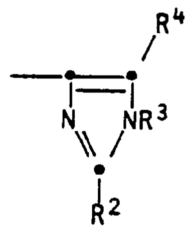

or

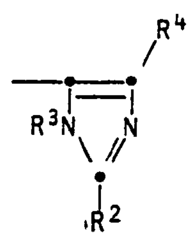

$R^1$ represents a hydrogen atom or a group selected from $C_{1-6}$alkyl, $C_{3-6}$alkenyl, $C_{3-10}$alkynyl, $C_{3-7}$cycloalkyl, $C_{3-7}$cycloalkyl$C_{1-4}$alkyl-, phenyl, phenyl$C_1$-$_3$alkyl-, $-CO_2R_5$, $-COR_5$, $-CONR_5R_6$ or $-SO_2R^5$ (wherein $R^5$ and $R^6$, which may be the same or different, each represents a hydrogen atom, a $C_{1-6}$alkyl or $C_{3-7}$cycloalkyl group, or a phenyl or phenyl$C_{1-4}$alkyl- group, in which the phenyl group is optionally substituted by one or more $C_{1-4}$alkyl, $C_{1-4}$alkoxy or hydroxy groups or halogen atoms, with the proviso that $R^5$ does not represent a hydrogen atom when $R^1$ represents a group $-CO_2R^5$ or $-SO_2R^5$);

one of the groups represented by $R^2$, $R^3$ and $R^4$ is a hydrogen atom or a $C_{1-6}$alkyl, $C_{3-7}$cycloalkyl, $C_{3-6}$alkenyl, phenyl or phenyl$C_{1-3}$alkyl-group, and each of the other two groups, which may be the same or different, represents a hydrogen atom or a $C_{1-6}$alkyl group;

Q represents a hydrogen or a halogen atom, or a hydroxy, $C_{1-4}$alkoxy, phenyl$C_{1-3}$alkoxy- or or $C_{1-6}$alkyl group or a group $-NR^7R^8$ or $-CONR^7R^8$ (wherein $R^7$ and $R^8$, which may be the same or different, each represents a hydrogen atom or a $C_{1-4}$alkyl or $C_{3-4}$alkenyl group, or together with the nitrogen atom to which they are attached form a saturated 5 to 7 membered ring);

n represents 1, 2 or 3;

and A-B represents the group $CH$-$CH_2$ or $C$=$CH$; and physiologically acceptable salts and solvates thereof.

We have now found a novel group of compounds which differ in structure from those described previously, and which are potent antagonists of the effect of 5-HT at 5-$HT_3$ receptors.

The present invention provides a tricyclic lactam of the general formula (I):

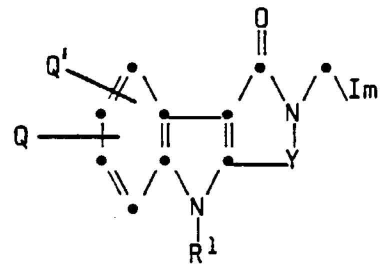 (I)

wherein Im represents an imidazolyl group of the formula:

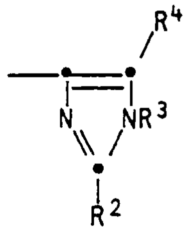 or 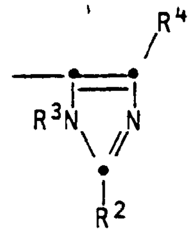

and $R^1$ represents a hydrogen atom or a group selected from $C_{1-6}$alkyl, $C_{3-6}$alkenyl, $C_{3-10}$alkynyl, $C_{3-7}$cycloalkyl, $C_{3-7}$cycloalkyl$C_{1-4}$alkyl-, phenyl, phenyl$C_{1-3}$alkyl-, $-CO_2R^5$, $-COR^5$, $-CONR^5R^6$ or $-SO_2R^5$ (wherein $R^5$ and $R^6$, which may be the same or different, each represents a hydrogen atom, a $C_{1-6}$alkyl or $C_{3-7}$cycloalkyl group, or a phenyl or phenyl$C_{1-4}$alkyl- group, in which the phenyl group is optionally substituted by one or more $C_{1-4}$alkyl, $C_{1-4}$alkoxy or hydroxy groups or halogen atoms, with the proviso that $R^5$ does not represent a hydrogen atom when $R^1$ represents a group $-CO_2R^5$ or $-SO_2R^5$);

one of the groups represented by $R^2$, $R^3$ and $R^4$ is a hydrogen atom or a $C_{1-6}$alkyl, $C_{3-7}$cycloalkyl, $C_{3-6}$alkenyl, phenyl or phenyl$C_{1-3}$alkyl-group, and each of the other two groups, which may be the same or different, represents a hydrogen atom or a $C_{1-6}$alkyl group;

Y represents the group CH=CH or $(CH_2)_n$, wherein n represents 2 or 3;

Q represents a halogen atom, or a group selected from hydroxy, $C_{1-4}$alkoxy, phenyl$C_{1-3}$alkoxy-, $C_{1-6}$alkyl, cyano, phenyl which may be unsubstituted or substituted by one or more $C_{1-4}$alkyl, $C_{1-4}$alkoxy or hydroxy groups or halogen atoms, $-NR^7R^8$, $-CONR^7R^8$ or $-(CH_2)_pCONR^7R^8$ (wherein $R^7$ and $R^8$, which may be the same or different, each represents a hydrogen atom or a $C_{1-4}$alkyl or $C_{3-4}$alkenyl group, or together with the nitrogen atom to which they are attached form a saturated 5 to 7 membered ring; and p represents 1,2 or 3), $-(CH_2)_qNR^9R^{10}$ (wherein $R^9$ represents a hydrogen atom or a $C_{1-4}$alkyl group, and $R^{10}$ represents a group $-COR^{11}$ or $-SO_2R^{11}$ wherein $R^{11}$ represents a $C_{1-4}$alkyl group; and q represents 0,1,2 or 3), or $-(CH_2)_2CO_2R^{11}$ ($R^{11}$ being as defined previously);

Q′ represents a hydrogen or a fluorine atom;

and physiologically acceptable salts and solvates thereof.

According to one aspect, the invention provides compounds of formula (I) wherein Y represents the group $(CH_2)_n$, Q′ represents a hydrogen atom, and Q represents a halogen atom or a group selected from hydroxy, $C_{1-4}$alkoxy, phenyl$C_{1-3}$alkoxy-, $C_{1-6}$alkyl, $-NR^7R^8$ or $-CONR^7R^8$ ($R^7$, $R^8$, $R^1$ and Im being as defined in formula (I)).

According to another aspect, the invention provides compounds of formula (I) wherein Y represents the group CH=CH, Q′ represents a hydrogen atom, and Q represents a halogen atom or a group selected from hydroxy, $C_{1-4}$alkoxy, phenyl$C_{1-3}$alkoxy-, $C_{1-6}$alkyl, $-NR^7R^8$ or $-CONR^7R^8$ ($R^7$, $R^8$,$R^1$ and Im being as defined in formula (I)).

According to yet another aspect, the invention provides compounds of formula (I) wherein Q′ represents a hydrogen atom and Q represents a group selected from cyano, phenyl which may be unsubstituted or substituted by one or more $C_{1-4}$alkyl, $C_{1-4}$alkoxy or hydroxy groups or halogen atoms, $-(CH_2)_pCONR^7R^8$ or$-(CH_2)_qNR^9R^{10}$(p,q, $R^7$,$R^8$,$R^9$,$R^{10}$,$R^1$, Im and Y being as defined in formula (I)).

Suitable physiologically acceptable salts of the compounds of general formula (I) include acid addition salts formed with organic or inorganic acids for example, hydrochlorides, hydrobromides, sulphates, alkyl- or arylsulphonates (e.g. methanesulphonates or p-toluenesulphonates), phosphates, acetates, citrates, succinates, tartrates, fumarates and maleates. The solvates may, for example, be hydrates.

All optical isomers of compounds of general formula (I) and their mixtures including the racemic mixtures thereof, and all the geometric isomers of compounds of formula (I), are embraced by the invention.

Referring to the general formula (I), an alkyl group may be a straight chain or branched chain alkyl group, for

example, methyl, ethyl, n-propyl, prop-2-yl, n-butyl, but-2-yl, 2-methylprop-2-yl, n-pentyl, pent-3-yl or n-hexyl. A $C_{3-6}$alkenyl group may be, for example, a propenyl or butenyl group. When $R^1$ represents a $C_{3-6}$alkenyl or $C_{3-10}$alkynyl group, or $R^3$ represents a $C_{3-6}$alkenyl group, or $R^7$ or $R^8$ represents a $C_{3-4}$alkenyl group, the double or triple bond may not be adjacent to the nitrogen atom. A phenyl$C_{1-3}$alkyl- group may be, for example, a benzyl, phenethyl or 3-phenylpropyl group. A $C_{3-7}$cycloalkyl group may be, for example, a cyclopropyl, cyclobutyl, cyclopentyl, cyclohexyl or cycloheptyl group. A $C_{1-4}$alkoxy group may be, for example, a methoxy group. A halogen atom may be, for example, a fluorine, chlorine or bromine atom.

The substituents Q and Q′ may be at the a, b, c or d-position of the indole moiety. Q is preferably at the d-position, and when Q′ is a fluorine atom, Q′ is preferably at the a-position.

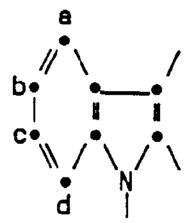

A preferred class of compounds of formula (I) is that wherein $R^1$ represents a hydrogen atom or a $C_{1-6}$alkyl (e.g. methyl or isopropyl), $C_{3-4}$alkynyl (e.g. prop-2-ynyl), $C_{4-6}$cycloalkylmethyl (e.g. cyclopentylmethyl) or $C_{1-3}$alkylsulphonyl (e.g. methylsulphonyl) group. More preferably $R^1$ represents a hydrogen atom or a $C_{1-4}$alkyl (e.g. methyl or isopropyl), $C_{3-4}$alkynyl (e.g prop-2-ynyl) or $C_{4-6}$cycloalkylmethyl (e.g. cyclopentylmethyl) group.

Another preferred class of compounds of formula (I) is that wherein $R^2$ represents a hydrogen atom or a $C_{1-4}$alkyl (e.g. methyl group, more preferably a hydrogen atom.

Another preferred class of compounds of formula (I) is that wherein $R^3$ represents a hydrogen atom or a $C_{1-4}$alkyl (e.g. methyl group, more preferably a hydrogen atom.

A further preferred class of compounds of formula (I) is that wherein $R^4$ represents a hydrogen atom or a $C_{1-4}$alkyl (e.g. methyl or n-propyl) group. More preferably $R^4$ represents a $C_{1-4}$alkyl (e.g. methyl or n-propyl) group. Most preferably $R^4$ represents a methyl group.

Another preferred class of compounds of formula (I) is that in which Q represents a halogen (e.g. fluorine or bromine) atom, or a hydroxy, $C_{1-4}$alkoxy (e.g. methoxy), phenyl$C_{1-3}$alkoxy- (e.g. phenylmethoxy), $C_{1-6}$alkyl (e.g. methyl), cyano, phenyl, $-CONH_2$ or $-(CH_2)_2CO_2CH_3$ group. More preferably Q represents a halogen (e.g. fluorine or bromine) atom, or a hydroxy, phenyl$C_{1-3}$alkoxy- (e.g. phenylmethoxy), $C_{1-3}$alkyl (e.g. methyl) or cyano group. Most preferably Q represents a fluorine atom.

A further preferred class of compound of formula (I) is that in which Q′ represents a hydrogen atom. When Q′ represents a fluorine atom, Q preferably represents a halogen (e.g. fluorine) atom.

Yet another preferred class of compounds of formula (I) is that in which Y represents the group $(CH_2)_2$.

A preferred group of compounds of formula (I) is that wherein $R^1$ represents a hydrogen atom or a $C_{1-6}$alkyl, $C_{3-4}$alkynyl, $C_{4-6}$cycloalkylmethyl or $C_{1-3}$alkylsulphonyl group; $R^2$ represents a hydrogen atom; $R^3$ represents a hydrogen atom or a $C_{1-4}$alkyl group; $R^4$ represents a $C_{1-4}$alkyl group; and Q represents a halogen atom or a hydroxy, $C_{1-4}$alkoxy, phenyl$C_{1-3}$alkoxy-, $C_{1-6}$alkyl, cyano, phenyl, $-CONH_2$ or $-(CH_2)_2CO_2CH_3$ group.

A particularly preferred group of compounds of formula (I) is that wherein $R^1$ represents a hydrogen atom or a $C_{1-4}$alkyl (e.g. methyl or isopropyl), $C_{3-4}$alkynyl (e.g. prop-2-ynyl) or $C_{4-6}$cycloalkylmethyl (e.g. cyclopentylmethyl) group; $R^2$ and $R^3$ each represent a hydrogen atom; $R^4$ represents a $C_{1-4}$alkyl (e.g. methyl or n-propyl) group; and Q represents a halogen (e.g. fluorine or bromine) atom or a hydroxy, phenyl$C_{1-3}$alkoxy- (e.g. phenylmethoxy), $C_{1-3}$alkyl (e.g. methyl) or cyano group.

Within the above preferred and particularly preferred groups of compounds, an especially important group of compounds is that in which Y represents the group $(CH_2)_2$ and Q′ is a hydrogen atom.

Preferred compounds according to the invention are:

6-fluoro-2,3,4,5-tetrahydro-5-methyl-2-[(5-methyl-1H-imidazol-4-yl)methyl]-1H-pyrido[4,3-b]indol-1-one;
2,3,4,5-tetrahydro-5,6-dimethyl-2-[(5-methyl-1H-imidazol-4-yl)methyl]-1H-pyrido[4,3-b]indol-1-one;
6,9-difluoro-2,3,4,5-tetrahydro-5-methyl-2-[(5-methyl-1H-imidazol-4-yl)methyl]-1H-pyrido[4,3-b]indol-1-one;
6-fluoro-2,3,4,5-tetrahydro-2-[(5-methyl-1H-imidazol-4-yl)methyl]-5-(2-propynyl)-1H-pyrido[4,3-b]indol-1-one;
2,3,4,5-tetrahydro-5-methyl-2-[(5-methyl-1H-imidazol-4-yl)methyl]-1-oxo-1H-pyrido[4,3-b]indole-6-carbonitrile;

and their physiologically acceptable salts and solvates.

The potent and selective antagonism of 5-HT at 5-$HT_3$ receptors by compounds of the invention has been demonstrated by their ability to inhibit 3-(5-methyl-1H-imidazol-4-yl)-1-[1-(methyl-$t_3$)-1H-indol-3-yl]-1-propanone binding in

rat entorhinal cortex homogenates (following the general procedure described by G. Kilpatrick et al. in Nature, 1987, 330, 746), and/or by their ability to inhibit the 5-HT-induced depolarisation of the rat isolated vagus nerve preparation.

In addition to their activity as potent and selective antagonists of 5-HT at 5-$HT_3$ receptors, certain compounds according to the invention have the advantage of an extended duration of action.

A particularly preferred compound on account of both its potency and duration of action is 6-fluoro-2,3,4,5-tetrahydro-5-methyl-2-[(5-methyl-1H-imidazol-4-yl)methyl]-1H-pyrido[4,3-b]indol-1-one and its physiologically acceptable salts and solvates. Preferred salts of this compound are the hydrochloride, maleate and benzoate.

Compounds of formula (I), which antagonise the effect of 5-HT at 5-$HT_3$ receptors, are useful in the treatment of conditions such as psychotic disorders (e.g. schizophrenia and mania); anxiety; and nausea and vomiting, particularly that associated with cancer chemotherapy and radiotherapy. Compounds of formula (I) are also useful in the treatment of gastric stasis; symptoms of gastrointestinal dysfunction such as occur with dyspepsia, peptic ulcer, reflux oesophagitis, flatulence and irritable bowel syndrome; migraine; obesity and conditions such as bulimia; and pain. Compounds of formula (I) may also be used in the treatment of dependency on drugs and substances of abuse, depression, and dementia and other cognitive disorders.

According to another aspect, the invention provides a method of treatment of a human or animal subject suffering from a psychotic disorder such as schizophrenia or mania; or from anxiety; nausea or vomiting; gastric stasis; symptoms of gastrointestinal dysfunction such as dyspepsia, reflux oesophagitis, peptic ulcer, flatulence and irritable bowel syndrome; migraine; obesity and conditions such as bulimia; pain; dependency on drugs or substances of abuse; depression; or dementia or another cognitive disorder which comprises administering an effective amount of a compound of formula (I) or a physiologically acceptable salt or solvate thereof.

Accordingly, the invention also provides a pharmaceutical composition which comprises at least one compound selected from compounds of the general formula (I), and their physiologically acceptable salts and solvates (e.g. hydrates), for use in human or verterinary medicine, and formulated for administration by any convenient route.

Such compositions may be formulated in conventional manner using one or more physiologically acceptable carriers and/or excipients.

Thus the compounds according to the invention may be formulated for oral, buccal, parenteral or rectal administration or in a form suitable for administration by inhalation or insufflation (either through the mouth or nose).

For oral administration, the pharmaceutical compositions may take the form of, for example, tablets or capsules prepared by conventional means with pharmaceutically acceptable excipients such as binding agents (e.g. pregelatinised maize starch, polyvinylpyrrolidone or hydroxylpropyl methylcellulose); fillers (e.g. lactose, microcrystalline cellulose or calcium hydrogen phosphate); lubricants (e.g. magnesium stearate, talc or silica); disintegrants (e.g. potato starch or sodium starch glycollate); or wetting agents (e.g. sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g. sorbitol syrup, cellulose derivatives or hydrogenated edible fats); emulsifying agents (e.g. lecithin or aracia); non-aqueous vehicles (e.g. almond oil, oily esters, ethyl alcohol fractionated vegetable oils); and preservatives (e.g. methyl or propyl-p-hydroxybenzoates or sorbic acid). The preparations may also contain buffer salts, flavouring, colouring and sweetening agents as appropriate.

Preparations for oral administration may be suitable formulated to give controlled release of the active compound.

For buccal administration the compositions may take the form of tablets or lozenges formulated in conventional manner.

The compounds of the invention may be formulated for parenteral administration by bolus injection or continuous infusion. Formulations for injection may be presented in unit dosage form e.g. in ampoules or in multi-dose containers, with an added preservative. The compositions may take such forms as suspensions, solutions or emulsions in oily or aqueous vehicles, and may contain formulatory agents such as suspending, stabilising and/or dispersing agents. Alternatively, the active ingredient may be in powder form for constitution with a suitable vehicle, e.g. sterile pyrogen-free water, before use.

The compounds of the invention may also be formulated in rectal compositions such as suppositories or retention enemas, e.g. containing conventional suppository bases such as cocoa butter or other glycerides.

In addition to the formulations described previously, the compounds of the invention may also be formulated as depot preparations. Such long acting formulations may be administered by implantation (for example subcutaneously or intramuscularly) or by intramuscular injection. Thus, for example, the compounds of the invention may be formulated with suitable polymeric or hydrophobic materials (for example as an emulsion in an acceptable oil) or ion exchange resins, or as sparingly soluble derivatives, for example, as a sparingly soluble salt.

For administration by inhalation the compounds according to the invention are conveniently delivered in the form of an aerosol spray presentation from pressurised packs or a nebuliser, with the use of a suitable propellant, e.g. dichlorodifluoromethane, trichlorofluoromethane, dichlorotetrafluoroethane, carbon dioxide or other suitable gas. In

the case of a pressurised aerosol the dosage unit may be determined by providing a valve to deliver a metered amount. Capsules and cartridges of e.g. gelatin for use in an inhaler or insufflator may be formulated containing a powder mix of a compound of the invention and a suitable powder base such as lactose or starch.

For intranasal administration, the compounds according to the invention may be formulated as solutions for administration via a suitable metered or unit dose device or alternatively as a powder mix with a suitable carrier for administration using a suitable delivery device.

The compounds of formula (I) may also be administered in combination with other therapeutic agents. Thus, for example, in the treatment of gastric stasis, symptoms of gastrointestinal dysfunction and nausea and vomiting, the compounds of formula (I) may be administered in combination with antisecretory agents such as histamine $H_2$-receptor antagonists (e.g. ranitidine, sufotidine, cimetidine, famotidine, nizatidine or roxatidine) or $H^+K^+$ATPase inhibitors (e.g. omeprazole). In the treatment of nausea and vomiting, compounds of formula (I) may also be administered in combination with dexamethasone or a cyclo-oxygenase inhibitor such as piroxicam.

A proposed dose of the compounds of the invention for administration to man (of approximately 70kg body weight) is 0.001 to 100mg, preferably 0.01 to 50mg, more preferably 0.1 to 20mg of the active ingredient per unit dose expressed as the weight of free base, which could be administered, for example, 1 to 4 times per day. It will be appreciated that it may be necessary to make routine variations to the dosage, depending on the age and condition of the patient. The dosage will also depend on the route of administration.

Compounds of general formula (I) and physiologically acceptable salts or solvates thereof may be prepared by the general methods outlined hereinafter. In the following description, the groups $R^1$, Y, Q, Q′ and Im are as defined for compounds of general formula (I) unless otherwise stated.

According to a first general process (A), a compound of general formula (I) may be prepared by alkylating a compound of formula (II):

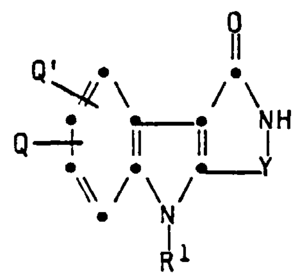

(II)

with a compound of formula (III):

X-Im (III)

or a protected derivative thereof, wherein X represents a group $-CH_2L$ and L represents a leaving atom or group, such as a halogen atom (e.g. chlorine, bromine or iodine), or an acyloxy group (e.g. trifluoroacetyloxy or acetoxy), or a sulphonyloxy group (e.g. trifluoromethanesulphonyloxy, p-toluenesulphonyloxy or methanesulphonyloxy) and the reaction is effected in the presence of a base; or X represents the group $-CH_2OH$ and the reaction is effected in the presence of an acid at an elevated temperature, followed where necessary by removal of any protecting groups.

According to one embodiment (a) of process (A), the reaction is effected with a compound of formula (III) wherein X represents the group $-CH_2L$, and L is preferably a halogen (e.g. chlorine) atom. The reaction may be carried out in an inert solvent such as an ether (e.g. dimethoxyethane, diglyme or tetrahydrofuran), a substituted amide (e.g. dimethylformamide or N-methylpyrrolidone), an aromatic hydrocarbon (e.g. toluene), a ketone (e.g. acetone), of dimethyl sulphoxide, at a temperature between ambient and 100°C, in the presence of a base. Suitable bases include alkali metal hydrides (e.g. sodium hydride), alkali metal carbonates (e.g. sodium carbonate), alkali metal amides (e.g. sodium amide), alkali metal alkoxides (e.g. potassium t-butoxide) or alkali metal hydroxides (e.g. sodium or potassium hydroxide).

According to another embodiment (b) of process (A), the reaction is effected with a compound of formula (III) wherein X represents the group $-CH_2OH$, in the presence of an acid. The acid may be, for example, a strong mineral acid (e.g. hydrochloric acid) or a hydrocarbylsulphonic acid (e.g. p-toluenesulphonic acid). The reaction may conveniently be effected in a high boiling polar solvent such as N-methylpyrrolidinone or dimethylacetamide, at an elevated temperature, for example in the range 100 to 200°C. Alternatively the reaction may be conveniently effected in water or an alcohol (e.g. isopropanol) at the reflux temperature of the solvent.

According to another general process (B), a compound of general formula (I) wherein Y represents the group $(CH_2)_n$ may be prepared by cyclising a compound of formula (IV):

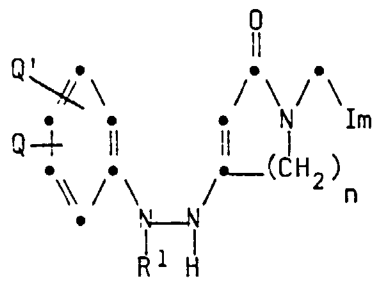

(IV)

or a salt or protected derivative thereof, followed where necessary by removal of any protecting groups.

It will be appreciated that these compounds of formula (IV) may exist in the corresponding enol hydrazone tautomeric form. The cyclisation may be carried out in aqueous or non-aqueous media, in the presence of an acid catalyst. When an aqueous medium is employed this may be water or a mixture of water and an organic solvent such as an alcohol (e.g. methanol, ethanol or isopropanol) or an ether (e.g. dioxan or tetrahydrofuran). The acid catalyst may be, for example, an inorganic acid such as concentrated hydrochloric or sulphuric acid. In some cases the acid catalyst may also act as the reaction solvent. In an anhydrous reaction medium, which may comprise one or more alcohols or ethers (e.g. as described above), carboxylic acids (e.g. acetic acid) or esters (e.g. ethyl acetate), the acid catalyst may alternatively be a Lewis acid such as boron trifluoride, zinc chloride or magnesium chloride. The cyclisation reaction may conveniently be carried out at temperatures of from 20 to 200°C, preferably 20 to 125°C.

Alternatively the cyclisation may be carried out in the presence of polyphosphate ester in a reaction medium which may comprise one or more organic solvents, preferably halogenated hydrocarbons such as chloroform, dichloromethane, dichloroethane, dichlorodifluromethane, or mixtures thereof. Polyphosphate ester is a mixture of esters which may be prepared from phosphorus pentoxide, diethyl ether and chloroform according to the method described in 'Reagents for Organic Synthesis', (Fieser and Fieser, John Wiley and Sons, 1967).

According to another general process (C), a compound of general formula (I) wherein $R^3$ represents a hydrogen atom, may be prepared by the reaction of a compound of formula (V):

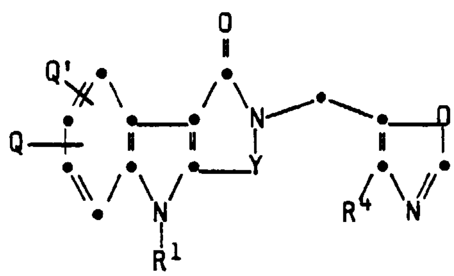

(V)

or a protected derivative thereof, with formamide, at a temperature in the range of 150 to 200°C, followed where necessary by removal of any protecting groups.

According to another general process (D), a compound of general formula (I) wherein Y represent the group $(CH_2)_n$ may be prepared by reacting a compound of formula (VI):

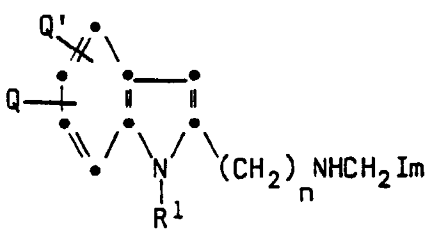

(VI)

or a protected derivative thereof, with phosgene in the presence of a Lewis acid, followed where necessary by removal of any protecting groups.

The Lewis acid may be, for example, anhydrous aluminium trichloride or stannic chloride. The reaction may conveniently be effected in an inert solvent such as an aromatic hydrocarbon (e.g. toluene) or a halogenated hydrocarbon (e.g. dichloromethane), or mixtures thereof, and at a temperature between ambient and 100°C.

According to another general process (E), a compound of general formula (I) wherein Y represents the group $(CH_2)_n$ may be prepared by oxidising a compound of formula (VII):

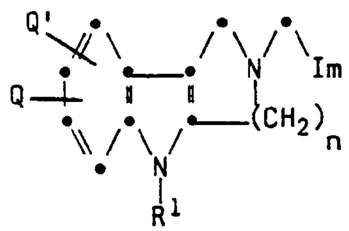

(VII)

or a protected derivative thereof, followed where necessary by removal of any protecting groups.

The oxidation may be effected using conventional methods using an oxidising agent suitable for the conversion of the group $CH_2$ to the group C=O. Suitable oxidising agents include quinones (e.g. 2,3-dichloro-5,6-dicyano-1,4-benzoquinone), potassium permanganate in acetone, mercuric acetate and acetic acid, ruthenium tetraoxide or chromium trioxide in concentrated sulphuric acid.

Suitable solvents include ethers (e.g. tetrahydrofuran or dioxan), ketones (e.g. acetone), chlorinated hydrocarbons (e.g. chloroform), and water, or mixtures thereof. The process is conveniently effected at a temperature of -70° to +50°. It will be understood that the choice of oxidising agent will affect the preferred reaction temperature

According to another general process (F), a compound of general formula (I) may be converted into another compound of formula (I) using conventional techniques. Such conventional techniques include hydrogenation, alkylation and acylation using protection and deprotection where necessary.

Thus, according to one embodiment of the interconversion process (F), a compound of formula (I) wherein Y represents the group $(CH_2)_2$ may be prepared by hydrogenating the corresponding compound of formula (I) in which Y presents the group CH=CH. Hydrogenation may also be used to prepared a compound of formula (I) in which Q represents a hydroxyl group from the corresponding compound of formula (I) in which Q represents a phenylmethoxy group. Hydrogenation may also be used to convert an alkenyl or an alkynyl substituent into an alkyl substituent, or an alkynyl into an alkenyl substituent. Hydrogenation according to general process (F) may be effected using conventional procedures, for example, using hydrogen in the presence of a noble metal catalyst (e.g. palladium, Raney nickel, platinum or rhodium). The catalyst may be supported on, for example, charcoal or alumina, or alternatively a homogeneous catalyst such as tris(triphenylphosphine)rhodium chloride may be used. The hydrogenation will generally be effected in a solvent such as an alcohol (e.g. methanol or ethanol), an ether (e.g. dioxan), or an ester (e.g. ethyl acetate), or in a mixture of an alcohol and either a hydrocarbon (e.g. toluene) or a halogenated hydrocarbon (e.g. dichloromethane), at a temperature in the range -20 to +100°C, and at a pressure of from 1 to 10 atmospheres.

The term 'alkylation' according to general process (F) includes the introduction of groups such as cycloalkyl, alkenyl or phenalkyl groups.

Thus, for example, a compound of formula (I) in which $R^1$ represents a $C_{1-6}$alkyl, $C_{3-6}$alkenyl, $C_{3-10}$alkynyl, $C_{3-7}$cycloalkyl, $C_{3-7}$cycloalkyl$C_{1-4}$alkyl or phenyl$C_{1-3}$alkyl group may be prepared by alkylating a compound of formula (I) in which $R^1$ represents a hydrogen atom, or a compound in which $R^3$ represents a $C_{1-6}$alkyl, $C_{3-7}$cycloalkyl, $C_{3-6}$alkenyl or phenyl$C_{1-3}$alkyl group may be prepared by alkylating the corresponding compound of formula (I) in which $R^3$ represents a hydrogen atom, using conventional procedures, for example as described in published European Patent Specification No. 242973. Thus the reactions may be effected using an appropriate alkylating agent of formula $R^{12}Z$ (where $R^{12}$ is the group to be introduced and Z is a leaving atom or group), preferably in the presence of a base.

According to another embodiment of general process (F), a compound of formula (I) wherein $R^1$ represents -$CO_2R^5$, -$COR^5$, -$CONR^5R^6$ or -$SO_2R^5$ may be prepared by acylating or sulphonylating as appropriate, a compound of formula (I) wherein $R^1$ represents a hydrogen atom. The acylation/sulphonylation reactions may be effected using an appropriate acylating/sulphonylating agent according to conventional procedures, for example, as decribed in published European Patent Specification No. 210840.

Compounds of formula (I) may also be prepared from other compounds of formula (I) by conventional functional group interconversion reactions, or by a series of such reactions.

Thus, for example, a compound of formula (I) wherein Q represents the group -$CONH_2$ may be prepared by reacting a compound of formula (I) in which Q represents a cyano group with a hydrolysing reagent suitable for the conversion of a cyano to an amido group. Suitable reagents include the hydroxide form of an anion exchange resin (e.g. Amberlite IRA 400 (OH)) and suitable solvents for this reaction include ethanol and water.

A compound of formula (I) wherein Q represents a group -$(CH_2)_2CO_2R^{11}$ may be prepared from a compound of formula (I) wherein Q represents a bromine atom, by reaction with a compound of formula $H_2C{=}CHCO_2H$ in the presence of an "arylpalladium" reagent which may be generated in situ, for example, by treating palladium acetate with tri-o-tolylphosphine in the presence of a base such as triethylamine. The reaction may conveniently be effected in a solvent such as acetonitrile and at an elevated temperature. The resulting carboxylic acid may be esterified using conventional methods, for example by reacting with the appropriate alcohol (e.g. methanol) and concentrated hydrochloric acid at an elevated temperature, and the propenoae so formed is then hydrogenated, for example, using hydrogen in the presence of a catalyst.

If required, a compound of formula (I) wherein Q represents a group $-(CH_2)_2CONR^7R^8$ may be prepared by reaction of a compound of formula (I) wherein Q represents a group $-(CH_2)_2CO_2R^{11}$ with the appropriate amine, $R^7R^8NH$.

It should be appreciated that in the above transformations it may be necessary or desirable to protect any sensitive groups in the molecule of the compound in question to avoid undesirable side reactions. For example, it may be necessary to protect the indole and/or imidazole nitrogen atoms, for example with an arylmethyl (e.g. trityl), arylmethoxymethyl (e.g. phenylmethoxymethyl), alkyl (e.g. t-butyl), alkoxymethyl (e.g. methoxymethyl), acyl (e.g. benzyloxycarbonyl) or a sulphonyl (e.g. N,N-dimethylaminosulphonyl or p-toluenesulphonyl) group. When Q represents a hydroxyl group it may be necessary to protect the hydroxyl group, for example with an arylmethyl (e.g. benzyl or trityl) group.

Thus according to another general process (G), a compound of general formula (I) may be prepared by the removal of any protecting groups from a protected form of a compound of formula (I). Deprotection may be effected using conventional techniques such as those described in 'Protective Groups in Organic Synthesis' by T. W. Greene (John Wiley and Sons, 1981).

For example, an arylmethoxymethyl N-protecting group may be cleaved by hydrogenolysis in the presence of a catalyst (e.g. palladium on charcoal). A trityl group may be cleaved by acid hydrolysis (e.g. using dilute hydrochloric or acetic acid). An alkoxyalkyl group may be removed using a mineral acid (e.g. dilute hydrochloric or hydrobromic acid). An acyl group may be removed by hydrolysis under acidic or basic conditions (e.g. using hydrogen bromide, dilute hydrochloric acid or sodium hydroxide). A sulphonyl group may also be removed by alkaline or acidic hydrolysis, and an N, N-dimethylaminosulphonyl group may also be removed (e.g. from an imidazole nitrogen atom) by photolysis. An arylmethyl OH-protecting group may be cleaved under acidic conditions (e.g. with dilute acetic acid, hydrobromic acid or boron tribromide) or by hydrogenolysis in the presence of a catalyst (e.g. palladium on charcoal).

Compounds of formula (II) wherein Y represents the group $(CH_2)_n$ and $R^1$ represents a hydrogen atom may be prepared, for example, by the cyclisation of a compound of formula (VIII):

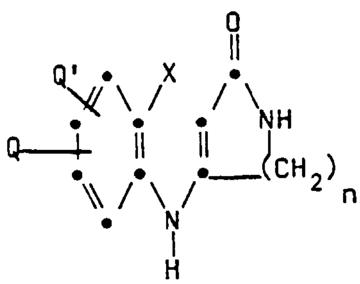

(VIII)

wherein X represents a hydrogen or a halogen (e.g. bromine or iodine) atom. The cyclisation may be effected using methods analogous to that described by H. Iida et al. in J. Org. Chem., 1980,45,2938.

Compounds of formula (II) wherein Y represents the group $(CH_2)_n$ and $R^1$ represents a group other than a hydrogen atom may be prepared from a compound of formula (II) wherein $R^1$ represents a hydrogen atom by conventional alkylation, acylation and sulphonylation processes, as described, for example, in the interconversion process (F) above.

Compounds of formula (II) wherein Y represents the group CH=CH may be prepared by heating a compound of formula (II) wherein Y represents the group $(CH_2)_2$, or a protected derivative thereof, with a noble metal catalyst such as palladium, palladium oxide, platinum or nickel, at a temperature of , for example, 200 to 350°C. The catalyst may be supported on, for example, charcoal or alumina, and the reaction may optionally be carried out in the presence of an inert solvent such as an aromatic hydrocarbon (e.g. p-cymene) or ethylene glycol.

Compounds of formula (VIII) may be prepared, for example, by the reaction of a compound of formula (IX):

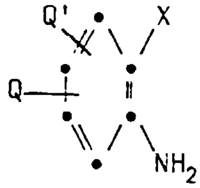

(IX)

wherein X is as defined above, with a compound of formula (X):

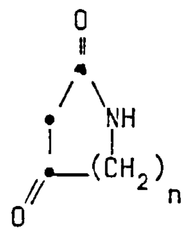

(X)

at an elevated temperature.

Compounds of formula (III) and protected derivatives thereof are either known or may be prepared, for example, by methods analogous to that described in published European Patent Specification No. 242973.

Compounds of formulae (IV), (V) and (VI) may be prepared for example, by methods analogous to those described in published European Patent Specification No.306323A.

Compounds of formula (VII) may be prepared, for example, by the reaction of a compound of formula (XI):

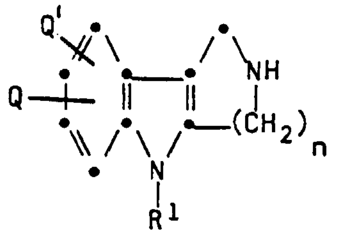

(XI)

with a compound of formula (III), using the conditions described in embodiment (a) of process (A).

Compounds of formula (VII) may also be prepared by the reduction of a compound of formula (I), using a reducing agent suitable for the conversion of the group C=O to the group $CH_2$, such as lithium aluminium hydride. The reaction may conveniently be effected in an inert solvent such as an ether (e.g. tetrahydrofuran) and at an elevated temperature.

Compounds of formulae (IX), (X) and (XI) are either known, or may be prepared from known compounds by conventional procedures.

Where it is desired to isolate a compound of the invention as a salt, for example a physiologically acceptable salt, this may be achieved by reacting the compound of formula (I) in the form of the free base with an appropriate acid, preferably with an equivalent amount, in a suitable solvent such as an alcohol (e.g. ethanol or methanol), an aqueous alcohol (e.g. aqueous ethanol), a halogenated hydrocarbon (e.g. dichloromethane), an ester (e.g. ethyl acetate) or an ether (e.g. tetrahydrofuran).

Physiologically acceptable salts may also be prepared from other salts, including other physiologically acceptable salts, of the compound of formula (I) using conventional methods.

Individual enantiomers of the compounds of the invention may be obtained by resolution of a mixture of enantiomers (e.g. a racemic mixture) using conventional means, such as an optically active resolving acid; see for example 'Stereochemistry of Carbon Compounds' by E.L.Eliel (McGraw Hill, 1962) and 'Tables of Resolving Agents' by S. H. Wilen.

The methods described above for preparing the compounds of the invention may be used for the introduction of the desired groups at any stage in the stepwise formation of the required compounds, and it will be appreciated that these methods can be combined in different ways in such multi-stage processes. The sequence of the reactions in multi-stage processes should of course be chosen so that the reaction conditions used do not affect groups in the molecule which are desired in the final product.

The invention is further illustrated by the following Intermediates and Examples. All temperatures are in °C. Thin layer chromatography (t.l.c.) was carried out on silica, and flash column chromatography (FCC) on silica (Merck 9385). Solvent System A as used for chromatography denotes dichloromethane:ethanol:0.88 ammonia solution. Organic extracts were dried, where indicated, over magnesium sulphate or sodium sulphate. The following abbreviations are used: DMF - dimethylformamide; THF - tetrahydrofuran; DME - dimethoxyethane. $^1$H-N.m.r. spectra were obtained at 250MHz for dilute solutions in $d_6$-dimethyl sulphoxide. The chloride form of Amberlite resin IRA 400 was obtained from B.D.H.

Intermediate 1

4-[(2-Fluorophenyl)amino]-5,6-dihydro-2(1H)-pyridinone

A mixture of 2-fluoroaniline (0.98g) and 2,4-dioxopiperidine (1.0g) was heated at 120° for 2h. The cooled mixture

was triturated with dry ether (5x40mℓ) and the solvent was decanted to leave the title compound (1.495g), m.p. 98-100°.

Intermediate 2

4-[(4-Fluorophenyl)amino]-5,6-dihydro-2(1H)-pyridinone

A mixture of 4-fluoroaniline (978mg) and 2,4-dioxopiperidine (1.0g) was heated at 120° for 1h, and then cooled. The solid was triturated with ether (30mℓ) and the solvent was decanted to leave the title compound (1.71g), m.p. 195-198°.

Intermediate 3

5,6-Dihydro-4-[(2-methylphenyl)amino]-2(1H)-pyridinone

A mixture of o-toluidine (943mg) and 2,4-dioxopiperidine (1.0g) was heated at 120° for 30 min. The oil was cooled, triturated with ether (30mℓ) and the solvent was decanted to give the title compound (1.74g), m.p. 155-158°.

Intermediate 4

4-[(2,6-Dibromophenyl)amino]-5,6-dihydro-2(1H)-pyridinone

A mixture of 2,6-dibromoaniline (4.4g) and 2,4-dioxopiperidine (2.0g) was heated at 120° for 2.5h. The mixture was then cooled (0°) and triturated with ether (100mℓ) to give a solid which was purified by FCC eluting with System A (100:8:1) to give the title compound (1.8g), m.p. 240-243°.

Intermediate 5

4-[(3-Fluorophenyl)amino]-5,6-dihydro-2(1H)-pyridinone

A mixture of 3-fluoroaniline (2g) and 2,4-dioxopiperidine (2.04g) was heated at 120° under nitrogen for 1h. The resultant solid was cooled and purified by FCC eluting with System A (100:8:1) to give the title compound (2.49g), m. p. 188-190°.

Intermediate 6

4-[(2,5-Difluorophenyl)amino]-5,6-dihydro-2(1H)-pyridinone

A mixture of 2,5-difluoroaniline (6.45g) and 2,4-dioxopiperidine (5.66g) was heated under nitrogen for 6h. The reaction mixture was then dissolved in ethanol (50mℓ), adsorbed onto silica, and purified by FCC eluting with System A (150:8:1) to give the title compound (2.3g), m.p. 252-255°.

Intermediate 7

3-[(2-Fluorophenyl)amino]-2-cyclohexen-1-one

2-Fluoroaniline (10g) and cyclohexane-1,3-dione (10g) were heated together under nitrogen at 120° for 1h. The cooled mixture was triturated with ether and filtered to give the title compound (14.8g), m.p. 116-118°.

Intermediate 8

6-Fluoro-2,3,4,5-tetrahydro-1H-pyrido[4,3-b]indol-1-one

Cupric acetate (2.71g) was added to a stirred solution of 4-[(2-fluorophenyl)amino]-5,6-dihydro-2(1H)-pyridinone (1.4g) and palladium (II) acetate (280mg) in dry DMF (28mℓ) under nitrogen. The mixture was heated at 130° for 1.5h and the solvent was removed in vacuo. The residue was treated with hot methanol (50mℓ) and the suspension was filtered and washed with hot methanol (3x50mℓ). The combined filtrates were evaporated to give a gum (2.16g) which was purified by FCC eluting with System A (200:10:1) to give the title compound (490mg), m.p. 255-257°.

Intermediate 9

8-Fluoro-2,3,4,5-tetrahydro-1H-pyrido[4,3-b]indol-1-one

Cupric acetate (2.9g) was added to a stirred solution of 4-[(4-fluorophenyl)amino]-5,6-dihydro-2(1H)-pyridinone (1.5g) and palladium (II) acetate (200mg) in dry DMF (40mℓ). The mixture was heated at 130° for 1h, evaporated in vacuo and the residue was extracted with methanol (250mℓ). This solution was concentrated in vacuo and the residue was purified by FCC eluting with System A (100:8:1) to give the title compound (840mg), m.p. 242-245°.

Intermediate 10

2,3,4,5-Tetrahydro-6-methyl-1H-pyrido[4,3-b]indol-1-one

5,6-Dihydro-4-[(2-methylphenyl)amino]-2(1H)-pyridinone (1.5g) was cyclised according to the method of Intermediate 9 to give the title compound (360mg), m.p. 300-302°.

Intermediate 11

6-Bromo-2,3,4,5-tetrahydro-1H-pyrido[4,3-b]indol-1-one

4-[(2,6-Dibromophenyl)amino]-5,6-dihydro-2(1H)-pyridinone (0.5g) was cyclised according to the method of Intermediate 9 to give the title compound (250mg), m.p. 268-270°.

Intermediate 12

7-Fluoro-2,3,4,5-tetrahydro-1H-pyrido[4,3-b]indol-1-one

4-[(3-Fluorophenyl)amino]-5,6-dihydro-2(1H)-pyridinone (2.3g) was cyclised according to the method of Intermediate 9 to give the title compound (1.0g), m.p. 213-215°.

Intermediate 13

6,9-Difluoro-2,3,4,5-tetrahydro-1H-pyrido[4,3-b]indol-1-one

4-[(2,5-Difluorophenyl)amino]-5,6-dihydro-2(1H)-pyridinone (2.24g) was cyclised according to the method of Intermediate 8 to give the title compound (900mg), m.p. 221-223°.

Intermediate 14

8-Fluoro-1,2,3,9-tetrahydro-4H-carbazol-4-one

3-[(2-Fluorophenyl)amino]-2-cyclohexen-1-one (14.8g), palladium (II) acetate (1g), and copper (II) acetate (29.5g) were heated together in DMF (100mℓ) under nitrogen at 140° for 2h. The solvent was removed in vacuo, and the residue was purified by FCC eluting with ether to give the title compound (10.1g), m.p. 222-224°.

Intermediate 15

2,3,4,5-Tetrahydro-6-methoxy-1H-pyrido[4,3-b]indol-1-one

A mixture of 2-methoxyaniline (8.7g) and 2,4-dioxopiperidine (8.0g) was heated at 120° for 3h. The mixture was then cooled and purified by FCC eluting with System A (100:8:1) to give an oil (11g). This oil was treated according to the method of Intermediate 14 (with the exception that System A (100:8:1) was used as the FCC eluant) to give the title compound (3.2g), m.p. 255-258°.

Intermediate 16

2,3,4,5-Tetrahydro-6-(phenylmethoxy)-1H-pyrido[4,3-b]indol-1-one

A mixture of 2-(phenylmethoxy)aniline (7.6g) and 2,4-dioxopiperidine (4,5g) was heated at 120° under nitrogen for 3h. The mixture was then cooled and purified by FCC eluting with System A (100:8:1) to give a solid (5.4g). This solid was treated according to the method of Intermediate 9 to give the title compound (4.0g), m.p. 182-185°.

Intermediate 17

6-Fluoro-2,3,4,5-tetrahydro-5-methyl-1H-pyrido[4,3-b]indol-1-one

Sodium hydride (60% dispersion in oil; 196mg) was added to a stirred suspension of 6-fluoro-2,3,4,5-tetrahydro-1H-pyrido[4,3-b]indol-1-one (500mg) in dry DMF (10mℓ) under nitrogen. After 30 min the solution was cooled (0°), and methyl iodide (0.153mℓ) was added. After stirring for 15 min the suspension was poured into water (50mℓ) and extracted with dichloromethane (3x25mℓ). The combined, dried organic extracts were evaporated to give a solid (ca. 530mg) which was purified by FCC eluting with System A (250:10:1) to give the title compound (130mg), m.p. 242°.

Intermediate 18

8-Fluoro-2,3,4,5-tetrahydro-5-methyl-1H-pyrido[4,3-b]indol-1-one

Sodium hydride (60% dispersion in oil; 196mg) was added to a stirred solution of 8-fluoro-2,3,4,5-tetrahydro-1H-pyrido[4,3-b]indol-1-one (500mg) in dry DMF (20mℓ) at 21° under nitrogen. After 15 min, the solution was cooled (0°), and a 10% (v/v) solution of methyl iodide in DMF (1.6mℓ) was added dropwise. After 10 min, water (150mℓ) was added and the mixture was extracted with ethyl acetate (3 x 50mℓ). The combined organic extracts were washed with water (3 x 50mℓ), then brine (100mℓ) and evaporated to give a solid which was purified by FCC eluting with dichloromethane: methanol (80:1) to give the title compound (240mg), m.p. 200-202°.

Intermediates 19 to 23 were prepared in a similar manner to Intermediate 18, i.e. by methylation of the appropriate 2,3,4,5-tetrahydro-1H-pyrido[4,3-b]indol-1-one using sodium hydride and methyl iodide in DMF.

Intermediate 19

2,3,4,5-Tetrahydro-5,6-dimethyl-1H-pyrido[4,3-b]indol-1-one

The methylation of 2,3,4,5-tetrahydro-6-methyl-1H-pyrido[4,3-b]indol-1-one (350mg) gave the title compound (190mg), m.p. 298-300°.

Intermediate 20

6-Bromo-2,3,4,5-tetrahydro-5-methyl-1H-pyrido[4,3-b]indol-1-one

The methylation of 6-bromo-2,3,4,5-tetrahydro-1H-pyrido[4,3-b]indol-1-one (200mg) gave the title compound (80mg), m.p. 212-214°.

Intermediate 21

7-Fluoro-2,3,4,5-tetrahydro-5-methyl-1H-pyrido[4,3-b]indol-1-one

The methylation of 7-fluoro-2,3,4,5-tetrahydro-1H-pyrido[4,3-b]indol-1-one (900mg) gave the title compound (200mg), m.p. 238-240°. (The FCC eluant was System A (100:8:1)).

Intermediate 22

6,9-Difluoro-2,3,4,5-tetrahydro-5-methyl-1H-pyrido[4,3-b]indol-1-one

The methylation of 6,9-difluoro-2,3,4,5-tetrahydro-1H-pyrido[4,3-b]indol-1-one (900mg) gave the title compound (110mg), m.p. 226-229°. (The FCC eluant was System A (150:8:1)).

Intermediate 23

2,3,4,5-tetrahydro-6-methoxy-5-methyl-1H-pyrido[4,3-b]indol-1-one

The methylation of 2,3,4,5-Tetrahydro-6-methoxy-1H-pyrido[4,3-b]indol-1-one (1.5g) gave the title compound (680mg), m.p. 242-245°. (The FCC eluant was System A (100:8:1)).

Intermediate 24

8-Fluoro-1,2,3,9-tetrahydro-9-methyl-4H-carbazol-4-one

To a suspension of sodium hydride (80% dispersion in oil; 1.15g) in dry THF (50mℓ) under nitrogen was added 8-fluoro-1,2,3,9-tetrahydro-4H-carbazol-4-one (6.5g) in dry THF (50mℓ), and the mixture was stirred for 1h. Methyl iodide (4.1mℓ) was added, and the mixture was stirred for 3h. The mixture was then poured into brine (300mℓ) and extracted with ether (2x300mℓ). The combined, dried organic extracts were evaporated in vacuo to give the title compound (5.77g), m.p. 126-128°.

Intermediate 25

2,3,4,5-Tetrahydro-5-methyl-6-(phenylmethoxy)-1H-pyrido[4,3-b]indol-1-one

2,3,4,5-Tetrahydro-6-(phenylmethoxy)-1H-pyrido[4,3-b]indol-1-one (2.0g) was methylated according to the method of Intermediate 17 to give the title compound (950mg), m.p. 199-201°. (The FCC eluant was System A (100:8:1)).

Intermediate 26

6-Fluoro-2,3,4,5-tetrahydro-5-[(phenylmethoxy)methyl]-1 H-pyrido[4,3-b]indol-1-one

6-Fluoro-2,3,4,5-tetrahydro-1H-pyrido[4,3-b]indol-1-one (800mg) was alkylated with benzylchloromethyl ether (0.55mℓ) using the method of Intermediate 17 to give the title compound (220mg), m.p. 130-132°. (The FCC eluent was System A (300:10:1)).

Intermediate 27

2,3,4,5-Tetrahydro-6-(phenylmethoxy)-5-[(phenylmethoxy)methyl]-1H-pyrido[4,3-b]indol-1-one

2,3,4,5-Tetrahydro-6-(phenylmethoxy)-1H-pyrido[4,3-b]indol-1-one (1.8g) was alkylated with benzylchloromethyl ether (0.86mℓ) using the method of Intermediate 18 to give the title compound (600mg), m.p. 188-190°. (The FCC eluant was System A (100:8:1)).

Intermediate 28

6-Fluoro-2,3,4,5-tetrahydro-5-(1-methylethyl)-1H-pyrido[4,3-b]indol-1-one

A stirred solution of 6-fluoro-2,3,4,5-tetrahydro-1H-pyrido[4,3-b]indol-1-one (1.006g) in dry DMF (50mℓ) was treated with sodium hydride (73.2% dispersion in oil; 333mg) and stirred under nitrogen for 1h. Isopropyl bromide (663mg) was then added, and the solution was stirred at room temperature for 30 min and then at 50° for 12h. A further portion of isopropyl bromide (150mg) was then added, and the reaction was then stirred under nitrogen at 50° for ca. 60h. The mixture was then cooled to room temperature and added to water (300mℓ). The mixture was then extracted with dichloromethane (3x200mℓ), adsorbed onto silica, and purified by FCC eluting with System A (150:8:1) to give a solid (240mg) which was triturated with ether to give the title compound (130mg), m.p. 183-184°.

Intermediate 29

5-(Cyclopentylmethyl)-6-fluoro-2,3,4,5-tetrahydro-1H-pyrido[4,3-b]indol-1-one

A stirred solution of 6-fluoro-2,3,4,5-tetrahydro-1H-pyrido[4,3-b]indol-1-one (1.023g) in dry DMF (50mℓ) was treated with sodium hydride (73.2% dispersion in oil; 331mg) and stirred under nitrogen at room temperature for 30 min. A

solution of cyclopentylmethyl(methyl sulphonate) (893mg) in dry DMF (20mℓ) was then added over 15 min and stirring was continued for 5 days. Water (20mℓ) was added to the reaction mixture which was then concentrated in vacuo to give a solid. This was dissolved in ethyl acetate (300mℓ) and methanol (1mℓ), and the resulting solution was washed with saturated sodium chloride solution (3x100mℓ) and then adsorbed into silica. Purification by FCC eluting with System A (150:8:1) gave a solid (283mg) which was recrystallised from ethyl acetate to give the title compound (237mg) m.p. 175-176°.

Intermediate 30

8-Fluoro-1,2,3,9-tetrahydro-9-methyl-4H-carbazol-4-one oxime

A mixture of 8-fluoro-1,2,3,9-tetrahydro-9-methyl-4H-carbazol-4-one (3.0g) and hydroxylamine hydrochloride (2.92g) in pyridine (ca. 35mℓ) was heated at 60° for 3h. The solution was concentrated in vacuo then azeotropically dried with toluene (2x10mℓ). The residue was treated with 8% sodium bicarbonate solution (125mℓ) and extracted with ethyl acetate (3x100mℓ). The combined extracts were filtered and concentrated in vacuo to give the title compound (1.8g) as a solid, t.l.c. (System A, 100:8:1) Rf 0.66.

Intermediate 31

7-Fluoro-3,4,5,6-tetrahydro-6-methylazepino[4,3-b]indol-1(2 H)-one

A mixture of 8-fluoro-1,2,3,9-tetrahydro-9-methyl-4H-carbazol-4-one oxime (1.8g) and polyphosphoric acid (ca. 20mℓ) in dioxan (30mℓ) was stirred under nitrogen at 100-110° for 1h. After cooling, the reaction mixture was poured into iced water (1ℓ) and the resulting suspension was extracted with dichloromethane. The organic extract was concentrated in vacuo to give a solid which was taken up in methanol and adsorbed onto silica. Purification by FCC eluting with System A (200:8:1) gave the title compound (850mg), m.p. 233-235°.

Intermediate 32

2,3,4,5-Tetrahydro-5-methyl-1-oxo-1H-pyrido[4,3-b]indole-6-carbonitrile

A mixture of 6-bromo-2,3,4,5-tetrahydro-5-methyl-1H-pyrido[4,3-b]indol-1-one (1.1g) and cuprous cyanide (1.0g) in 1,3-dimethyl-3,4,5,6-tetrahydro-2(1H)-pyrimidone (30mℓ) was heated at 180° for 24h. The mixture was poured onto ice (ca. 500mℓ) and ferric (III) chloride (20g), and stirred for 1h. It was then extracted with dichloromethane (3x300mℓ), and the combined organic extracts were washed with water (2x300mℓ) and concentrated in vacuo. The residue was triturated with hexane (250mℓ) followed by a mixture of ether/hexane (50:50; 100mℓ) and finally with ether (60mℓ) to give the title compound (620mg), m.p. 230-231°.

Intermediate 33

2,3,4,5-Tetrahydro-5-methyl-6-phenyl-1H-pyrido[4,3-b]indol-1-one

A mixture of 6-bromo-2,3,4,5-tetrahydro-5-methyl-1H-pyrido[4,3-b]indol-1-one (200mg), phenylboronic acid (131mg), tetrakis(triphenylphosphine)palladium (0) (11mg) and 2N sodium carbonate (5mℓ) in DME (10mℓ) was heated at reflux for 3h. The cooled mixture was diluted with 2N sodium carbonate (50mℓ) and extracted with dichloromethane (3x100mℓ). The combined organic extracts were concentrated in vacuo and the residue was purified by FCC eluting with System A (100:8:1) to give a solid (180mg) which was triturated with ether (20mℓ) to give the title compound (160mg), m.p. 242-245°.

Intermediate 34

6-Fluoro-2,5-dihydro-5-methyl-1H-pyrido[4,3-b]indol-1-one

A mixture of 6-fluoro-2,3,4,5-tetrahydro-5-methyl-1H-pyrido[4,3-b]indol-1-one (300mg) and 10% palladium oxide on carbon catalyst (50% aqueous paste; 150mg) in ethylene glycol (30mℓ) was heated at reflux under nitrogen for 24h. The cooled mixture was filtered and evaporated to give a solid (ca.300mg) which was purified by FCC eluting with System A (200:10:1) to give a solid (100mg). A sample of this solid was further purified by HPLC on a Spherisorb 55W column, eluting with System A (95:5:0.5) at a flow rate of 15ml/min to give the title compound, m.p. 294-296°.

Intermediate 35

6-Fluoro-2,3,4,5-tetrahydro-2-[(5-methyl-1H-imidazol-4-yl)methyl]-5-[(phenylmethoxy)methyl]-1H-pyrido[4,3-b]indol-1-one

Sodium hydride (60% dispersion in oil; 28mg) was added to a stirred solution of 6-fluoro-2,3,4,5-tetrahydro-5-[(phenylmethoxy)methyl]-1H-pyrido[4,3-b]indol-1-one (190mg) in dry DME (10mℓ). The mixture was heated at 50° for 6h then treated with 4-(chloromethyl)-5-methyl-1-(triphenylmethyl)-1H-imidazole (261mg) and stirring was continued under nitrogen for 18h. Water (2mℓ) and acetic acid (2mℓ) were added and the solution was heated at reflux for 2.5h. The solution was poured into 8% sodium bicarbonate solution (50mℓ) and extracted with dichloromethane (3x25mℓ). The combined, dried organic extracts were evaporated to give an oil (ca. 750mg) which was purified by FCC eluting with System A (200:10:1) to give the title compound (188mg), t.l.c. (System A, 200:10:1) Rf 0.33.

Intermediate 36

6-Fluoro-2,3,4,5-tetrahydro-2-[[5-methyl-1-(triphenylmethyl)-1 H-imidazol-4-yl]methyl]-1H-pyrido[4,3-b]indol-1-one

A solution of triphenylmethyl chloride (0.625g) in dry DMF (10mℓ) was added dropwise to a stirred solution of 6-fluoro-2,3,4,5-tetrahydro-2-[(5-methyl-1H-imidazol-4-yl)methyl]-1H-pyrido[4,3-b]indol-1-one (530mg) in dry DMF (20mℓ) containing triethylamine (0.25g). The reaction mixture was then stirred at room temperature for 20h, added to water (500mℓ), and extracted with ethyl acetate (3x200mℓ). The combined organic extracts were washed with water (2x300mℓ), dried and adsorbed onto silica. Purification by FCC eluting with System A (150:8:1) gave the title compound (509mg), m.p. 252-253°.

Intermediate 37

2,3,4,5-Tetrahydro-2-[(5-methyl-1H-imidazol-4-yl)methyl]-6-(phenylmethoxy)-5-[(phenylmethoxy)methyl]-1H-pyrido[4,3-b]indol-1-one

2,3,4,5-Tetrahydro-6-(phenylmethoxy)-5-[(phenylmethoxy)methyl]-1H-pyrido[4,3-b]indol-1-one (500mg) was reacted with 4-(chloromethyl)-5-methyl-1-(triphenylmethyl)-1H-imidazole (671mg) according to the method of Intermediate 35 to give the title compound (340mg), m.p. 170-172°. (The FCC eluant was System A (100:8:1)).

Intermediate 38

4-[(6-Bromo-2,3,4,5-tetrahydro-5-methyl-1-oxo-1H-pyrido[4,3-b]indol-2-yl)methyl]-N,N,5-trimethyl-1 H-imidazole-1-sulphonamide

Dimethylsulphamoyl chloride (0.31mℓ) was added dropwise to a stirred suspension of 6-bromo-2,3,4,5-tetrahydro-5-methyl-2-[(5-methyl-1H-imidazol-4-yl)methyl]-1H-pyrido[4,3-b]indol-1-one (1.1g) and triethylamine (0.48mℓ) in dry dichloromethane (100mℓ). The mixture was heated at reflux for 18h, then a further quantity of triethylamine (0.48mℓ) and dimethylsulphamoyl chloride (0.31mℓ) was added. The solution was heated at reflux for 8h then left at 21° for ca. 60h. The solution was concentrated in vacuo and purified by FCC eluting with System A (100:8:1) to give a solid, which was triturated with hexane (50mℓ) to give the title compound (500mg), m.p. 182-185°.

Intermediate 39

Methyl 3-[2,3,4,5-tetrahydro-5-methyl-2-[(5-methyl-1 H-imidazol-4-yl)- methyl]-1-oxo-1H-pyrido[4,3-b]indol-6-yl]-2-propenoate

A mixture of 4-[(6-bromo-2,3,4,5-tetrahydro-5-methyl-1-oxo-1H-pyrido[4,3-b]indol-2-yl)methyl]-N,N,5-trimethyl-1H-imidazole-1-sulphonamide (500mg) acrylic acid (0.08mℓ), palladium acetate (27mg) and tri-o-tolylphosphine (54mg) in triethylamine (0.27mℓ) and acetonitrile (7mℓ) was heated at 80-90° for 18h in a closed vessel. A further quantity of palladium acetate (27mg) and triethylamine (0.27mℓ) were added and the mixture was heated for 6h then concentrated in vacuo. The residue was treated with 2N sodium hydroxide (30mℓ), washed with ether (3x30mℓ) and the aqueous phase (plus oily residue) was then acidified (to pH1) with 5N hydrochloric acid. The aqueous phase was extracted with dichloromethane:methanol (10:1; 3x150mℓ) and the combined organic extracts were concentrated in vacuo. Methanol (100mℓ) was added to the residue, followed by concentrated hydrochloric acid (0.15mℓ), and the

mixture was heated at reflux for 5h. The mixture was then concentrated in vacuo and the residue was basified (to pH9) with 8% sodium bicarbonate solution. The mixture was filtered and the solid was washed with water (ca. 100mℓ) and dried at 60° in vacuo to give the title compound (230mg), m.p. 175-178°.

Intermediate 40

4-Amino-5,6-dihydro-1-[(5-methyl-1H-imidazol-4-yl)methyl]-2(1H)-pyridinone

To a solution of 5,6-dihydro-4-methoxy-1-[(5-methyl-1H-imidazol-4-yl)methyl]-2(1H)-pyridinone (1.00g) THF (10mℓ) was added hydrochloric acid (4.3mℓ), and the mixture was stirred at 25° for 24h. The solvent was removed in vacuo. The residue was dissolved in methanol (10mℓ), adsorbed onto silica, and purified by FCC eluting with System A (50:8:1) to give the title compound (286mg) as a solid, m.p 268-271°.

Intermediate 41

4-[2-(2-Fluorophenyl)-2-methylhydrazine]-5,6-dihydro-1-[(5-methyl-1H-imidazol-4-yl)methyl]-2(1H)-pyridinone

A mixture of 1-(2-fluorophenyl)-1-methylhydrazine (57mg) and 4-amino-5,6-dihydro-1-[(5-methyl-1H-imidazol-4-yl)methyl]-2(1H)-pyridinone (70mg) in absolute ethanol (3mℓ) was stirred at room temperature for 4h then at reflux for 20h. The solvent was removed in vacuo and the residue was purified by FCC eluting with System A (75:10:1) to give the title compound (85mg) as an oil, t.l.c. (System A, 200:10:1) Rf 0.27.

Intermediate 42

6-Fluoro-2,3,4,5-tetrahydro-5-methyl-2-[(4-methyloxazol-5-yl)methyl]-1H-pyrido[4,3-b]indol-1-one

Sodium hydride (73% dispersion in oil; 167mg) was added to a stirred suspension of 6-fluoro-2,3,4,5-tetrahydro-5-methyl-1H-pyrido[4,3-b]indol-1-one (500mg) in dry DME (150mℓ), and the suspension was stirred at 60° under nitrogen for 6h.
5-Chloromethyl-4-methyloxazole (440mg) was then added and the mixture was stirred overnight at 60°. A further quantity of sodium hydride (73% dispersion in oil; 84mg) was added and the mixture was stirred for 3h, and then cooled (0°). Water (200mℓ) was added, and the mixture was extracted with dichloromethane (3x200mℓ). The combined organic extracts were concentrated in vacuo and the residue was purified by FCC eluting with System A (100:8:1) to give the title compound (550mg) as a solid, m.p. 158-161°.

Intermediate 43

Ethyl 7-fluoro-1-methyl-1H-indole-2-carboxylate

To a suspension of sodium hydride (73.2% dispersion in oil; 962mg) in dry THF (50 mℓ) under nitrogen at 0° was added ethyl 7-fluoro-1H-indole-2-carboxylate (5.5g) in dry THF (50mℓ), and the mixture was stirred for 1h. Methyl iodide (2.16mℓ) was added, and the mixture was stirred for 2h, then at 50° for 3h.
The mixture was quenched with 10% aqueous THF (5mℓ), diluted with ether (500mℓ), washed with saturated brine solution (2x500 mℓ), dried and evaporated in vacuo to leave an oil. This was purified by FCC eluting with hexane:ether (20:1) to give the title compound (4.53g) as a liquid, t.l.c. (hexane:ether, 20:1) Rf 0.37.

Intermediate 44

7-Fluoro-1-methyl-1H-indole-2-methanol

Ethyl 7-fluoro-1-methyl-1H-indole-2-carboxylate (4.5g) was dissolved in dry THF (50mℓ), and cooled to -78° under nitrogen.
Diisobutylaluminium hydride (DIBAL) (1M solution in hexane; 22.3mℓ) was added and the mixture was stirred at -78° for 3h, then allowed to warm to room temperature over 1h. A further portion of DIBAL (4mℓ) was added to the recooled mixture at -78°, and stirring was continued for a further 16h. Saturated ammonium chloride solution (10mℓ) was added and the mixture was stirred for 30 min. Magnesium sulphate (dried, 10g) was added, and the resultant mixture was filtered and evaporated in vacuo to leave an oil which was purified by FCC eluting with ether:hexane (1:1) to give the title compound (1.55g) as a solid, m.p. 89-91°.

Intermediate 45

7-Fluoro-1-methyl-1H-indole-2-carboxaldehyde

7-Fluoro-1-methyl-1H-indole-2-methanol (2.0g) was dissolved in 1,4-dioxan (50mℓ). Manganese dioxide (2.91g) was added and the mixture was heated at reflux for 1h. The mixture was then filtered and the filtrate was evaporated in vacuo to give the title compound (1.77g) as a solid, m.p. 61-63°.

Intermediate 46

(E)-7-Fluoro-1-methyl-2-(2-nitroethenyl)-1H-indole

To a solution of 7-fluoro-1-methyl-1H-indole-2-carboxaldehyde (1.80g) in nitromethane (20mℓ) was added ammonium acetate (780mg) and the mixture was heated at reflux far 1h. The solvent was removed in vacuo and the residue was suspended in ethyl acetate (300mℓ). This suspension was washed with 8% aqueous sodium bicarbonate solution (2x300mℓ), dried, and the solvent was removed in vacuo to leave the title compound (1.26g) as a solid, m.p. 153-155.5°.

Intermediate 47

2,2,2-Trifluoro-N-[2-(7-fluoro-1-methyl-1H-indol-2-yl)ethyl]acetamide

(E)-7-Fluoro-1-methyl-2-(2-nitroethenyl)-1H-indole (1.2g) in dry HF (40mℓ) was added slowly to a stirred suspension of lithium aluminium hydride (1.0g) in dry THF (40mℓ) under nitrogen and the mixture was stirred overnight at room temperature. Another portion of lithium aluminium hydride (200mg) was added, and the mixture was stirred for a further 18h. 10% Aqueous THF (10mℓ) was added slowly and the mixture was stirred for 30 min. Magnesium sulphate (20g) was added, the mixture was filtered, and the filter cake was washed with ethyl acetate (100mℓ). The combined filtrates were evaporated in vacuo to leave a gum which was dissolved in dichloromethane (100mℓ). Triethylamine (5mℓ) was added, and the mixture was cooled to 0°. Trifluoroacetic anhydride (1mℓ) was added dropwise and the resulting mixture was stirred far 1h. The mixture was diluted with dichloromethane (100mℓ), washed with 8% aqueous sodium bicarbonate solution (2x100mℓ), dried and the solvent removed in vacuo to leave a gum. This was purified by FCC eluting with hexane:ether (2:1) to give the title compound (293mg) as a solid, m.p. 108.5-109.5°.

Intermediate 48

N-[[1-[(Dimethylamino)sulphonyl]-5-methyl-1 H-imidazol-4-yl)methyl]- 2,2,2-trifluoro-N-[2-(7-fluoro-1-methyl-1H-indol-2-yl)ethyl]acetamide

2,2,2-Trifluoro-N-[2-(7-fluoro-1-methyl-1H-indol-2-yl)ethyl]acetamide (263.5mg) in dry DME (5mℓ) was added to a suspension of sodium hydride (73.2% dispersion in oil; 33mg) in dry DME (4mℓ) under nitrogen, and the mixture was stirred at 50° for 1h. 4-(Chloromethyl)-N,N,5-trimethyl-1H-imidazole-1-sulphonamide (239mg) in dry DME (5mℓ) was then added, and the mixture was stirred at 50° overnight. The mixture was then diluted with ethyl acetate (100mℓ), washed with brine (100mℓ), dried and evaporated in vacuo to leave an oil. This was purified by FCC eluting with ethyl acetate:ether (1:1) to give the title compound (243mg) as a foam, m.p. 43-45°.

Intermediate 49

4-[[[2-(7-Fluoro-1-methyl-1H-indol-2-yl)ethyl]amino]methyl]-N,N,5-trimethyl-1H-imidazole-1-sulphonamide

To a solution of N-[[1-[(dimethylamino)sulphonyl]-5-methyl-1H-imidazol -4-yl]methyl]-2,2,2-trifluoro-N-[2-(7-fluoro-1-methyl-1H-indol-2-yl)-ethyl]acetamide (230mg) in methanol (10mℓ) was added potassium carbonate (350mg), and the mixture was heated at reflux for 2h. The mixture was then diluted with ethyl acetate (100mℓ), washed with 8% sodium bicarbonate solution (100mℓ), dried, and evaporated in vacuo to leave an oil. This was purified by FCC eluting with System A (200:8:1) to give the title compound (110mg) as a solid, m.p. 114-115.5°.

Intermediate 50

4-[(6-Fluoro-2,3,4,5-tetrahydro-5-methyl-1-oxo-1H-pyrido[4,3-b]indol-2-yl)methyl]-N,N,5-trimethyl-1H-imidazole-1-sulphonamide

To a stirred solution of phosgene (12.5% v/v in toluene; 4mℓ) in dichloromethane (4mℓ) was added a solution of 4-[[[2-(7-fluoro-1-methyl-1H-indol-2-yl)ethyl]amino]methyl]-N,N,5-trimethyl-1H-imidazole-1-sulphonamide (100mg) in dichloromethane (4mℓ), and the resulting mixture was stirred for 1h. The solvent was removed in vacuo, and the residue was dissolved in dichloromethane (5mℓ). Aluminium chloride (68mg) was added, and the mixture was heated at reflux for 3h. The cooled mixture was then poured into 2M sodium hydroxide solution (80mℓ) and extracted with dichloromethane (2x50mℓ). The combined, dried organic extracts were evaporated in vacuo to leave a solid which was dissolved in dichloromethane:methanol (1:1; 50mℓ) and adsorbed onto silica. Purification by FCC eluting with System A (200:8:1) gave a solid (21mg) which was triturated with ether to give the title compound (14mg), m.p. 162-166°.

Intermediate 51

6-Fluoro-2,3,4,5-tetrahydro-5-methyl-2-[(5-methyl-1H-imidazol-4-yl)-methyl]-1H-pyrido[4,3-b]indole

A stirred solution of 6-fluoro-2,3,4,5-tetrahydro-5-methyl-2-[(5-methyl-1H-imidazol-4-yl)methyl]-1H-pyrido[4,3-b]indol-1-one (277mg) in dry THF (75mℓ) was treated portionwise with lithium aluminium hydride (67mg) under nitrogen, and the suspension was then heated at reflux for 2h. After cooling, water (10mℓ) was added dropwise followed by sodium sulphate, and the mixture was then filtered. The filtrate was adsorbed onto silica and purified by FCC eluting with System A (100:8:1) to give the title compound (196mg) as a solid, m.p. 188.5-189.5°.

Intermediate 52

6-Fluoro-2,3,4,5-tetrahydro-2-[[1-(methoxymethyl)-5-methyl-1H-imidazol-4-yl]methyl]-5-methyl-1H-pyrido[4,3-b]indol-1-one and 6-Fluoro-2,3,4,5-tetrahydro-2-[[1-(methoxymethyl)-4-methyl-1H-imidazol-5-yl]methyl]-5-methyl-1H-pyrido[4,3-b]indol-1-one

A stirred solution of 6-fluoro-2,3,4,5-tetrahydro-5-methyl-2-[(5-methyl-1H-imidazol-4-yl)methyl]-1H-pyrido[4,3-b]indol-1-one maleate (728mg) and triethylamine (0.52mℓ) in dichloromethane (50mℓ) was treated with a solution of chloromethyl methyl ether (0.27mℓ) in dichloromethane (20mℓ), and the mixture was stirred at 20° under nitrogen for 2 $\frac{1}{2}$ days. The reaction mixture was then added to water (50mℓ) and sodium hydroxide (100mℓ) and extracted with dichloromethane (2x75mℓ). The combined, dried organic extracts were adsorbed onto silica, and purified by FCC eluting with System A (150:8:1) to give an oil. This oil was triturated with ether to give the title compounds (60mg) as a solid, t.l.c. (System A, 100:8:1) Rf 0.32.

Intermediate 53

Phenylmethyl 4-[(6-fluoro-2,3,4,5-tetrahydro-5-methyl-1-oxo-1 H-pyrido[4,3-b]indol-2-yl)methyl]-5-methyl-1H-imidazole-1-carboxylate

A solution of benzyl chloroformate (0.47mℓ) in dichloromethane (1mℓ) was added to a stirred solution of 6-fluoro-2,3,4,5-tetrahydro-5-methyl-2-[(5-methyl-1H-imidazol-4-yl)methyl]-1H-pyrido[4,3-b]indol-1-one (496mg) and triethylamine (0.67mℓ) in dry dichloromethane (50mℓ) at 20° under nitrogen, and the mixture was stirred overnight. The cooled reaction mixture was then added to 2N sodium hydroxide (100mℓ) and extracted with dichloromethane (2x100mℓ). The combined, dried organic extracts were adsorbed onto silica and purified by FCC eluting with System A (200:8:1) to give the title compound (442mg) as a solid. A sample was recrystallised from hot ethyl acetate, and the resultant solid was triturated with ether to give a crystalline solid, m.p 126-128°.

Example 1

6-Fluoro-2,3,4,5-tetrahydro-5-methyl-2-[(5-methyl-1H-imidazol-4-yl)- methyl]-1H-pyrido[4,3-b]indol-1-one maleate

Sodium hydride (60% dispersion in oil; 25mg) was added to a stirred suspension of 6-fluoro-2,3,4,5-tetrahydro-5-methyl-1H-pyrido[4,3-b]indol-l-one (115mg) in dry DME (7.5mℓ) under nitrogen. The mixture was heated at 50° for 6h then treated with 4-(chloromethyl)-5-methyl-1-(triphenylmethyl)-1H-imidazole (236mg), and stirring was continued

at 50° for 18h. Water (1.25mℓ) and acetic acid (1.25mℓ) were added and the solution was heated at reflux for 6h. The mixture was poured into 8% sodium bicarbonate solution (40mℓ) and extracted with dichloromethane (3x20mℓ). The combined, dried organic extracts were evaporated to give a solid (375mg) which was purified by FCC eluting with System A (200:10:1) to give the free base of the title compound as a solid (147mg). This was dissolved in dichloromethane (3mℓ) and treated with a solution of maleic acid (55mg) in absolute ethanol (0.5mℓ). The solvent was removed in vacuo and the residue was triturated with dry ether (3x5mℓ) to give the title compound (185mg), m.p. 178-180°.

| | | | |
|---|---|---|---|
| Analysis Found: | C,59.0; | H,5.0; | N,12.85; |
| $C_{17}H_{17}FN_4O.C_4H_4O_4$ requires | C,58.9; | H,4.9; | N,13.1%. |

Examples 2 to 13 were prepared in a similar manner to Example 1, i.e. by reacting the appropriate lactam with 4-(chloromethyl)-5-methyl-1-(triphenylmethyl)-1H-imidazole (hereinafter referred to as Compound X) in DME in the presence of sodium hydride. Deprotection was effected with acetic acid and water (followed by 2N hydrochloric acid in the case of Example 8), and subsequent basification of the solution was then effected with 2N sodium hydroxide solution rather than 8% sodium bicarbonate solution. The basic solution'was then extracted with dichloromethane (or ethyl acetate in the case of Examples 6 and 12), and the combined, dried organic extracts were evaporated in vacuo. Purification of the residue was by FCC eluting with System A [Ex. 2 (160:8:1), Exs. 3,6,7,12,13 (150:8:1) and Exs. 4,5,8,9,10,11 (100:8:1)] to give the free base of the title compound. Maleate formation was as described in Example 1 except that methanol was used (instead of ethanol) as the recrystallisation solvent, and the mixture was heated on a steam bath for 10min. The product was obtained from this methanolic solution either by evaporation to dryness in vacuo, and subsequent trituration of the resultant residue with ether (Exs. 4,5,8,9,11) or recrystallisation of the residue from a mixture of methanol and ether (Ex. 10), or the product was precipitated from the methanolic solution by the addition of ether (Exs.2,3,6,7,12,13). The products of Examples 2 and 11 were further purified as described below.

Example 2

8-Fluoro-2,3,4,5-tetrahydro-5-methyl-2-[(5-methyl-1H-imidazol-4-yl)methyl]-1H-pyrido[4,3-b]indol-1-one maleate

The reaction of 8-fluoro-2,3,4,5-tetrahydro-5-methyl-1H-pyrido[4,3-b]indol-1-one (200mg) with compound X (371mg) gave the free base of the title compound (210mg). Maleate formation gave a solid (190mg) which was recrystallized from methanol/ether to give a solid (85mg), a portion of which (78mg) was reconverted to the free base by addition of 2N sodium hydroxide (10mℓ) and extraction into dichloromethane (3x10mℓ). The combined, dried organic extracts were concentrated in vacuo to give the free base (70mg) which was purified by HPLC (Zorbax 5-6µm silica, 250x9.4mm column) eluting with hexane: chloroform: ethanol: 0.88 ammonia solution (40:100:20:0.2) to give a solid (40mg). This was treated with maleic acid (15mg) in methanol on a steam bath for 10 min. The solution was concentrated in vacuo and the residue was triturated with ether (15mℓ) to give the title compound (45mg), m.p. 145°.
$^1$H-N.m.r. δ2.35 (3H,s), 3.12 (2H,t), 3.68 (2H,t), 3.75 (3H,s), 4.67 (2H,brs), 6.08 (2H,s), 7.085 (1H,ddd), 7.53-7.655 (2H,m), 8.83 (1H,brs).

Example 3

2,3,4,5-Tetrahydro-5,6-dimethyl-2-[(5-methyl-1H-imidazol-4-yl)methyl]-1H-pyrido[4,3-b]indol-1-one maleate

The reaction of 2,3,4,5-tetrahydro-5,6-dimethyl-1H-pyrido[4,3-b]indol-1-one (150mg) with Compound X (260mg) gave the free base of the title compound (120mg). Maleate formation gave the title compound (llOmg), t.l.c. (System A, 150:8:1) Rf 0.3.
$^1$H-N.m.r. δ2.35 (3H,s), 2.75 (3H,s), 3.08 (2H,t), 3.64 (2H,t), 3.93 (3H,s), 4.62 (2H,s), 6.07 (2H,s), 6.90 (1H,brd), 7.01 (1H,t), 7.88 (1H,brd), 8.73 (1H,brs).

Example 4

6-Bromo-2,3,4,5-tetrahydro-5-methyl-2-[(5-methyl-1H-imidazol-4-yl)methyl]-1H-pyrido[4,3-b]indol-1-one maleate

The reaction of 6-bromo-2,3,4,5-tetrahydro-5-methyl-1H-pyrido-[4,3-b]indol-1-one (140mg) with Compound X (280mg) gave the free base of the title compound (130mg). Maleate formation gave the title compound (170mg), m.p. 155°.
Water Analysis Found 2.86%w/w ≡ 0.79mol $H_2O$.

| | | | |
|---|---|---|---|
| Analysis Found: | C,50.1; | H,4.4; | N,10.7; |
| $C_{17}H_{17}BrN_4O.C_4H_4O_4.0.79H_2O$ requires | C,50.1; | H,4.5; | N,11.1%. |

Example 5

7-Fluoro-2,3,4,5-tetrahydro-5-methyl-2-[(5-methyl-1H-imidazol-4-yl )methyl]-1H-pyrido[4,3-b]indol-1-one maleate

The reaction of 7-fluoro-2,3,4,5-tetrahydro-5-methyl-1H-pyrido-[4,3-b]indol-1-one (150mg) with Compound X (386mg) gave the free base of the title compound (200mg). Maleate formation gave the title compound (200mg), m.p. 150°.

$^1$H-N.m.r. δ 2.36(3H,s), 3.11(2H,t), 3.67(2H,t), 3.72(3H,s), 4.66(2H,s), 6.08(2H,s), 7.04(1H,m), 7.47(1H,dd), 7.92(1H, dd), 8.87(1H,s)

Example 6

6,9-Difluoro-2,3,4,5-tetrahydro-5-methyl-2-[(5-methyl-1 H-imidazol-4-yl)methyl]-1H-pyrido[4,3-b]indol-1-one maleate

The reaction of 6,9-difluoro-2,3,4,5-tetrahydro-5-methyl-1H-pyrido [4,3-b]indol-1-one (1.0g) with Compound X (1.7g) gave the free base of the title compound (846mg). Maleate formation gave the title compound (920mg), m.p. 206-208°.

| | | | |
|---|---|---|---|
| Analysis Found: | C,56.1; | H,4.6; | N,12.6; |
| $C_{17}H_{16}F_2N_4O.C_4H_4O_4$ requires | C,56.5; | H,4.5; | H,12.6%. |

Example 7

7-Fluoro-3,4,5,6-tetrahydro-6-methyl-2-[(5-methyl-1H-imidazol-4-yl)methyl)-azepino[4,3-b]indol-1(2H)-one maleate

The reaction of 7-fluoro-3,4,5,6-tetrahydro-6-methylazepino[4,3-b]indol-1(2H)-one (850mg) with Compound X (1.64g) gave the free base of the title compound (358mg). Maleate formation gave the title compound (383mg) m.p. 143-145°.

Water Analysis Found: 1.19%w/w ≡ 0.296 mol $H_2O$.

| | | | |
|---|---|---|---|
| Analysis Found: | C,59.2; | H,5.6; | N,12.5; |
| $C_{18}H_{19}N_4OF.C_4H_4O_4.0.296H_2O$ requires | C,59.0; | H,5.3; | N,12.5%. |

Example 8

2,3,4,5-Tetrahydro-6-methoxy-5-methyl-2-[(5-methyl-1 H-imidazol-4-yl)methyl]-1H-pyrido[4,3-b]indol-1-one maleate

The reaction of 2,3,4,5-tetrahydro-6-methoxy-5-methyl-1H-pyrido[4,3-b]indol-1-one (600mg) with Compound X (1.46g) gave the free base of the title compound (600mg). A portion of the free base (200mg) was treated with maleic acid to give the title compound (250mg), m.p. 158°.

Water Analysis Found: 1.64%w/w ≡ 0.4mol $H_2O$.

| | | | |
|---|---|---|---|
| Analysis Found: | C,58.9; | H,5.6; | N,12.3; |
| $C_{18}H_{20}N_4O_2.C_4H_4O_4.O.4H_2O$ requires | C,58.9; | H,5.5; | N,12.5% |

Example 9

2,3,4,5-Tetrahydro-5-methyl-2-[(5-methyl-1H-imidazol-4-yl)methyl]-6-(phenylmethoxy)-1H-pyrido[4,3-b]indol-1-one maleate

The reaction of 2,3,4,5-tetrahydro-5-methyl-1H-pyrido[4,3-b]indol-l-one (900mg) with Compound X (1.6g) gave the free base of the title compound (800mg). A portion of the free base (90mg) was treated with maleic acid to give

the title compound (90mg), m.p. 158-160°.
T.l.c. (System A, 100:8:1) Rf 0.2.

Example 10

2,3,4,5-Tetrahydro-5-methyl-2-[(5-methyl-1H-imidazol-4-yl)methyl] 1-oxo-1H-pyrido[4,3-b]indole-6-carbonitrile maleate

The reaction of 2,3,4,5-tetrahydro-5-methyl-1-oxo-1H-pyrido-[4,3-b]indole-6-carbonitrile (550mg) with Compound X (1.3g) gave the free base of the title compound (410mg). A portion of the free base (100mg) was treated with maleic acid to give the title compound (50mg), t.l.c. (System A, 100:8:1) Rf 0.2.

| | | | |
|---|---|---|---|
| Analysis Found: | C,60.4; | H,4.8; | N,15.6; |
| $C_{18}H_{17}N_5O.C_4H_4O_4$ | C,60.7; | H,4.9; | N,16.0%. |

Example 11

2,3,4,5-Tetrahydro-5-methyl-2-[(5-methyl-1H-imidazol-4-y-1)methyl]-6-phenyl-1H-pyrido[4,3-b]indol-1-one maleate

The reaction of 2,3,4,5-tetrahydro-5-methyl-6-phenyl-1H-pyrido-[4,3-b]indol-1-one (100mg) with Compound X (193mg) gave the free base of the title compound (120mg) . Maleate formation gave a solid (150mg) which was purified by HPLC (Spherisorb 55W, 25cm x 20mm column) eluting with chloroform: hexane: ethanol: 0.88 ammonia (100:50: 10:0.1) at a flow rate of 20mℓ/min to give the free base of the title compound (49mg). This was reconverted to the maleate by the method used in Example 4 to give the title compound (53mg), m.p. 215°. $^1H$-N.m.r.δ 2.37 (3H,s), 3.07 (2H,t), 3.17 (3H,s), 3.67 (2H,t), 4.67 (2H,s), 6.10 (2H,s), 6.98 (lH,dd), 7.22(1H,t), 7.4-7.55(5H,m), 8.07 (1H,brd), 8.92 (1H,s).

Example 12

6-Fluoro-2,3,4,5-tetrahydro-5-(1-methylethyl)-2-[(5-methyl-1 H-imidazol-4-yl)methyl]-1H-pyrido[4,3-b]indol-1-one maleate

The reaction of 6-fluoro-2,3,4,5-tetrahydro-5-(1-methylethyl)-1H-pyrido[4,3-b]indol-1-one (130mg) with Compound X (295mg) gave the free base of the title compound (69mg). A portion of the free base (66mg) was treated with maleic acid to give the title compound (79mg), m.p. 185-187°.

| | | | |
|---|---|---|---|
| Analysis Found: | C,60.2; | H, 5.6; | N, 12.0; |
| $C_{19}H_{21}FN_4O.C_4H_4O_4$ requires | C,60.5; | H, 5.5; | N, 12.3%. |

Example 13

5-(Cyclopentylmethyl)-6-fluoro-2,3,4,5-tetrahydro-2-[(5-methyl-1H-imidazol-4-yl)methyl]-1H-pyrido[4,3-b]indol-1-one maleate

The reaction of 5-(cyclopentylmethyl)-6-fluoro-2,3,4,5-tetrahydro-1H-pyrido[4,3-b]indol-1-one (222mg) with Compound X (317mg) gave the free base of the title compound (100mg). Maleate formation gave the title compound (85mg), m.p. 164-166°.

| | | | |
|---|---|---|---|
| Analysis Found: | C, 62.5; | H, 5.9; | N, 11.0; |
| $C_{22}H_{25}FN_4O.C_4H_4O_4$ requires | C, 62.9 | H, 5.9; | N, 11.3%. |

Example 14

6-Fluoro-2,5-dihydro-5-methyl-2-[(5-methyl-1H-imidazol-4-yl)methyl)-1H-pyrido[4,3-b]indol-1-one maleate

6-Fluoro-2,5-dihydro-5-methyl-1H-pyrido[4,3-b]indol-1-one (140mg) was reacted with Compound X (290mg) according to the method of Example 1 to give the free base of the title compound (143mg). This material was dissolved in dichloromethane:ethanol (1:1; 20mℓ) and treated with a solution of maleic acid (56mg) in ethanol (2mℓ). The solvent was removed in vacuo to leave a solid which was crystallised from ethyl acetate/methanol to give the title compound (75mg), m.p. 183-184°.

| | | | |
|---|---|---|---|
| Analysis Found: | C,59.2; | H,4.3; | N,12.9; |
| $C_{17}H_{15}FN_4O.C_4H_4O_4$ requires | C,59.2; | H,4.5; | N,13.1%. |

Example 15

6-Fluoro-2,3,4,5-tetrahydro-5-methyl-2-[(5-propyl-1H-imidazol-4-yl)-methyl]-1H-pyrido[4,3-b]indol-1-one maleate

Sodium hydride (73% dispersion in oil; 66mg); was added to a stirred suspension of 6-fluoro-2,3,4,5-tetrahydro-5-methyl-1H-pyrido[4,3-b]indol-1-one (289mg) in dry DME (30mℓ), and the mixture was heated at 60° under nitrogen for 6h. 4-(Chloromethyl)-N,N-dimethyl-5-propyl-1H-imidazole-1-sulphonamide (467mg) was then added as a suspension in DME (5mℓ) and stirring was continued at $60^0$ for 18h. 2N Hydrochloric acid (2mℓ) was then added and the mixture was heated under reflux for 5h. The mixture was poured into 8% sodium bicarbonate solution (100mℓ) and extracted with dichloromethane:ethanol (10:1; 4x50mℓ). The combined, dried organic extracts were evaporated under reduced pressure to give a solid which was purified by FCC eluting with System A (100:10:1) to give the free base of the title compound (279mg). The free base was dissolved in absolute ethanol (10mℓ) and treated with a solution of maleic acid (100mg) in absolute ethanol (2mℓ). Ether (ca. 5mℓ) was added to the solution, with cooling, to precipitate the title compound (265mg), m.p. $145-148^0$.

| | | | |
|---|---|---|---|
| Analysis Found: | C,60.6; | H,5.5; | N,12.1; |
| $C_{19}H_{21}FN_4O.C_4H_4O_4$ requires | C,60.5; | H,5.5; | N,12.3%. |

Example 16

6-Fluoro-2,3,4,5-tetrahydro-5-methyl-2-[(1,5-dimethyl-1 H-imidazol-4-yl)methyl]-1H-pyrido[4,3-b]indol-1-one

A stirred solution of 6-fluoro-2,3,4,5-tetrahydro-5-methyl-2-[(5-methyl-1H-imidazol-4-yl)methyl]-1H-pyrido[4,3-b]indol-1-one (347mg) in dry DMF was treated with sodium hydride (73% dispersion in oil; 49mg) and stirred under nitrogen for 30 min. The reaction mixture was then cooled to 0° and iodomethane (217mg) was added dropwise. The reaction mixture was then allowed to warm to room temperature, with stirring being continued for 2h. DMF was removed in vacuo and the residue was dissolved in dichloromethane (75mℓ) and washed with water (3x75mℓ) and brine (2x50mℓ). The combined aqueous washings were extracted with dichloromethane (2x50mℓ) and the combined organic extracts were dried and concentrated in vacuo to give an oil. This was dissolved in dichloromethane and adsorbed onto silica. Purification by FCC eluting with System A (200:10:1) gave a solid which was triturated with ether and then dried in vacuo to give the title compound (62mg), m.p. 174-175°.
$^1$H-N.m.r. δ 2.32 (3H,s), 3.08 (2H,t), 3.67 (2H,t), 3.69 (3H,s), 3.88 (3H,d), 4.63 (2H,s), 6.06 (2H,s), 7.02 (1H,dd), 7.12 (1H,dt), 7.78 (1H,d), 8.58 (1H,s).

Example 17

6-Fluoro-2,3,4,5-tetrahydro-2-[(5-methyl-1H-imidazol-4-yl)methyl]-5-(2-propynyl)-1H-pyrido[4,3-b]indol-1-one maleate

A stirred solution of 6-fluoro-2,3,4,5-tetrahydro-2-[[5-methy1-1-(triphenylmethyl)-1H-imidazol-4-yl]methyl]-1H-pyrido[4,3-b]indol-1-one (228mg) in dry acetone (40mℓ) and anhydrous potassium carbonate (116mg) was treated with propargyl bromide (10% v/v solution in dry acetone; 1mℓ) and heated at reflux overnight. After cooling, excess acetone was removed in vacuo, and the residue was partitioned between water (100mℓ) and ethyl acetate (100mℓ). The organic phase was washed with water (2x50mℓ) and the combined aqueons extracts were washed with ethyl acetate (50mℓ). The combined organic extracts were then concentrated in vacuo, and the residue was dissolved in a mixture of water (10mℓ), glacial acetic acid (10mℓ) and THF (15mℓ) and heated to reflux for 2h. The cooled solution was then basified with 2N sodium hydroxide (ca.100mℓ) and extracted with ethyl acetate (2xl00mℓ). The combined, dried organic extracts were adsorbed onto silica and purified by FCC eluting with System A (100:8:1) to give the free base of the title compound (95mg). This was dissolved in the minimum hot dry methanol, and maleic acid (32mg) was added. The solution was

heated and then left to cool. Ether was added to precipitate the title compound (80mg), m.p. 123-124$^0$.

| | | | |
|---|---|---|---|
| Analysis Found: | C,60.9; | H,4.7; | N,12.0; |
| $C_{19}H_{17}FN_4O.C_4H_4O_4$ requires | C,61.1; | H,4.7; | N,12.4%. |

Example 18

6-Fluoro-2,3,4,5-tetrahydro-2-[(5-methyl-1H-imidazo1-4-yl)methyl]-5-(methylsulphonyl)-1H-pyrido[4,3-b]indol-1-one b]-indol-1-one maleate

A stirred solution of 6-fluoro-2,3,4,5-tetrahydro-2-[[5-methyl-1-(triphenylmethyl)-1H-imidazol-4-yl]methyl]-1H-pyrido[4,3-b]indol-1-one (64mg) in dry DMF (10mℓ) was treated with sodium hydride (73% dispersion in oil; 6mg), and stirred under nitrogen for 30min. Methanesulphonyl chloride (10% v/v solution in DMF; 1ml) was added dropwise and the mixture was stirred for a further 45min at room temperature. Additional sodium hydride (6mg) and methanesulphonyl chloride solution (2mℓ) were then added, and stirring was continued for 2h. The mixture was then added to water (75mℓ) and extracted with ethyl acetate (3x50mℓ). The combined organic extracts were washed with water (2.50mℓ) and brine (2x50mℓ), dried and concentrated in vacuo to give a gum. This was dissolved in water (5mℓ), glacial acetic acid (5mℓ) and THF (5mℓ), and heated to reflux for 2h. The reaction mixture was then added to 2N sodium hydroxide (50mℓ) and extracted with ethyl acetate (2x50mℓ). The combined, dried organic extracts were adsorbed onto silica and purified by FCC eluting with System A (100:8:1) to give the free base of the title compound (24mg). This was triturated with ether, and then dissolved in hot methanol (2mℓ). Maleic acid (7.4mg) was added, and the resulting solution was heated and then concentrated in vacuo to give an oil. This was triturated with ether to give the title compound (20mg), m.p. 205-208°.
$^1$H-N.m.r. δ 2.33 (3H,s), 3.37 (1H,t), 3.67 (2H,t), 3.82 (3H,s), 4.67 (2H,s), 6.07 (1H,s), 7.27 (1H,dd), 7.41 (1H,dt), 7.98 (1H,d), 8.7 (1H,s).

Example 19

2,3,4,5-Tetrahydro-5-methyl-2-[(5-methyl-1H-imidazol-4-yl)methyl]-1-oxo-1H-pyrido[4,3-b]indole-6-carboxamide maleate

A mixture of 6-cyano-2,3,4,5-tetrahydro-5-methyl-2-[(5-methyl-1H-imidazol-4-yl)methyl]-1H-pyrido[4,3-b]indol-1-one (100mg), Amberlite resin IRA 400 (OH) [made by stirring Amberlite resin IRA 400 (Cl) (0.88g) in 2N sodium hydroxide (5mℓ) at 21° for 2½h, then filtering and washing with water (ca. 50mℓ) until the water becomes neutral] and water (15mℓ) was heated at reflux for 36h. After this time, the water had evaporated and the resultant residue was stirred in hot ethanol (200mℓ). The solid was then filtered off, placed in a Soxhlet and extracted with ethanol (250mℓ) overnight. The combined ethanolic extracts were concentrated in vacuo and the residue was purified by FCC eluting with System A (100:8:1) to give the free base of the title compound (80mg). A solution of the free base (80mg) in methanol (50mℓ) was treated with maleic acid (27mg) and heated on a steam bath for 5min. The solution was concentrated in vacuo and a solution of the residue in methanol (10mℓ) was treated with ether (ca. 80mℓ) to precipitate the title compound (70mg), m.p. 238°.
$^1$H-N.m.r. δ 2.37 (3H,s), 3.13 (2H,t), 3.67 (2H,t), 3.72 (3H,s), 4.67 (2H,s), 6.07 (2H,s), 7.1-7.3 (2H,m), 8.08 (1H,dd), 7.67 and 8.13 (2H, 2 x brs), 8.87 (1H,s).

Example 20

Methyl 2,3,4,5-tetrahydro-5-methyl-2-[(5-methyl-1H-imidazol-4-yl)methyl]-1-oxo-1H-pyrido[4,3-b]indole-6-propanoate maleate

A solution of methyl 3-[2,3,4,5-tetrahydro-5-methyl-2-[(5-methyl-1H-imidazol-4-yl)methyl]-1-oxo-1H-pyrido[4,3-b]indol-6-yl]-2-propenoate 200mg) in ethanol (50ml) was hydrogenated at room temperature and atmospheric pressure over 10% palladium on carbon catalyst (50% aqueous paste; 20mg) for 18h. The mixture was then filtered, concentrated in vacuo, and the residue was purified by FCC eluting with System A (100:8:1) to give the free base of the title compound (70mg). A portion of the free base (60mg) in methanol (10mℓ) was treated with maleic acid (18mg), then heated on a steam bath for 10min. The resultant solution was concentrated in vacuo and the residue was triturated with ether (10mℓ) to give the title compound (70mg), m.p. 205$^0$.
T.l.c. (System A, 100:8:1) Rf 0.3.

Example 21

2,3,4,5-Tetrahydro-6-hydroxy-5-methyl-2-[(5-methyl-1 H-imidazol-4-yl)methyl]-1H-pyrido[4,3-b]indol-1-one maleate

A solution of 2,3,4,5-tetrahydro-5-methyl-2-[(5-methyl-1H-imidazol-4-yl)methyl]-6-(phenylmethoxy)-1H-pyrido [4,3-b]indol-1-one (500mg) in absolute ethanol (100mℓ) was hydrogenated at room temperature and atmospheric pressure over 10% palladium on carbon catalyst (50% aqueous paste; 100mg) for 6h. The mixture was then filtered, concentrated in vacuo, and the resultant solid was recrystallised from ethanol (ca. 50mℓ) to give the free base of the title compound (90mg). Maleate formation using the method described in Example 20 gave the title compound (60mg), m. p. 200$^0$.
$^1$H-N.m.r. δ 2.37 (3H,s), 3.06 (2H,t), 3.65 (2H,t), 3.97 (3H,s), 4.65 (2H,s), 6.08 (2H,s), 6.58 (1H,d), 6.92 (1H,t), 7.45 (1H,d), 8.89 (1H,s).

Example 22

6-Fluoro-2,3,4,5-tetrahydro-2-[(5-methyl-1H-imidazol-4-yl)methyl]-1 H-pyrido[4,3-b]indol-1-one maleate

A solution of 6-fluoro-2,3,4,5-tetrahydro-2-[(5-methyl-1H-imidazol-4-yl)-methyl]-5-(phenylmethoxymethyl)-1H-pyrido[4,3-b]indol-1-one (175mg) in absolute ethanol (10ml) and acetic acid (2.5ml) was hydrogenated at room temperature and atmospheric pressure over 10% palladium on carbon catalyst (50% aqueous paste; 45mg) in absolute ethanol (2ml) for 20h. More catalyst (45mg) was added and stirring was continued for 20h. The mixture was filtered and the filtrate was evaporated to dryness. The residue was treated with 8% sodium bicarbonate solution (50ml) and extracted with dichloromethane (3x25ml). The combined, dried organic extracts were evaporated to give an oil (ca. 130mg) which was purified by FCC eluting with System A (150:10:1) to give the free base of the title compound (102mg). This was dissolved in ethanol (ca. 2ml) and treated with a solution of maleic acid (42mg) in ethanol (0.5ml). The solvent was removed in vacuo and the residue was triturated with dry ether (5x5ml) to give the title compound (120mg), m.p. 186-188°.

| | | | |
|---|---|---|---|
| Analysis Found : | C,57.8; | H,4.7; | N,13.2; |
| $C_{16}H_{15}FN_4O.C_4H_4O_4$ requires | C,58.0; | H,4.6; | N,13.5%. |

Example 23

2,3,4,5-Tetrahydro-6-hydroxy-2-[(5-methyl-1H-imidazol-4-yl)methyl]-1 H-pyrido[4,3-b]indol-1-one maleate

A solution of 2,3,4,5-tetrahydro-2-[(5-methyl-1H-imidazol-4-yl)methyl]-6-(phenylmethoxy)-5-[(phenylmethoxy)methyl]-1H-pyrido[4,3-b]-indol-1-one (600mg) in absolute ethanol (80mℓ) and glacial acetic acid (4mℓ) was hydrogenated at room temperature and atmospheric pressure over 10% palladium on carbon catalyst (50% aqueous paste; 100mg) for 24h. The mixture was then filtered and the filtrate was concentrated in vacuo. The residue was treated with 8% sodium bicarbonate solution (ca. 150mℓ) and filtered. The filtered solid was then washed with water (ca. 100mℓ), dissolved in ethanol (200mℓ) and concentrated in vacuo to give a solid (320mg) which was purified by FCC eluting with System A (100:8:1) to give the free base of the title compound (100mg). A solution of the free base (100mg) in methanol (20mℓ) was treated with maleic acid (39mg). The solution was heated on a steam bath for 10 min and concentrated in vacuo. A solution of the residue in methanol (2mℓ) was treated with ether (ca. 80mℓ) to give the title compound (100mg), m.p. 130°.
T.l.c. (System A, 100:8:1)(2 x elution) Rf 0.3.

Example 24

6-Fluoro-2,3,4,5-tetrahydro-5-methyl-2-[(5-methyl-1H-imidazol-4-yl)methyl)-1H-pyrido[4,3-b]indol-1-one hydrochloride

A solution of 6-fluoro-2,3,4,5-tetrahydro-5-methyl-2-[(5-methyl-1H-imidazol-4-yl)methyl]-1H-pyrido[4,3-b]indol-1-one (260mg) in methanol (10ml) was treated with ethereal hydrogen chloride and the mixture was then concentrated in vacuo. The residue was triturated with ether (15ml) to give the title compound (230mg) as a solid, m.p. 275-278°. Water Analysis Found 3.73% w/w ≡ 0.75mol $H_2O$.

| | | | |
|---|---|---|---|
| Analysis Found | C,55.8; | H,5.3; | N,15.2; |
| $C_{17}H_{17}FN_4O.HCl.0.75H_2O$ requires | C,56.3; | H,5.4; | N,15.4%. |

Example 25

6-Fluoro-2,3,4,5-tetrahydro-5-methyl-2-[(5-methyl-1H-imidazol-4-yl)methyl]-1H-pyrido[4,3-b]indol-1-one benzoate

A solution of 6-fluoro-2,3,4,5-tetrahydro-5-methyl-2-[(5-methyl-1H-imidazol-4-yl)methyl]-1H-pyrido[4,3-b]indol-1-one (170mg) in methanol (10mℓ) was treated with benzoic acid (66mg). Ether (ca. 20ml) was added to precipitate a solid which was filtered off to give the title compound (220mg), m.p. 169-171°.

| | | | |
|---|---|---|---|
| Analysis Found: | C,66.3; | H,5.4; | N,12.8; |
| $C_{17}H_{17}FN_4O.C_7H_6O_2$ requires | C,66.3; | H,5.3; | N,12.9%. |

Example 26

6-Fluoro-2,3,4,5-tetrahydro-5-methyl-2-[(5-methyl-1H-imidazol-4-yl)methyl]-1H-pyrido[4,3-b]indol-1-one

A solution of 6-fluoro-2,3,4,5-tetrahydro-5-methyl-1H-pyrido[4,3-b]indol-1-one (100mg) in N-methylpyrrolidinone (10mℓ) was treated with 4-toluenesulphonic acid monohydrate (17mg) and 4-hydroxymethyl-5-methylimidazole hydrochloride (37mg). The mixture was then heated to 125° for 18h during which time three further portions of 4-hydroxymethyl-5-methylimidazole hydrochloride (37mg) were added at 1,2 and 3h respectively. The solution was then poured into 8% sodium bicarbonate solution (100mℓ) and extracted with dichloromethane (3x100 mℓ). The combined extracts were concentrated in vacuo and the N-methylpyrrolidinone was distilled at 100°. The residue was purified by FCC eluting with System A (200:8:1) to give the title compound (100mg), t.l.c. (System A, 100:8:1) Rf 0.3.
$^1$H-N.m.r. δ 2.36 (3H,s), 3.12 (2H,t), 3.67 (2H,t), 3.90 (3H,s), 4.66 (2H,s), 6.07 (2H,s), 6.95-7.2 (2H,m), 7.80 (1H,d), 8.80 (1H,s).

Example 27

6-Fluoro-2,3,4,5-tetrahydro-5-methyl-2-[(5-methyl-1H-imidazol-4-yl)methyl]-1H-pyrido[4,3-b]indol-1-one

A mixture of 6-fluoro-2,5-dihydro-5-methyl-2-[(5-methyl-1H-imidazol-4-yl)methyl]-1H-pyrido[4,3-b]indol-1-one (150mg) and 10% palladium on carbon catalyst (50% aqueous paste; 75mg) in absolute ethanol (30mℓ) was hydrogenated at 80° and 80 p.s.i. for 24h. The resulting suspension was filtered, and the filtrate evaporated to give a solid (190mg) which was purified by HPLC (Spherisorb 55W, 25cm × 20mm column eluting with hexane:ethanol: dichloromethane: 0.88 ammonia solution (65:25:10:1) to give the title compound (21mg), m.p. 231-233°.

The $^1$H-n.m.r. data obtained for this material were consistent with those obtained for the product of Example 26.

Example 28

6-Fluoro-2,3,4,5-tetrahydro-5-methyl-2-[(5-methyl-1H-imidazol-4-yl)methyl-1H-pyrido[4,3-b)indol-1-one

4-[2-(2-Fluorophenyl)-2-methylhydrazino]-5,6-dihydro-1-[(5-methyl-1H-imidazol-4-yl)methyl]-2(1H)-pyridinone (65mg) was treated with concentrated sulphuric acid (0.5mℓ) and left standing for 5 min. The solution was then neutralised with 8% sodium bicarbonate solution (30mℓ) and extracted with dichloromethane:ethanol (5:1) (3x15mℓ). The combined, dried organic extracts were evaporated to give an oil (52mg) )which was purified by FCC eluting with System A (200:10:1) to give the title compound (3mg), t.l.c. (System A, 200:10:1) Rf 0.28.
The $^1$H-n.m.r. data obtained for this material were consistent with those obtained for the product of Example 26.

Example 29

6-Fluoro-2,3,4,5-tetrahydro-5-methyl-2-[(5-methyl-1H-imidazol-4-yl)methyl]-1H-pyrido[4,3-b]indol-1-one

A mixture of 6-fluoro-2,3,4,5-tetrahydro-5-methyl-2-[(4-methyloxazol-5-yl)methyl]-1H-pyrido[4,3-b]indol-1-one (180mg) and formamide (20mℓ) was heated at 180° for 18h. The solution was then cooled (0°), diluted with water

(100mℓ) and extracted with dichloromethane (3x100mℓ). The combined extracts were concentrated in vacuo and purified by FCC eluting with System A (100:8:1) to give the title compound (110mg), m.p. 230-233°.
The $^1$H-n.m.r. data obtained for this material were consistent with those obtained for the product of Example 26.

Example 30

6-Fluoro-2,3,4,5-tetrahydro-5-methyl-2-[(5-methyl-1H-imidazol-4-yl)-methyl]-1 H-pyrido[4,3-b]indol-1-one

A stirred solution of 4-[(6-fluoro-2,3,4,5-tetrahydro-5-methyl-1-oxo-1H-pyrido[4,3-b]indol-2-yl)methyl]-N,N,5-trimethyl-1H-imidazole-1-sulphonamide (92mg) in absolute ethanol (5mℓ) was treated with 2N hydrochloric acid (20mℓ) and stirred under nitrogen at 100-110° for 6h, and then cooled. 2N Sodium hydroxide (60mℓ) was added, and the mixture was extracted with ethyl acetate (2x75mℓ).

The combined, dried organic extracts were concentrated in vacuo to give a solid (71mg) which was purified by FCC eluting with System A (100:8:1) to give the title compound (57mg), m.p. 233-234°.
The $^1$H-n.m.r. data obtained for this material were consistent with those obtained for the product of Example 26.

Example 31

6-Fluoro-2,3,4,5-tetrahydro-5-methyl-2-[(5-methyl-1H-imidazol-4-yl)methyl]-1H-pyrido[4,3-b]indol-1-one

A solution of 2,3-dichloro-5,6-dicyano-1,4-benzoquinone (133mg) in THF (4mℓ) was added dropwise to a stirred solution of 6-fluoro-2,3,4,5-tetrahydro-5-methyl-2-[(5-methyl-1H-imidazol-4-yl)methyl]-1H-pyrido[4,3-b]indole (70mg) in THF (12mℓ) at -10° under nitrogen, and stirring was continued for 4h. Water (20mℓ) was then added, and the resulting solution was left to stand overnight. Excess THF was removed in vacuo, and the resultant aqueous solution was extracted with ethyl acetate (3x50mℓ). The combined, dried organic extracts were adsorbed onto silica and purified by FCC eluting with System A (100:10:1) to give the title compound (15mg), m.p. 231.5-233°.

The $^1$H-n.m.r. data obtained for this material were consistent with those obtained for the product of Example 26.

Example 32

6-Fluoro-2,3,4,5-tetrahydro-5-methyl-2-[(5-methyl-1H-imidazol-4-yl)methyl]-1H-pyrido[4,3-b]indol-1-one

A stirred solution of 6-fluoro-2,3,4,5-tetrahydro-2-[[5-methyl-1-(triphenylmethyl)-1H-imidazol-4-yl]methyl]-1H-pyrido[4,3-b]indol-1-one (100mg) in dry DMF (15mℓ) was treated with sodium hydride (73.2% dispersion in oil; 10.5mg) and stirred under nitrogen at room temperature for 20min. Methyl iodide (2.3% v/v solution in dry DMF; 1ml) was added and the solution was stirred for a further 20min. The reaction mixture was then added to water (100mℓ) and extracted with ethyl acetate (2x50mℓ). The combined organic extracts were washed with water (2x100ml), dried and concentrated in vacuo to give a solid. This solid was dissolved in THF (10mℓ), water (10mℓ) and glacial acetic acid (10mℓ) and heated at reflux for 3h. THF was removed in vacuo, and the remaining solution was added to 2N sodium hydroxide (pH→14) and extracted with ethyl acetate (3x50mℓ). The combined, dried organic extracts were adsorbed onto silica and purified by FCC eluting with System A (100:8:1) to give the title compound (45mg), m.p. 234-235°.
The $^1$H-n.m.r. data obtained for this material were consistent with those obtained for the product of Example 26.

Example 33

6-Fluoro-2,3,4,5-tetrahydro-5-methyl-2-[(5-methyl-1H-imidazol-4-yl)methyl]-1H-pyrido[4,3-b]indol-1-one

A solution of 6-fluoro-2,3,4,5-tetrahydro-2-[[1-(methoxymethyl)-5-methyl-1H-imidazol-4-yl]methyl]-5-methyl-1H-pyrido[4,3-b]indol-1-one and 6-fluoro-2,3,4,5-tetrahydro-2-[[1-(methoxymethyl)-4-methyl-1H-imidazol-5-yl]methyl]-5-methyl-1H-pyrido[4,3-b]indol-1-one (32mg) in 47% aqueous hydrobromic acid (4mℓ) was heated on a steam bath for ca. 2h. After cooling, the reaction mixture was added to 2N sodium hydroxide (50mℓ) and extracted with ethyl acetate (2x50mℓ). The combined, dried organic extracts were adsorbed onto silica and purified by FCC eluting with System A (100:8:1) to give the title compound (6.5mg), m.p. 229-232°.
The $^1$H-n.m.r. data obtained for this material were consistent with those obtained for the product of Example 26.

Example 34

6-Fluoro-2,3,4,5-tetrahydro-5-methyl-2-[(5-methyl-1H-imidazol-4-yl)methyl]-1H-pyrido[4,3-b]indol-1-one

A solution of phenylmethyl 4-[(6-fluoro-2,3,4,5-tetrahydro-5-methyl-1-oxo-1H-pyrido[4,3-b]indol-2-yl)methyl]-5-methyl-1H-imidazole-1-carboxylate (100mg) in ethanol (20mℓ) and 2N hydrochloric acid (10mℓ) was heated on a steam bath for 10 min. The cooled reaction mixture was then added to water (5mℓ) and 2N sodium hydroxide (20 mℓ), and extracted with ethyl acetate (2x75mℓ). The combined, dried organic extracts were adsorbed onto silica and purified aby FCC eluting with System A (100:8:1) to give a solid which was triturated with ether to give the title compound (50mg), m.p. 232-233°.

The $^1$H-n.m.r. data obtained for this material were consistent with those obtained for the product of Example 26.

The following examples illustrate pharmaceutical formulations according to the invention, containing 6-fluoro-2,3,4,5-tetrahydro-5-methyl-2-[(5-methyl-1H-imidazol-1-yl)methyl]-1H-pyrido[4,3-b]indol-1-one as the active ingredient. Physiologically acceptable salts and/or solvates of this compound, and other compounds of formula (I) and their physiologically acceptable salts and/or solvates may be formulated in a similar manner.

TABLETS FOR ORAL ADMINISTRATION

Tablets may be prepared by the normal methods such as direct compression or wet granulation.

The tablets may be film coated with suitable film forming materials, such as hydroxypropyl methylcellulose, using standard techniques. Alternatively the tablets may be sugar coated.

Direct Compression

| Tablet | mg/tablet |
|---|---|
| Active Ingredient | 1.00 |
| Anhydrous Lactose USNF | 79.00 |
| Microcrystalline Cellulose USNF | 19.55 |
| Magnesium Stearate BP | 0.45 |
| Compression weight | 100.00 |

* of a grade suitable for direct compression.

The active ingredient is passed through a 60 mesh sieve, blended with the anhydrous lactose, microcrystalline cellulose and magnesium stearate. The resultant mix is compressed into tablets using a suitable tablet machine fitted with 5.5mm diameter punches.

Tablets of other strengths may be prepared by altering the ratio of active ingredient to lactose or the compression weight and using punches to suit.

| Sub-Lingual Tablet | mg/tablet |
|---|---|
| Active Ingredient | 1.0 |
| Compressible Sugar NF | 64.0 |
| Magnesium Stearate BP | 0.5 |
| Compression Weight | 65.5 |

The active ingredient is sieved through a suitable sieve, blended with the excipients and compressed using suitable punches.

Tablets of other strengths may be prepared by altering either the ratio of active ingredient to excipients or the compression weight and using punches to suit.

Wet Granulation

| Conventional Tablet | mg/tablet |
|---|---|
| Active Ingredient | 1.0 |
| Lactose BP | 153.0 |
| Starch BP | 30.0 |
| Pregelatinised Maize Starch BP | 15.0 |
| Magnesium Stearate BP | 1.5 |
| Compression Weight | 200.5 |

The active ingredient is sieved through a suitable sieve and blended with lactose, starch and pregelatinised maize starch. Suitable volumes of purified water are added and the powders are granulated. After drying, the granules are screened and blended with the magnesium stearate. The granules are then compressed into tablets using 7mm diameter punches.

Tablets of other strengths may be prepared by altering the ratio of active ingredient to lactose or the compression weight and using punches to suit.

| Sub-Lingual Tablet | mg/tablet |
|---|---|
| Active Ingredient | 1.0 |
| Mannitol BP | 58.0 |
| Hydroxypropylmethylcellulose | 5.0 |
| Magnesium Stearate BP | 1.0 |
| Compression Weight | 65.0 |

The active ingredient is sieved through a suitable sieve and blended with the mannitol and hydroxypropylmethylcellulose. Suitable volumes of purified water are added and the powders are granulated. After drying, the granules are screened and blended with the magnesium stearate. The granules are compressed into tablets using suitable punches.

Tablets of other strengths may be prepared by altering the ratio of active ingredient to mannitol or the compression weight and punches to suit.

| CAPSULES | mg/capsule |
|---|---|
| Active Ingredient | 1.0 |
| * Starch 1500 | 98.0 |
| Magnesium Stearate BP | 1.0 |
| Fill Weight | 100.0 |

* a form of directly compressible starch.

The active ingredient is sieved and blended with the excipients. The mix is filled into size No. 2 hard gelatin capsules using suitable machinery. Other doses may be prepared by altering the fill weight and if necessary changing the capsule size to suit.

In the above examples of tablets and capsules, when the active ingredient is included as a suitable salt, the quantity of the major excipient used is adjusted accordingly.

SYRUP

This may be either a sucrose or sucrose free presentation.

| A. Sucrose Syrup | | mg/5ml dose |
|---|---|---|
| Active Ingredient | | 1.0 |
| Sucrose BP | | 2750.0 |
| Glycerine BP | | 500.0 |
| Buffer ) Flavour ) Colour ) Preservative ) | | as required |
| Purified Water BP | to | 5.0ml |

The active ingredient, buffer, flavour, colour and preservative are dissolved in some of the water and the glycerine is added. The remainder of the water is heated to dissolve the sucrose and is then cooled. The two solutions are combined, adjusted to volume and mixed. The syrup is clarified by filtration.

| B. Sucrose-Free | | mg/5ml dose |
|---|---|---|
| Active Ingredient | | 1.0 |
| Hydroxypropylmethylcellulose USP (viscosity type 4000) | | 22.5 |
| Buffer ) Flavour ) Colour ) Preservative ) Sweetener ) | | as required |
| Purified Water BP | to | 5.0ml |

The hydroxypropylmethylcellulose is dispersed in hot water, cooled and then mixed with an aqueous solution containing the active ingredient and the other components of the formulation. The resultant solution is adjusted to volume and mixed. The syrup is clarified by filtration.

INJECTION FOR INTRAVENOUS ADMINISTRATION

| | mg/mℓ | |
|---|---|---|
| Active ingredient | 0.05 | 0.5 |
| Sodium Chloride BP | as required | as required |
| Water for Injection BP to | 1.0mℓ | 1.0mℓ |

Sodium chloride may be added to adjust the tonicity of the solution and the pH may be adjusted, using acid or alkali, to that of optimum stability and/or facilitate solution of the active ingredient. Alternatively, suitable buffer salts may be used.

The solution is prepared, clarified and filled into appropriate size ampoules sealed by fusion of the glass. The injection is sterilised by heating in an autoclave using one of the acceptable cycles. Alternatively, the solution may be sterilised by filtration and filled into sterile ampoules under aseptic conditions. The solution may be packed under an inert atmosphere of nitrogen or other suitable gas.

METERED DOSE PRESSURISED AEROSOL

| Suspension Aerosol | mg/metered dose | Per can |
|---|---|---|
| Active Ingredient micronised | 0.050 | 12.0mg |
| Lecithin USNF | 0.020 | 4.80mg |
| Trichlorofluoromethane BP | 23.64 | 5.67g |
| Dichlorodifluoromethane BP | 61.25 | 14.70g |

The active ingredient is micronised in a fluid energy mill to a fine particle size range. The lecithin is mixed with the trichlorofluoromethane at a temperature of 10-15°C and the micronised drug is mixed into the solution with a high shear mixer. The suspension is metered into aluminium aerosol cans and suitable metering valves, delivering 85mg of suspension are crimped onto the cans and the dichlorodifluoromethane is pressure filled into the cans through the valves.

Solution Aerosol

| | mg/metered dose | Per can |
|---|---|---|
| Active Ingredient | 0.05 | 12.0mg |
| Ethanol BP | 7.500 | 1.80g |
| Trichlorofluoromethane BP | 18.875 | 4.53g |
| Dichlorodifluoromethane BP | 48.525 | 11.65g |

Oleic acid BP, or a suitable surfactant e.g. Span 85 (sorbitan trioleate) may also be included).

The active ingredient is dissolved in the ethanol together with the oleic acid or surfactant if used. The alcoholic solution is metered into suitable aerosol containers followed by the trichlorofluoromethane. Suitable metering valves are crimped onto the containers and dichlorodifluoromethane is pressure filled into them through the valves.

Inhalation Cartridges

| | mg/cartridge |
|---|---|
| Active Ingredient (micronised) | 0.05 |
| Lactose BP to | 25.00 |

The active ingredient is micronised in a fluid energy mill to a fine particle size range prior to blending with normal tabletting grade lactose in a high energy mixer. The powder blend is filled into No. 3 hard gelatin capsules on a suitable encapsulating machine. The contents of the cartridges are administered using a powder inhaler.

SUPPOSITORY

| | |
|---|---|
| Active Ingredient | 1.0mg |
| * Witepsol H15 to | 1.0g |

* Witepsol H15 is a proprietary grade of Adeps Solidus Ph. Eur.

A suspension of the active ingredient is prepared in the molten Witepsol and filled, using suitable machinery, into 1g size suppository moulds.

## Claims

**Claims for the following Contracting States : AT, BE, CH, DE, FR, GB, IT, LI, LU, NL, SE**

1. Compounds of the general formula (I)

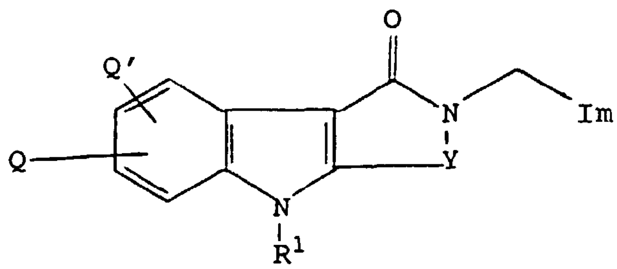

(I)

wherein Im represents an imidazolyl group of the formula:

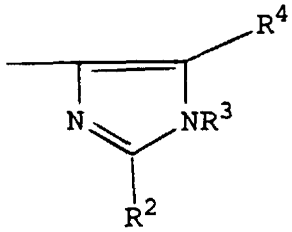

or

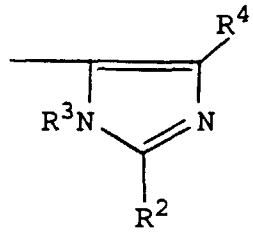

and $R^1$ represents a hydrogen atom or a group selected from $C_{1-6}$alkyl, $C_{3-6}$alkenyl, $C_{3-10}$alkynyl, $C_{3-7}$cycloalkyl, $C_{3-7}$cycloalkyl$C_{1-4}$alkyl-, phenyl, phenyl$C_{1-3}$alkyl-, $-COR_2R^5$,$COR^5$, $-CONR^5R^6$ or $-SO_2R^5$ (wherein $R^5$ and $R^6$, which may be the same or different, each represents a hydrogen atom, a $C_{1-6}$alkyl or $C_{3-7}$cycloalkyl group, or a phenyl or phenyl$C_{1-4}$alkyl- group, in which the phenyl group is optionally substituted by one or more $C_{1-4}$alkyl, $C_{1-4}$alkoxy or hydroxy groups or halogen atoms, with the proviso that $R^5$ does not represent a hydrogen atom when $R^1$ represents a group $-CO_2R^5$ or $-SO_2R^5$);
one of the groups represented by $R^2$, $R^3$ and $R^4$ is a hydrogen atom or a $C_{1-6}$alkyl, $C_{3-7}$cycloalkyl, $C_{3-6}$alkenyl, phenyl or phenyl$C_{1-3}$alkyl- group, and each of the other two groups, which may be the same or different, represents a hydrogen atom or a $C_{1-6}$alkyl group;
Y represents the group CH=CH or $(CH_2)_n$, wherein n represents 2 or 3;
Q represents a halogen atom, or a group selected from hydroxy, $C_{1-4}$alkoxy, phenyl$C_{1-3}$alkoxy-, $C_{1-6}$alkyl, cyano, phenyl which may be unsubstituted or substituted by one or more $C_{1-4}$alkyl, $C_{1-4}$alkoxy or hydroxy groups or halogen atoms, $-NR^7R^8$, $-CONR^7R^8$ or $-(CH_2)_pCONR^7R^8$ (wherein $R^7$ and $R^8$, which may be the same or different, each represents a hydrogen atom or a $C_{1-4}$alkyl or $C_{3-4}$alkenyl group, or together with the nitrogen atom to which they are attached form a saturated 5 to 7 membered ring; and p represents 1, 2 or 3), $-(CH_2)_qNR^9R^{10}$ (wherein $R^9$ represents a hydrogen atom or a $C_{1-4}$alkyl group, and $R^{10}$ represents a group $-COR^{11}$ or $-SO_2R^{11}$ wherein $R^{11}$ represents a $C_{1-4}$alkyl group; and q represents 0, 1, 2 or 3), or $-(CH_2)_2Co_2R^{11}$ ($R^{11}$ being as defined previously);
Q' represents a hydrogen or a fluorine atom;

and physiologically acceptable salts and solvates thereof.

2. Compounds as claimed in claim 1 in which $R^1$ represents a hydrogen atom or a $C_{1-6}$alkyl, $C_{3-4}$alkynyl, $C_{4-6}$cycloalkylmethyl, or $C_{1-3}$alkylsulphonyl group.

3. Compounds as claimed in claim 1 or 2 in which $R^2$, $R^3$ and $R^4$ each independently represent a hydrogen atom or a $C_{1-4}$alkyl group.

4. Compounds as claimed in any of claims 1 to 3 in which Q represents a halogen atom or a hydroxy, $C_{1-4}$alkoxy, phenyl$C_{1-3}$alkoxy-, $C_{1-6}$alkyl, cyano, phenyl, $-CONH_2$, or $-(CH_2)_2CO_2CH_3$ group.

5. Compounds as claimed in any of claims 1 to 3 in which Q represents a halogen atom, or a hydroxy,

phenyl$C_{1-3}$alkoxy-, $C_{1-3}$alkyl or cyano group.

**6.** Compounds as claimed in any of claims 1 to 3 in which Q represents a fluorine atom.

**7.** Compounds as claimed in claim 1 in which $R^1$ represents a hydrogen atom or a $C_{1-4}$alkyl, $C_{3-4}$alkynyl or $C_{4-6}$cycloalkylmethyl group; $R^2$ and $R^3$ each represent a hydrogen atom; $R^4$ represents a $C_{1-4}$alkyl group; and Q represents a halogen atom or a hydroxy, phenyl$C_{1-3}$alkoxy-, $C_{1-3}$alkyl or cyano group.

**8.** Compounds as claimed in any of claims 1 to 7 in which Y represents the group $(CH_2)_2$ and Q' is a hydrogen atom.

**9.** 6-Fluoro-2,3,4,5-tetrahydro-5-methyl-2-[(5-methyl-1H-imidazol-4-yl)methyl]-1H-pyrido[4,3-b]indol-1-one and physiologically acceptable salts and solvates thereof.

**10.** 2,3,4,5-Tetrahydro-5,6-dimethyl-2-[(5-methyl-1H-imidazol-4-yl)-methyl]-1H-pyrido[4,3-b]indol-1-one; 6,9-difluoro-2,3,4,5-tetrahydro-5-methyl-2-[(5-methyl-1H-imidazol-4-yl)methyl]-1H-pyrido[4,3-b]indol-1-one; 6-fluoro-2,3,4,5-tetrahydro-2-[(5-methyl-1H-imidazol-4-yl)methyl]-5-(2-propynyl)-1H-pyrido[4,3-b]indol-1-one; 2,3,4,5-tetrahydro-5-methyl-2-[(5-methyl-1H-imidazol-4-yl)methyl]-1-oxo-1H-pyrido[4,3-b]indole-6-carbonitrile; and physiologically acceptable salts and solvates thereof.

**11.** A process for the preparation of compounds of general formula (I) as defined in any of claims 1 to 10 or a physiologically acceptable salt or solvate thereof, which comprises:

(A) alkylating a compound of formula (II)

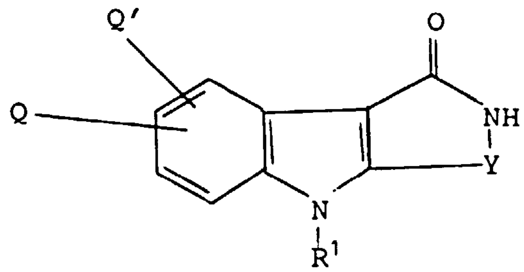

(II)

with a compound of formula (III)

X -Im (III)

or a protected derivative thereof, wherein X represents a group $-CH_2L$ and L represents a leaving atom or group and the reaction is effected in the presence of a base; or X represents the group $-CH_2OH$ and the reaction is effected in the presence of an acid at an elevated temperature followed if necessary by removal of any protecting groups present; or

(B) for the preparation of a compound of formula (I) in which Y represents $(CH_2)_n$, cyclising a compound of formula (IV)

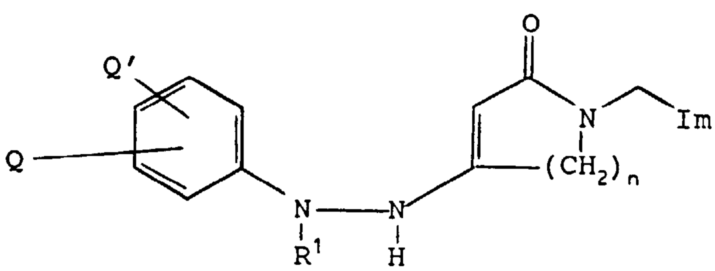

(IV)

or a salt or protected derivative thereof, followed if necessary by removal of any protecting groups present; or

(C) for the preparation of a compound of formula (I) in which $R^3$ represents a hydrogen atom, reacting a compound of formula (V)

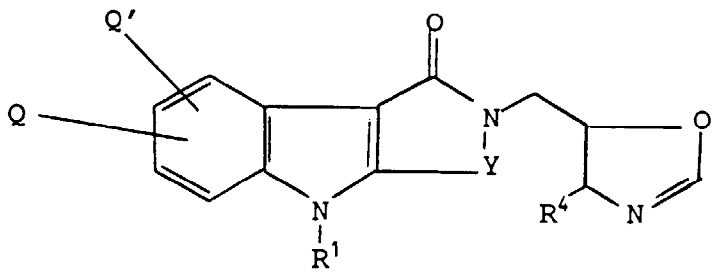

(V)

or protected derivative thereof, with formamide, followed if necessary by removal of any protecting groups present; or

(D) for the preparation of a compound of formula (I) in which Y represents $(CH_2)_n$, reacting a compound of formula (VI)

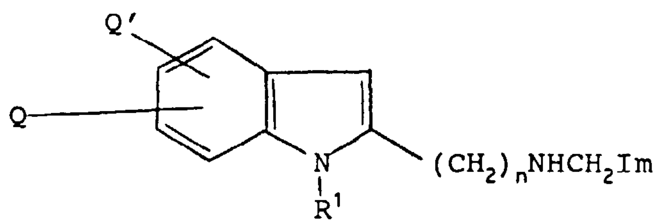

(VI)

or a protected derivative thereof, with phosgene in the presence of a Lewis acid, followed if necessary by removal of any protecting groups present; or

(E) for the preparation of a compound of formula (I) in which Y represents $(CH_2)_n$, oxidising a compound of formula (VII)

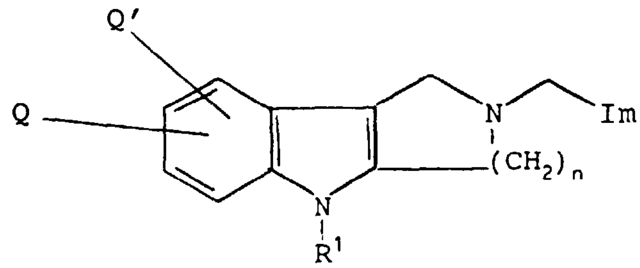

(VII)

or a protected derivative thereof, followed if necessary by removal of any protecting groups present; or

(F) converting a compound of general formula (I) into another compound of formula (I) by hydrogenation alkylation or acylation; or

(G) removing protecting group(s) from a protected form of a compound of formula (I);

and when the compound of formula (I) is obtained as a mixture of enantiomers, optionally resolving the mixture to obtain the desired enantiomer;
and/or where the compound of formula (I) is in the form of a free base, optionally converting the free base into a salt.

**12.** A pharmaceutical composition comprising at least one compound of general formula (I) as defined in claim 1 or a physiologically acceptable salt or solvate thereof together with at least one physiologically acceptable carrier or excipient.

**13.** A pharmaceutical composition as claimed in claim 12 in a form adapted for oral or parenteral administration.

**14.** A pharmaceutical composition as claimed in claim 12 or 13 wherein the active ingredient is 6-fluoro-2,3,4,5-tetrahydro-5-methyl-2-[(5-methyl-1H-imidazol-4-yl)methyl]-1H-pyrido[4,3-b]indol-1-one or a physiologically acceptable salt or solvate thereof.

**15.** A compound of formula (I) as defined in any of claims 1 to 10 or a physiologically acceptable salt or solvate thereof for use as an active therapeutic agent.

**16.** The use of a compound of formula (I) as defined in any of claims 1 to 10 or a physiologically acceptable salt or

solvate thereof for the manufacture of a medicament for the treatment of a condition mediated through 5-$HT_3$ receptors.

**17.** The use of a compound of formula (I) as defined in any of claims 1 to 10 or a physiologically acceptable salt or solvate thereof for the manufacture of a medicament for the treatment of nausea and vomiting.

**Claims for the following Contracting States : GR, ES**

**1.** A process for the preparation of compounds of the general formula (I)

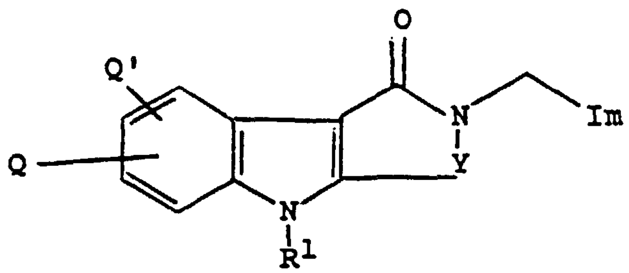

(I)

wherein Im represents an imidazolyl group of the formula:

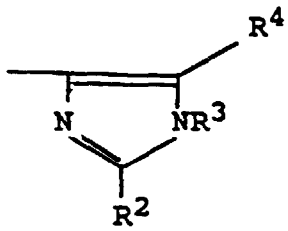

or

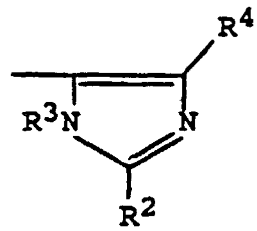

and $R^1$ represents a hydrogen atom or a group selected from $C_{1-6}$alkyl, $C_{3-6}$alkenyl, $C_{3-10}$alkynyl, $C_{3-7}$cycloalkyl, $C_{3-7}$cycloalkyl$C_{1-4}$alkyl-, phenyl, phenyl$C_{1-3}$alkyl-, -$CO_2R^5$, -$COR^5$, -$CONR^5R^6$ or -$SO_2R^5$ (wherein $R^5$ and $R^6$, which may be the same or different, each represents a hydrogen atom, a $C_{1-6}$alkyl or $C_{3-7}$cycloalkyl group, or a phenyl or phenyl$C_{1-4}$alkyl- group, in which the phenyl group is optionally substituted by one or more $C_{1-4}$alkyl, $C_{1-4}$alkoxy or hydroxy groups or halogen atoms, with the proviso that $R^5$ does not represent a hydrogen atom when $R^1$ represents a group -$CO_2R^5$ or -$SO_2R^5$);
one of the groups represented by $R^2$, $R^3$ and $R^4$ is a hydrogen atom or a $C_{1-6}$alkyl; $C_{3-7}$cycloalkyl, $C_{3-6}$alkenyl, phenyl or phenyl$C_{1-3}$alkyl- group, and each of the other two groups, which may be the same or different, represents a hydrogen atom or a $C_{1-6}$alkyl group;
Y represents the group CH=CH or $(CH_2)_n$, wherein n represents 2 or 3;
Q represents a halogen atom, or a group selected from hydroxy, $C_{1-4}$alkoxy, phenyl$C_{1-3}$alkoxy-, $C_{1-6}$alkyl, cyano, phenyl which may be unsubstituted or substituted by one or more $C_{1-4}$alkyl, $C_{1-4}$alkoxy or hydroxy groups or halogen atoms, -$NR^7R^8$, -$CONR^7R^8$ or -$(CH_2)_pCONR^7R^8$ (wherein $R^7$ and $R^8$, which may be the same or different, each represents a hydrogen atom or a $C_{1-4}$alkyl or $C_{3-4}$alkenyl group, or together with the nitrogen atom to which they are attached form a saturated 5 to 7 membered ring; and p represents 1, 2 or 3), -$(CH_2)_qNR^9R_{10}$ (wherein $R^9$ represents a hydrogen atom or a $C_{1-4}$alkyl group, and $R^{10}$ represents a group -$COR^{11}$ or -$SO_2R^{11}$ wherein $R^{11}$ represents a $C_{1-4}$alkyl group; and q represents 0, 1, 2 or 3), or -$(CH_2)_2CO_2R^{11}$ ($R^{11}$ being as defined previously);
Q' represents a hydrogen or a fluorine atom; and physiologically acceptable salts and solvates thereof, which comprises:

(A) alkylating a compound of formula (II)

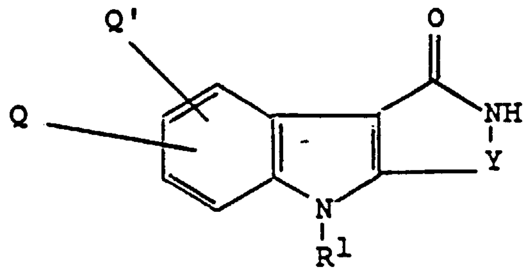

(II)

with a compound of formula (III)

X -Im (III)

or a protected derivative thereof, wherein X represents a group $-CH_2L$ and L represents a leaving atom or group and the reaction is effected in the presence of a base; or X represents the group $-CH_2OH$ and the reaction is effected in the presence of an acid at an elevated temperature followed if necessary by removal of any protecting groups present; or

(B) for the preparation of a compound of formula (I) in which Y represents $(CH_2)_n$, cyclising a compound of formula (IV)

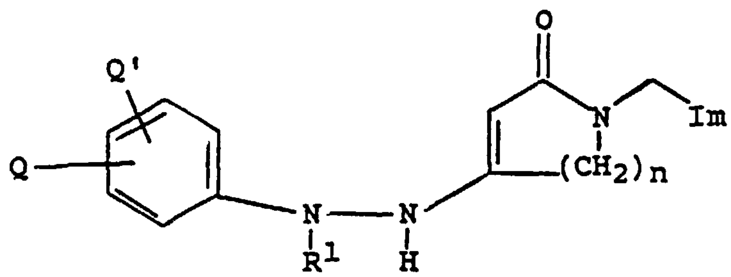

(IV)

or a salt or protected derivative thereof, followed if necessary by removal of any protecting groups present; or

(C) for the preparation of a compound of formula (I) in which $R^3$ represents a hydrogen atom, reacting a compound of formula (V)

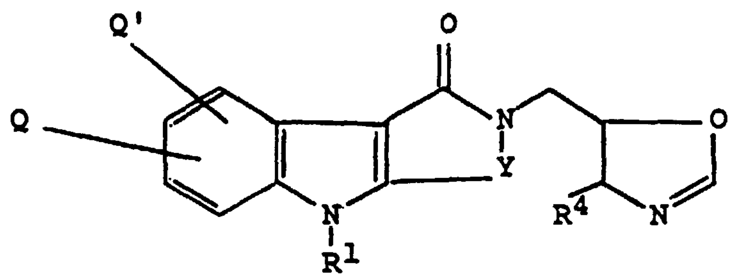

(V)

or protected derivative thereof, with formamide, followed if necessary by removal of any protecting groups present; or

(D) for the preparation of a compound of formula (I) in which Y represents $(CH_2)_n$, reacting a compound of formula (VI)

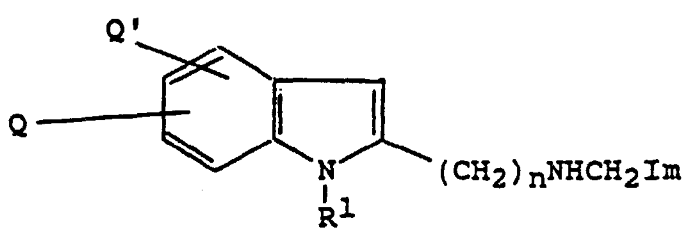

(VI)

or a protected derivative thereof, with phosgene in the presence of a Lewis acid, followed if necessary by removal of any protecting groups present; or

(E) for the preparation of a compound of formula (I) in which Y represents $(CH_2)_n$, oxidising a compound of formula (VII)

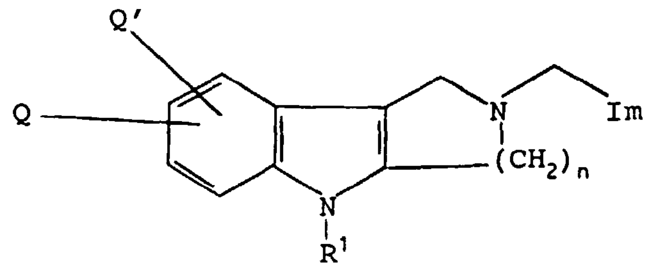

(VII)

or a protected derivative thereof, followed if necessary by removal of any protecting groups present; or
(F) converting a compound of general formula (I) into another compound of formula (I) by hydrogenation, alkylation or acylation; or
(G) removing protecting group(s) from a protected form of a compound of formula (I);

and when the compound of formula (I) is obtained as a mixture of enantiomers, optionally resolving the mixture to obtain the desired enantiomer;
and/or where the compound of formula (I) is in the form of a free base, optionally converting the free base into a salt.

**2.** A process as claimed in claim 1 for the preparation of compounds in which $R^1$ represents a hydrogen atom or a $C_1$-$_6$alkyl, $C_3$-$_4$alkynyl, $C_{4-6}$cycloalkylmethyl, or $C_1$-$_3$alkylsulphonyl group.

**3.** A process as claimed in claim 1 or 2 for the preparation of compounds in which $R^2$, $R^3$ and $R^4$ each independently represent a hydrogen atom or a $C_1$-$_4$alkyl group.

**4.** A process as claimed in any of claims 1 to 3 for the preparation of compounds in which Q represents a halogen atom or a hydroxy, $C_1$-$_4$alkoxy, phenyl$C_1$-$_3$alkoxy-, $C_1$-$_6$alkyl, cyano, phenyl, $-CONH_2$, or $-(CH_2)_2CO_2CH_3$ group.

**5.** A process as claimed in any of claims 1 to 3 for the preparation of compounds in which Q represents a halogen atom, or a hydroxy, phenyl$C_{1-3}$alkoxy-, $C_{1-3}$alkyl or cyano group.

**6.** A process as claimed in any of claims 1 to 3 for the preparation of compounds in which Q represents a fluorine atom.

**7.** A process as claimed in claim 1 for the preparation of compounds in which $R^1$ represents a hydrogen atom or a $C_{1-4}$alkyl, $C_{3-4}$alkynyl or $C_{4-6}$cycloalkylmethyl group; $R^2$ and $R^3$ each represent a hydrogen atom; $R^4$ represents a $C_{1-4}$alkyl group; and Q represents a halogen atom or a hydroxy, phenyl$C_{1-3}$alkoxy-, $C_{1-3}$alkyl or cyano group.

**8.** A process as claimed in any of claims 1 to 7 for the preparation of compounds in which Y represents the group $(CH_2)_2$ and Q′ is a hydrogen atom.

**9.** A process as claimed in claim 1 for the preparatior. of the compound:
6-fluoro-2,3,4,5-tetrahydro-5-methyl-2-[(5-methyl-1H-imidazol-4-yl)methyl]-1H-pyrido[4,3-b]indol-1-one and physiologically acceptable salts and solvates thereof.

**10.** A process as claimed in claim 1 for the preparation of a compound selected from:

2,3,4,5-tetrahydro-5,6-dimethyl-2-[(5-methyl-1H-imidazol-4-yl)-methyl]-1H-pyrido[4,3-b]indol-1-one;
6,9-difluoro-2,3,4,5-tetrahydro-5-methyl-2-[(5-methyl-1H-imidazol-4-yl)methyl]-1H-pyrido[4,3-b]indol-1-one;
6-fluoro-2,3,4,5-tetrahydro-2-[(5-methyl-1H-imidazol-4-yl)methyl]-5-(2-propynyl)-1H-pyrido[4,'3-b]indol-1-one;
2,3,4,5-tetrahydro-5-methyl-2-[(5-methyl-1H-imidazol-4-yl)methyl]-1-oxo-1H-pyrido[4,3-b]indole-6-carbonitrile; and physiologically acceptable salts and solvates thereof.

**Patentansprüche**

**Patentansprüche für folgende Vertragsstaaten : AT, BE, CH, DE, FR, GB, IT, LI, LU, NL, SE**

1. Verbindungen der allgemeinen Formel (I)

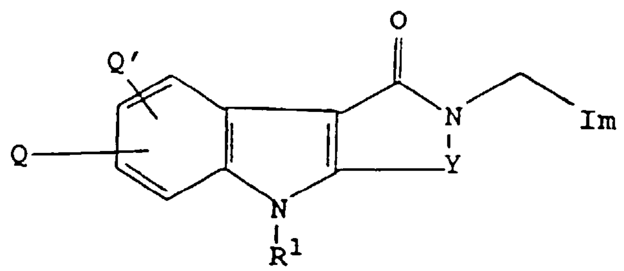

(I)

worin Im eine Imidazolylgruppe der Formel

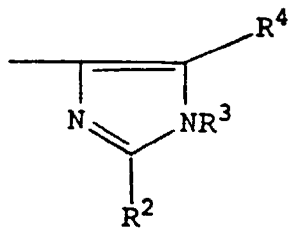

oder

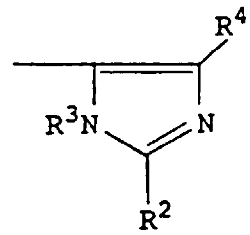

bedeutet,

und $R^1$ ein Wasserstoffatom oder eine Gruppe, ausgewählt aus $C_{1-6}$-Alkyl, $C_{3-6}$-Alkenyl, $C_{3-10}$-Alkinyl, $C_{3-7}$-Cycloalkyl, $C_{3-7}$-Cycloalkyl-$C_{1-4}$-alkyl, Phenyl, Phenyl-$C_{1-3}$-alkyl, $-CO_2R^5$, $-COR^5$, $-CONR^5R^6$ oder $-SO_2R^5$ (worin $R^5$ und $R^6$, die gleich oder unterschiedlich sein können, je ein Wasserstoffatom, eine $C_{1-6}$-Alkyl- oder $C_{3-7}$-Cycloalkylgruppe oder eine Phenyl- oder Phenyl-$C_{1-4}$-alkylgruppe, worin die Phenylgruppe gegebenenfalls durch ein oder mehrere $C_{1-4}$-Alkyl-, $C_{1-4}$-Alkoxy- oder Hydroxygruppen oder Halogenatome substituiert ist, mit der Maßgabe, daß $R^5$ nicht ein Wasserstoffatom bedeutet, wenn $R^1$ eine Gruppe $-CO_2R^5$ oder $-SO_2R^5$ bedeutet), bedeutet,
eine der Gruppen, die durch $R^2$, $R^3$ und $R^4$ dargestellt werden, ein Wasserstoffatom oder eine $C_{1-6}$-Alkyl-, $C_{3-7}$-Cycloalkyl-, $C_{3-6}$-Alkenyl-, Phenyl- oder Phenyl-$C_{1-3}$-alkylgruppe bedeutet, und jede der beiden anderen Gruppen, die gleich oder unterschiedlich sein können, ein Wasserstoffatom oder eine $C_{1-6}$-Alkylgruppe bedeuten,
Y die Gruppe CH=CH oder $(CH_2)_n$ bedeutet, worin n 2 oder 3 bedeutet,
Q ein Halogenatom oder eine Gruppe, ausgewählt aus Hydroxy, $C_{1-4}$-Alkoxy, Phenyl-$C_{1-3}$-alkoxy, $C_{1-6}$-Alkyl, Cyano, Phenyl, welches unsubstituiert oder substituiert durch eine oder mehrere $C_{1-4}$-Alkyl-, $C_{1-4}$-Alkoxy- oder Hydroxygruppen oder Halogenatome sein kann, $-NR^7R^8$, $-CONR^7R^8$ oder $-(CH_2)_pCONR^7R^8$ (worin $R^7$ und $R^8$, die gleich oder unterschiedlich sein können, je ein Wasserstoffatom oder eine $C_{1-4}$-Alkyl- oder $C_{3-4}$-Alkenylgruppe bedeuten, oder zusammen mit dem Stickstoffatom, an das sie gebunden sind, einen gesättigten 5- bis 7gliedrigen Ring bilden, und p 1, 2 oder 3 bedeutet), $-(CH_2)_qNR^9R^{10}$ (worin $R^9$ ein Wasserstoffatom oder eine $C_{1-4}$-Alkylgruppe bedeutet, und $R^{10}$ eine Gruppe $-COR^{11}$ oder $-SO_2R^{11}$ bedeutet, worin $R^{11}$ eine $C_{1-4}$-Alkylgruppe bedeutet, und q 0, 1, 2 oder 3 bedeutet), oder $-(CH_2)_2CO_2R^{11}$ (worin $R^{11}$ die zuvor gegebene Definition besitzt), bedeutet,
Q' ein Wasserstoffatom oder ein Fluoratom bedeutet
und der physiologisch annehmbaren Salze und Solvate davon.

2. Verbindungen nach Anspruch 1, worin $R^1$ ein Wasserstoffatom oder eine $C_{1-6}$-Alkyl-, $C_{3-4}$-Alkinyl-, $C_{4-6}$-Cycloalkylmethyl- oder $C_{1-3}$-Alkylsulfonylgruppe bedeutet.

3. Verbindungen nach Anspruch 1 oder 2, worin $R^2$, $R^3$ und $R^4$ je unabhängig ein Wasserstoffatom oder eine $C_{1-4}$-Alkylgruppe bedeuten.

**4.** Verbindungen nach einem der Ansprüche 1 bis 3, worin Q ein Halogenatom oder eine Hydroxy-, $C_{1-4}$-Alkoxy-, Phenyl-$C_{1-3}$-alkoxy-, $C_{1-6}$-Alkyl-, Cyano-, Phenyl-, -$CONH_2$- oder -$(CH_2)_2CO_2CH_3$-Gruppe bedeutet.

**5.** Verbindungen nach einem der Ansprüche 1 bis 3, worin Q ein Halogenatom oder eine Hydroxy-, Phenyl-$C_{1-3}$-alkoxy-, $C_{1-3}$-Alkyl- oder Cyanogruppe bedeutet.

**6.** Verbindungen nach einem der Ansprüche 1 bis 3, worin Q ein Fluoratom bedeutet.

**7.** Verbindungen nach Anspruch 1, worin $R^1$ ein Wasserstoffatom oder eine $C_{1-4}$-Alkyl-, $C_{3-4}$-Alkinyl- oder $C_{4-6}$-Cycloalkylmethylgruppe bedeutet, $R^2$ und $R^3$ je ein Wasserstoffatom bedeuten, $R^4$ eine $C_{1-4}$-Alkylgruppe bedeutet, und Q ein Halogenatom oder eine Hydroxy-, Phenyl-$C_{1-3}$-alkoxy-, $C_{1-3}$-Alkyl- oder Cyanogruppe bedeutet.

**8.** Verbindungen nach einem der Ansprüche 1 bis 7, worin Y die Gruppe $(CH_2)_2$ bedeutet und Q' ein Wasserstoffatom bedeutet.

**9.** 6-Fluor-2,3,4,5-tetrahydro-5-methyl-2-[(5-methyl-1H-imidazol-4-yl)methyl]-1H-pyrido[4.3-b]indol-1-on und die physiologisch annehmbaren Salze und Solvate davon.

**10.** 2,3,4,5-Tetrahydro-5,6-dimethyl-2-[(5-methyl-1H-imidazol-4-yl)methyl]-1H-pyrido[4.3-b]indol-1-on; 6,9-Difluor-2,3,4,5-tetrahydro-5-methyl-2-[(5-methyl-1H-imidazol-4-yl)methyl]-1H-pyrido[4.3-b]indol-1-on; 6-Fluor-2,3,4,5-tetrahydro-2-[(5-methyl-1H-imidazol-4-yl)methyl]-5-(2-propinyl)-1H-pyrido[4.3-b]indol-1-on; 2,3,4,5-Tetrahydro-5-methyl-2-[(5-methyl-1H-imidazol-4-yl)methyl]-1-oxo-1H-pyrido[4.3-b]indol-6-carbonitril; und die physiologisch annehmbaren Salze und Solvate davon.

**11.** Verfahren zur Herstellung von Verbindungen der allgemeinen Formel (I) nach einem der Ansprüche 1 bis 10 oder ein physiologisch annehmbares Salz oder Solvat davon, umfassend:

(A) die Alkylierung einer Verbindung der Formel (II)

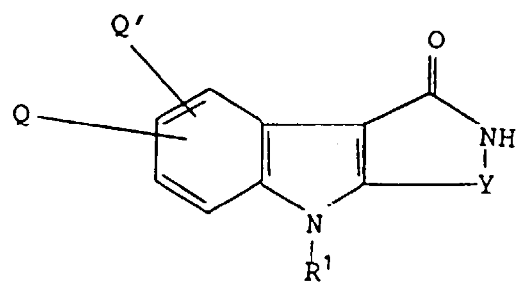

(II)

mit einer Verbindung der Formel (III)

X-Im (III)

oder einem geschützten Derivat davon, worin X eine Gruppe -$CH_2L$ bedeutet, und L ein Austrittsatom oder -gruppe bedeutet und die Reaktion in Anwesenheit einer Base durchgeführt wird, oder worin X die Gruppe -$CH_2OH$ bedeutet und die Reaktion in Anwesenheit einer Säure bei erhöhter Temperatur durchgeführt wird, gefolgt, sofern erforderlich, von der Entfernung von irgendwelchen vorhandenen Schutzgruppen, oder

(B) zur Herstellung einer Verbindung der Formel (I), worin Y $(CH_2)_n$ bedeutet, die Cyclisierung einer Verbindung der Formel (IV)

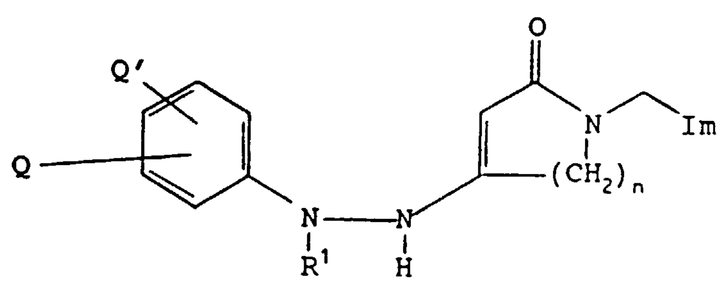

(IV)

oder eines Salzes oder geschützten Derivates davon, gefolgt, sofern erforderlich, von der Entfernung von irgendwelchen vorhandenen Schutzgruppen, oder

(C) für die Herstellung einer Verbindung der Formel (I), worin $R^3$ ein Wasserstoffatom bedeutet, Umsetzung einer Verbindung der Formel (V)

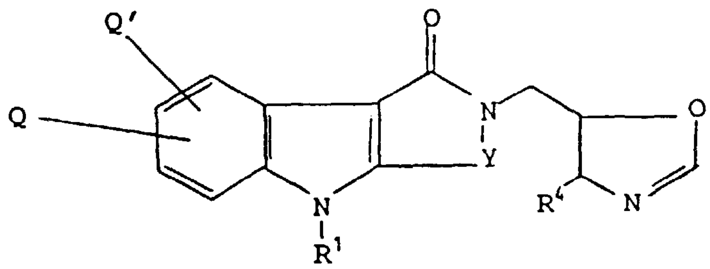

(V)

oder eines geschützten Derivates davon, mit Formamid, gefolgt, sofern erforderlich, von der Entfernung von irgendwelchen vorhandenen Schutzgruppen; oder

(D) für die Herstellung einer Verbindung der Formel (I), worin Y $(CH_2)_n$ bedeutet, Umsetzung einer Verbindung der Formel (VI)

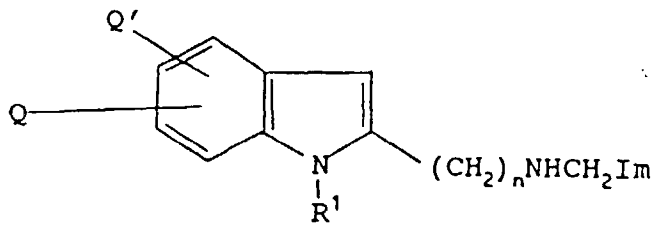

(VI)

oder eines geschützten Derivates davon, mit Phosgen, in Anwesenheit einer Lewis-Säure, gefolgt, sofern erforderlich, von der Entfernung von irgendwelchen vorhandenen Schutzgruppen, oder

(E) für die Herstellung einer Verbindung der Formel (I), worin Y $(CH_2)_n$ bedeutet, Oxidation einer Verbindung der Formel (VII)

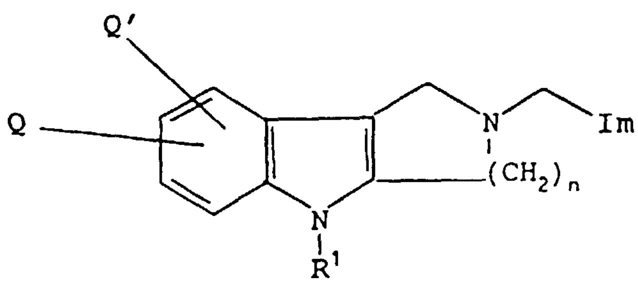

(VII)

oder eines geschützten Derivates davon, gefolgt, sofern erforderlich, von der Entfernung von irgendwelchen vorhandenen Schutzgruppen, oder

(F) Umwandlung einer Verbindung der allgemeinen Formel (I) in eine andere Verbindung der Formel (I) durch Hydrierung, Alkylierung oder Acylierung, oder

(G) Entfernung von einer oder mehreren Schutzgruppe(n) von einer geschützten Form einer Verbindung der Formel (I),

und wenn die Verbindung der Formel (I) als Gemisch der Enantiomeren erhalten wird, gegebenenfalls Spaltung des Gemisches unter Herstellung des gewünschten Enantiomeren,

und/oder wenn die Verbindung der Formel (I) in Form einer freien Base vorliegt, gegebenenfalls Umwandlung der freien Base in ein Salz.

**12.** Pharmazeutische Zubereitung, enthaltend mindestens eine Verbindung der allgemeinen Formel (I), wie in Anspruch 1 definiert, oder ein physiologisch annehmbares Salz oder Solvat davon, zusammen mit mindestens einem physiologisch annehmbaren Träger oder Exzipiens.

**13.** Pharmazeutische Zubereitung nach Anspruch 12 in einer für die orale oder parenterale Verabreichung angepaßten Form.

**14.** Pharmazeutische Zubereitung nach Anspruch 12 oder 13, worin der aktive Bestandteil 6-Fluor-2,3,4,5-tetrahydro-5-methyl-2-[(5-methyl-1H-imidazol-4-yl)methyl]-1H-pyrido[4.3-b]indol-1-on oder ein physiologisch annehmbares Salz oder Solvat davon ist.

**15.** Verbindung der Formel (I) nach einem der Ansprüche 1 bis 10 oder ein physiologisch annehmbares Salz oder Solvat davon für die Verwendung als aktives therapeutisches Mittel.

**16.** Verwendung einer Verbindung der Formel (I) nach einem der Ansprüche 1 bis 10 oder eines physiologisch annehmbaren Salzes oder Solvats davon für die Herstellung eines Arzneimittels für die Behandlung eines Zustands, der durch 5-$HT_3$-Rezeptoren vermittelt wird.

**17.** Verwendung einer Verbindung der Formel (I) nach einem der Ansprüche 1 bis 10 oder eines physiologisch annehmbaren Salzes oder Solvats davon für die Herstellung eines Arzneimittels für die Behandlung von Nausea und Erbrechen.

**Patentansprüche für folgende Vertragsstaaten : GR, ES**

**1.** Verfahren zur Herstellung einer Verbindung der allgemeinen Formel (I)

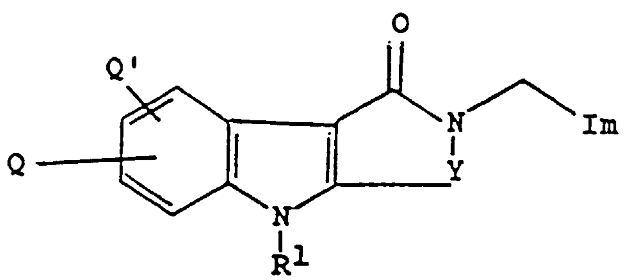

(I)

worin Im eine Imidazolylgruppe der Formel

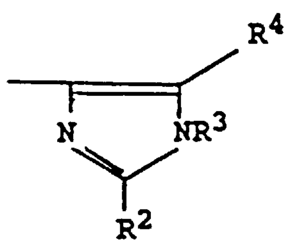

oder

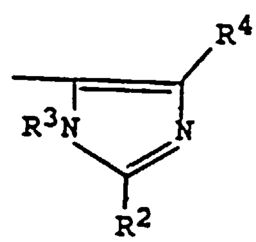

bedeutet,

und $R^1$ ein Wasserstoffatom oder eine Gruppe, ausgewählt aus $C_{1-6}$-Alkyl, $C_{3-6}$-Alkenyl, $C_{3-10}$-Alkinyl, $C_{3-7}$-Cycloalkyl, $C_{3-7}$-Cycloalkyl-$C_{1-4}$-alkyl, Phenyl, Phenyl-$C_{1-3}$-alkyl, -$CO_2R^5$, -$COR^5$, -$CONR^5R^6$ oder -$SO_2R^5$ (worin $R^5$ und $R^6$, die gleich oder unterschiedlich sein können, je ein Wasserstoffatom, eine $C_{1-6}$-Alkyl- oder $C_{3-7}$-Cycloalkylgruppe oder eine Phenyl- oder Phenyl-$C_{1-4}$-alkylgruppe, worin die Phenylgruppe gegebenenfalls durch ein oder mehrere $C_{1-4}$-Alkyl-, $C_{1-4}$-Alkoxy- oder Hydroxygruppen oder Halogenatome substituiert ist, mit der Maßgabe, daß $R^5$ nicht ein Wasserstoffatom bedeutet, wenn $R^1$ eine Gruppe -$CO_2R^5$ oder -$SO_2R^5$ bedeutet), bedeutet,
eine der Gruppen, die durch $R^2$, $R^3$ und $R^4$ dargestellt werden, ein Wasserstoffatom oder eine $C_{1-6}$-Alkyl-, $C_{3-7}$-Cycloalkyl-, $C_{3-6}$-Alkenyl-, Phenyl- oder Phenyl-$C_{1-3}$-alkylgruppe bedeutet, und jede der beiden anderen Gruppen, die gleich oder unterschiedlich sein können, ein Wasserstoffatom oder eine $C_{1-6}$-Alkylgruppe bedeuten,
Y die Gruppe CH=CH oder $(CH_2)_n$ bedeutet, worin n 2 oder 3 bedeutet,
Q ein Halogenatom oder eine Gruppe, ausgewählt aus Hydroxy, $C_{1-4}$-Alkoxy, Phenyl-$C_{1-3}$-alkoxy, $C_{1-6}$-Alkyl, Cyano, Phenyl, welches unsubstituiert oder substituiert durch eine oder mehrere $C_{1-4}$-Alkyl-, $C_{1-4}$-Alkoxy- oder Hydroxygruppen oder Halogenatome sein kann, -$NR^7R^8$, -$CONR^7R^8$ oder-$(CH_2)_pCONR^7R^8$ (worin $R^7$ und $R^8$, die gleich oder unterschiedlich sein können, je ein Wasserstoffatom oder eine $C_{1-4}$-Alkyl- oder $C_{3-4}$-

Alkenylgruppe bedeuten, oder zusammen mit dem Stickstoffatom, an das sie gebunden sind, einen gesättigten 5- bis 7gliedrigen Ring bilden, und p 1, 2 oder 3 bedeutet), $-(CH_2)_qNR^9R^{10}$ (worin $R^9$ ein Wasserstoffatom oder eine $C_{1-4}$-Alkylgruppe bedeutet und $R^{10}$ eine Gruppe $-COR^{11}$ oder $-SO_2R^{11}$ bedeutet, worin $R^{11}$ eine $C_{1-4}$-Alkylgruppe bedeutet, und q 0, 1, 2 oder 3 bedeutet), oder $-(CH_2)_2CO_2R^{11}$ (worin $R^{11}$ die zuvor gegebene Definition besitzt), bedeutet,
Q' ein Wasserstoffatom oder ein Fluoratom bedeutet
und der physiologisch annehmbaren Salze und Solvate davon, umfassend

(A) die Alkylierung einer Verbindung der Formel (II)

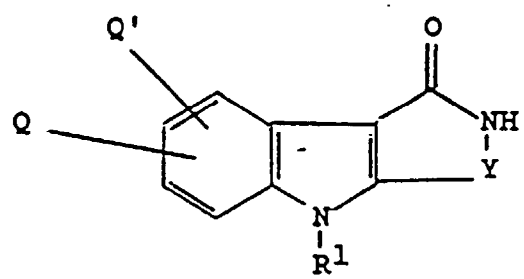

(II)

mit einer Verbindung der Formel (III)

X-Im (III)

oder einem geschützten Derivat davon, worin X eine Gruppe $-CH_2L$ bedeutet, und L ein Austrittsatom oder -gruppe bedeutet und die Reaktion in Anwesenheit einer Base durchgeführt wird, oder worin X die Gruppe $-CH_2OH$ bedeutet und die Reaktion in Anwesenheit einer Säure bei erhöhter Temperatur durchgeführt wird, gefolgt, sofern erforderlich, von der Entfernung von irgendwelchen vorhandenen Schutzgruppen, oder
(B) zur Herstellung einer Verbindung der Formel (I), worin Y $(CH_2)_n$ bedeutet, die Cyclisierung einer Verbindung der Formel (IV)

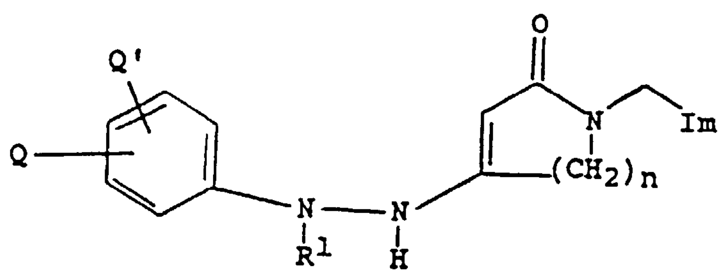

(IV)

oder eines Salzes oder geschützten Derivates davon, gefolgt, sofern erforderlich, von der Entfernung von irgendwelchen vorhandenen Schutzgruppen, oder
(C) für die Herstellung einer Verbindung der Formel (I), worin $R^3$ ein Wasserstoffatom bedeutet, Umsetzung einer Verbindung der Formel (V)

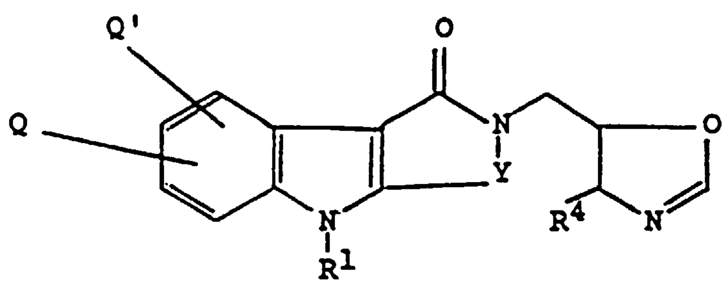

(V)

oder eines geschützten Derivates davon, mit Formamid, gefolgt, sofern erforderlich, von der Entfernung von irgendwelchen vorhandenen Schutzgruppen; oder
(D) für die Herstellung einer Verbindung der Formel (I), worin Y $(CH_2)_n$ bedeutet, Umsetzung einer Verbindung der Formel (VI)

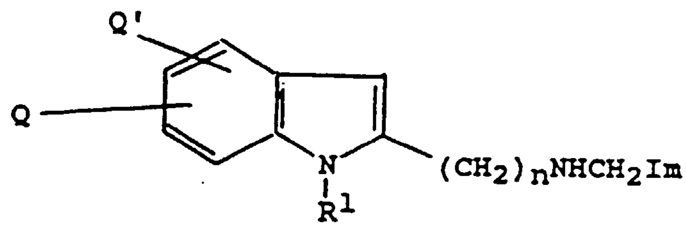

(VI)

oder eines geschützten Derivates davon, mit Phosgen, in Anwesenheit einer Lewis-Säure, gefolgt, sofern erforderlich, von der Entfernung von irgendwelchen vorhandenen Schutzgruppen, oder
(E) für die Herstellung einer Verbindung der Formel (I), worin Y $(CH_2)_n$ bedeutet, Oxidation einer Verbindung der Formel (VII)

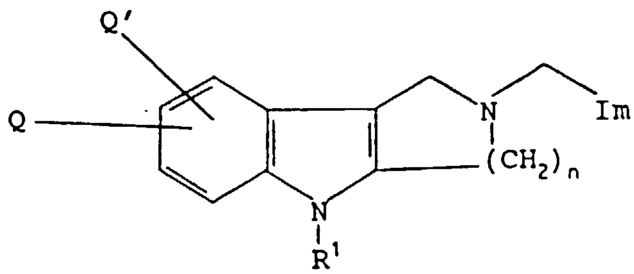

(VII)

oder eines geschützten Derivates davon, gefolgt, sofern erforderlich, von der Entfernung von irgendwelchen vorhandenen Schutzgruppen, oder
(F) Umwandlung einer Verbindung der allgemeinen Formel (I) in eine andere Verbindung der Formel (I) durch Hydrierung, Alkylierung oder Acylierung, oder
(G) Entfernung von einer oder mehreren Schutzgruppe(n) von einer geschützten Form einer Verbindung der Formel (I),

und wenn die Verbindung der Formel (I) als Gemisch der Enantiomeren erhalten wird, gegebenenfalls Spaltung des Gemisches unter Herstellung des gewünschten Enantiomeren,
und/oder wenn die Verbindung der Formel (I) in Form einer freien Base vorliegt, gegebenenfalls Umwandlung der freien Base in ein Salz.

**2.** Verfahren nach Anspruch 1 für die Herstellung von Verbindungen, worin $R^1$ ein Wasserstoffatom oder eine $C_{1-6}$-Alkyl-, $C_{3-4}$-Alkinyl-, $C_{4-6}$-Cycloalkylmethyl- oder $C_{1-3}$-Alkylsulfonylgruppe bedeutet.

**3.** Verfahren nach Anspruch 1 oder 2 für die Herstellung von Verbindungen, worin $R^2$, $R^3$ und $R^4$ je unabhängig ein Wasserstoffatom oder eine $C_{1-4}$-Alkylgruppe bedeuten.

**4.** Verfahren nach einem der Ansprüche 1 bis 3 für die Herstellung von Verbindungen, worin Q ein Halogenatom oder eine Hydroxy-, $C_{1-4}$-Alkoxy-, Phenyl-$C_{1-3}$-alkoxy-, $C_{1-6}$-Alkyl-, Cyano-, Phenyl-, $-CONH_2$- oder $-(CH_2)_2CO_2CH_3$-Gruppe bedeutet.

**5.** Verfahren nach einem der Ansprüche 1 bis 3 für die Herstellung von Verbindungen, worin Q ein Halogenatom oder eine Hydroxy-, Phenyl-$C_{1-3}$-alkoxy-, $C_{1-3}$-Alkyl- oder Cyanogruppe bedeutet.

**6.** Verfahren nach einem der Ansprüche 1 bis 3 für die Herstellung von Verbindungen, worin Q ein Fluoratom bedeutet.

**7.** Verfahren nach Anspruch 1 für die Herstellung von Verbindungen, worin $R^1$ ein Wasserstoffatom oder eine $C_{1-4}$-Alkyl-, $C_{3-4}$-Alkinyl- oder $C_{4-6}$-Cycloalkylmethylgruppe bedeutet, $R^2$ und $R^3$ je ein Wasserstoffatom bedeuten, $R^4$ eine $C_{1-4}$-Alkylgruppe bedeutet, und Q ein Halogenatom oder eine Hydroxy-, Phenyl-$C_{1-3}$-alkoxy-, $C_{1-3}$-Alkyl- oder Cyanogruppe bedeutet.

**8.** Verfahren nach einem der Ansprüche 1 bis 7 für die Herstellung von Verbindungen, worin Y die Gruppe $(CH_2)_2$ bedeutet und Q' ein Wasserstoffatom bedeutet.

**9.** Verfahren nach Anspruch 1 für die Herstellung der Verbindung 6-Fluor-2,3,4,5-tetrahydro-5-methyl-2-[(5-methyl-1H-imidazol-4-yl)methyl]-1H-pyrido[4.3-b]indol-1-on und der physiologisch annehmbaren Salze und Solvate davon.

10. Verfahren nach Anspruch 1 für die Herstellung einer Verbindung, ausgewählt aus:

2,3,4,5-Tetrahydro-5,6-dimethyl-2-[(5-methyl-1H-imidazol-4-yl)methyl]-1H-pyrido[4.3-b]indol-1-on;
6,9-Difluor-2,3,4,5-tetrahydro-5-methyldazol-4-yl)methyl]-1H-pyrido[4.3-b]indol-1-on;
6-Fluor-2,3,4,5-tetrahydro-2-[(5-methyl-1H-imidazol-4-yl)methyl]-5-(2-propinyl)-1H-pyrido[4.3-b]indol-1-on;
2,3,4,5-Tetrahydro-5-methyl-2-[(5-methyl-1H-imidazol-4-yl)methyl]-1-oxo-1H-pyrido[4.3-b]indol-6-carbonitril;
und der physiologisch annehmbaren Salzen und Solvaten davon.

## Revendications

### Revendications pour les Etats contractants suivants : AT, BE, CH, DE, FR, GB, IT, LI, LU, NL, SE

1. Composés de la formule générale (I)

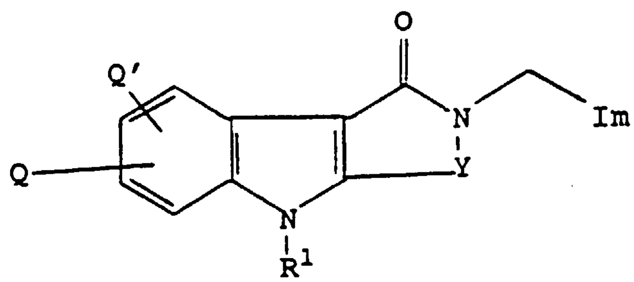

(I)

dans laquelle Im représente un radical imidazolyle de la formule

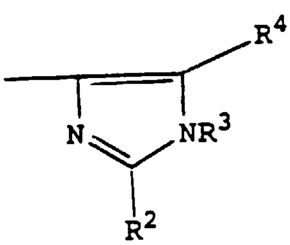

ou

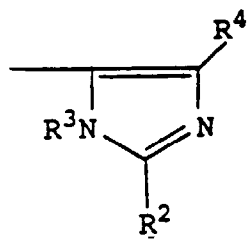

et $R^1$ représente un atome d'hydrogène ou un radical choisi parmi les groupes alkyle en $C_1$ à $C_6$, alcényle en $C_3$ à $C_6$, alcynyle en $C_3$ à $C_{10}$, cycloalkyle en $C_3$ à $C_7$, cycloalkyl($C_3$-$C_7$)alkyle($C_1$-$C_4$), phényle, phénylalkyle ($C_1$-$C_3$), $-CO_2R^5$, $-COR^5$, $-CONR^5R^6$ ou $-SO_2R^5$ (où $R^5$ et $R^6$, qui peuvent avoir des significations identiques ou différentes, représentent chacun un atome d'hydrogène, un radical alkyle en $C_1$ à $C_6$ ou cycloalkyle en $C_3$ à $C_7$, ou un radical phényle ou phénylalkyle($C_1$-$C_4$), où le groupe phényle est éventuellement substitué par un ou plusieurs radicaux alkyle en $C_1$ à $C_4$, alcoxy en $C_1$ à $C_4$, ou hydroxyle, ou un ou plusieurs atomes d'halogènes, avec la condition que $R^5$ ne représente pas d'atome d'hydrogène lorsque $R^1$ représente un groupe $-CO_2R^5$ ou $-SO_2R^5$);
l'un des symboles représentés par $R^2$, $R^3$ et $R^4$ désigne un atome d'hydrogène ou un radical alkyle en $C_1$ à $C_6$, cycloalkyle en $C_3$ à $C_7$, alcényle en $C_3$ à $C_6$, phényle ou phénylalkyle($C_1$-$C_3$) et chacun des deux autres symboles, qui peuvent avoir des significations identiques ou différentes, représente un atome d'hydrogène ou un radical alkyle en $C_1$ à $C_6$;
Y représente un radical CH=CH ou $(CH_2)_n$, où n est égal à 2 ou à 3;
Q représente un atome d'halogène, ou un radical choisi parmi les groupes hydroxyle, alcoxy en $C_1$ à $C_4$, phénylalcoxy($C_1$-$C_3$), alkyle en $C_1$ à $C_6$, cyano, phényle qui peut ne pas être substitué ou être substitué par un ou plusieurs radicaux alkyle en $C_1$ à $C_4$, alcoxy en $C_1$ à $C_4$, ou hydroxyle, ou un ou plusieurs atomes d'halogènes, $-NR^7R^8$, $-CONR^7R^8$ ou $-(CH_2)_pCONR^7R^8$ (où $R^7$ et $R^8$, qui peuvent avoir des significations identiques ou différentes, représentent chacun un atome d'hydrogène ou un radical alkyle en $C_1$ à $C_4$ ou alcényle

en $C_3$ à $C_4$, ou bien ils représentent ensemble avec l'atome d'azote auquel ils sont attachés, un noyau saturé pentagonal ou heptagonal et p est égal à 1, 2 ou 3), $-(CH_2)_qNR^9R^{10}$ (où $R^9$ représente un atome d'hydrogène ou un radical alkyle en $C_1$ à $C_4$ et $R^{10}$ représente un groupe $-COR^{11}$ ou $-SO_2R^{11}$, où $R^{11}$ représente un radical alkyle en $C_1$ à $C_4$; et q est égal à 0, 1, 2 ou 3) ou $-(CH_2)_2CO_2R^{11}$ (où $R^{11}$ possède les significations qui lui ont été précédemment attribuées);
Q' représente un atome d'hydrogène ou un atome de fluor;

ainsi que les solvates et les sels physiologiquement acceptables de ces composés.

**2.** Composés suivant la revendication 1, caractérisés en ce que $R^1$ représente un atome d'hydrogène, ou un radical alkyle en $C_1$ à $C_6$, alcynyle en $C_3$ à $C_4$, cycloalkyl($C_4$-$C_6$)méthyle ou alkylsulfonyle en $C_1$ à $C_3$.

**3.** Composés suivant la revendication 1 ou 2, caractérisés en ce que $R^2$, $R^3$ et $R^4$ représentent chacun indépendamment un atome d'hydrogène ou un radical alkyle en $C_1$ à $C_4$.

**4.** Composés suivant l'une quelconque des revendications 1 à 3, caractérisés en ce que Q représente un atome d'halogène ou un radical hydroxyle, alcoxy en $C_1$ à $C_4$, phénylalcoxy($C_1$-$C_3$), alkyle en $C_1$ à $C_6$, cyano, phényle, $-CONH_2$ ou $-(CH_2)_2CO_2CH_3$.

**5.** Composés suivant l'une quelconque des revendications 1 à 3, caractérisés en ce que Q représente un atome d'halogène, ou un radical hydroxyle, phénylalcoxy-($C_1$-$C_3$), alkyle en $C_1$ à $C_3$, ou cyano.

**6.** Composés suivant l'une quelconque des revendications 1 à 3, caractérisés en ce que Q représente un atome de fluor.

**7.** Composés suivant la revendication 1, caractérisés en ce que $R^1$ représente un atome d'hydrogène, ou un radical alkyle en $C_1$ à $C_4$, alcynyle en $C_3$ à $C_4$, ou cycloalkyl($C_4$-$C_6$)méthyle; $R^2$ et $R^3$ représentent chacun un atome d'hydrogène; $R^4$ représente un radical alkyle en $C_1$ à $C_4$; et Q représente un atome d'halogène ou un radical hydroxyle, phénylalcoxy($C_1$-$C_3$), alkyle en $C_1$ à $C_3$, ou cyano.

**8.** Composés suivant l'une quelconque des revendications 1 à 7, caractérisés en ce que Y représente le radical $(CH_2)_2$ et Q' représente un atome d'hydrogène.

**9.** 6-Fluoro-2,3,4,5-tétrahydro-5-méthyl-2-[(5-méthyl-1H-imidazole-4-yl)méthyl]-1H-pyrido[4,3-b]indole-1-one et ses solvates et sels physiologiquement acceptables.

**10.** 2,3,4,5-Tétrahydro-5,6-diméthyl-2-[(5-méthyl-1H-imidazole-4-yl)méthyl]-1H-pyrido[4,3-b]indole-1-one;
6,9-difluoro-2,3,4,5-tétrahydro-5-méthyl-2-[(5-méthyl-1H-imidazole-4-yl)méthyl]-1H-pyrido[4,3-b]indole-1-one;
6-fluoro-2,3,4,5-tétrahydro-2-[(5-méthyl-1H-imidazole-4-yl)méthyl]-5-(2-propynyl)-1H-pyrido[4,3-b]indole-1-one;
2,3,4,5-tétrahydro-5-méthyl-2-[(5-méthyl-1H-imidazole-4-yl)méthyl]-1-oxo-1H-pyrido[4,3-b]indole-6-carbonitrile;
et leurs solvates et sels physiologiquement acceptables.

**11.** Procédé de préparation de composés de la formule générale (I) tels que définis dans l'une quelconque des revendications 1 à 10, ou d'un solvant ou d'un sel physiologiquement acceptable de ces composés, caractérisé en ce que :

(A) on alkyle un composé de la formule (II)

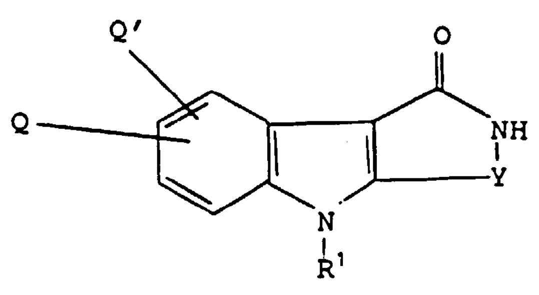

(II)

avec un composé de la formule (III)

X-Im (III)

ou un dérivé protégé de celui-ci, où X représente un radical $-CH_2L$ et L représente un groupe ou atome labile ou sortant et on réalise la réaction en présence d'une base; ou bien X représente le radical $-CH_2OH$ et on réalise la réaction en présence d'un acide, à température élevée, réaction suivie, si cela se révèle nécessaire, de l'élimination de n'importe quels radicaux protecteurs; ou bien

(B) pour la préparation d'un composé de la formule (I) dans laquelle Y représente $(CH_2)_n$, on cyclise un composé de la formule (IV)

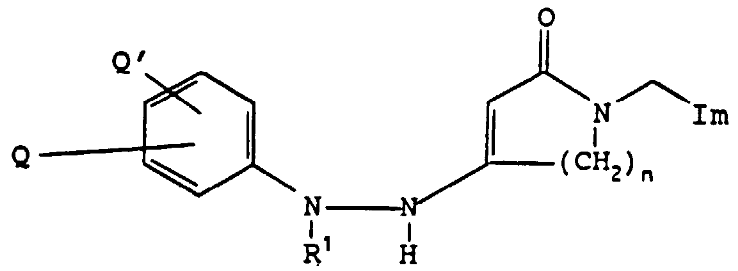

(IV)

ou un sel ou dérivé protégé de celui-ci, cyclisation suivie, si cela se révèle nécessaire, de l'élimination de n'importe quels radicaux protecteurs présents; ou bien

(C) pour la préparation d'un composé de la formule (I) dans laquelle $R^3$ représente un atome d'hydrogène, on fait réagir un composé de la formule (V)

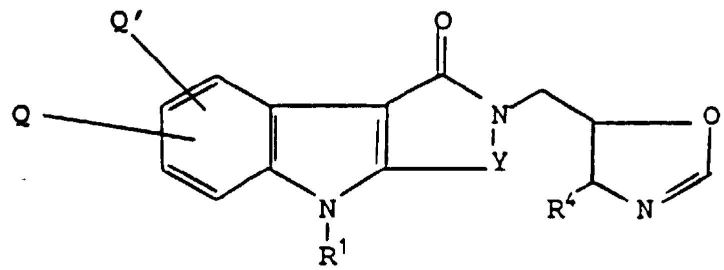

(V)

ou un dérivé protégé de celui-ci, avec le formamide, réaction suivie, si cela se révèle nécessaire, de l'élimination de n'importe quels radicaux protecteurs présents; ou bien

(D) pour la préparation d'un composé de la formule (I) dans laquelle Y représente $(CH_2)_n$, on fait réagir un composé de la formule (VI)

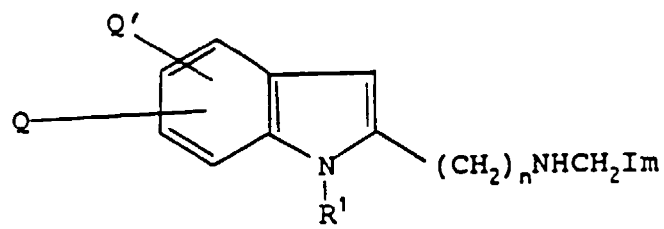

(VI)

ou un dérivé protégé de celui-ci, avec le phosgène, en présence d'un acide de Lewis, réaction suivie, si cela se révèle nécessaire, de l'élimination de n'importe quels radicaux protecteurs présents; ou bien

(E) pour la préparation d'un composé de la formule (I) dans laquelle Y représente $(CH_2)_n$, on oxyde un composé de la formule (VII)

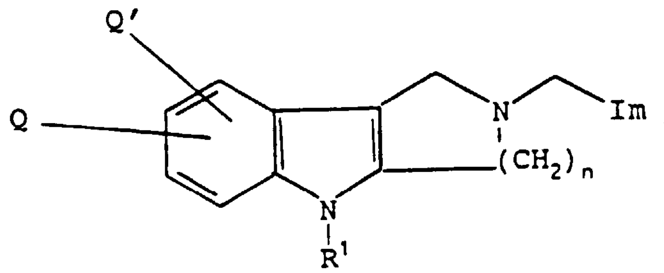

(VII)

ou un dérivé protégé de celui-ci, oxydation suivie, si cela se révèle nécessaire, de l'élimination de n'importe quels radicaux protecteurs présents; ou bien

(F) on convertit un composé de la formule générale (I) en un autre composé de la formule (I) par hydrogénation, alkylation ou acylation; ou bien

(G) on élimine le ou les groupes protecteurs d'une forme protégée d'un composé de la formule (I);

et lorsque le composé de la formule (I) est obtenu sous la forme d'un mélange d'énantiomères, on résout éventuellement le mélange de manière à obtenir l'énantiomère souhaité;
et/ou lorsque le composé de la formule (I) se présente sous la forme d'une base libre, on convertit éventuellement la base libre en un sel.

**12.** Composition pharmaceutique comprenant au moins un composé de la formule générale (I) tel que défini dans la revendications 1, ou un solvate ou sel physiologiquement acceptable d'un tel composé, en même temps qu'au moins un véhicule ou excipient physiologiquement acceptable.

**13.** Composition pharmaceutique suivant la revendication 12, sous une forme convenant à l'administration par la voie orale ou parentérale.

**14.** Composition pharmaceutique suivant la revendication 12 ou 13, caractérisée en ce que l'ingrédient actif est la 6-fluoro-2,3,4,5-tétrahydro-5-méthyl-2-[(5-méthyl-1H-imidazole-4-yl)méthyl]-1H-pyrido[4,3-b]indole-1-one ou un solvate ou sel physiologiquement acceptable de celle-ci.

**15.** Composé de la formule (I) tel que défini dans l'une quelconque des revendications 1 à 10, ou un solvate ou sel physiologiquement acceptable d'un tel composé, à utiliser comme agent thérapeutique actif.

**16.** Utilisation d'un composé de la formule (I) tel que défini dans l'une quelconque des revendications 1 à 10, ou d'un solvate ou d'un sel physiologiquement acceptable de ce composé en vue de la fabrication d'un médicament destiné au traitement d'un état commandé par les récepteurs 5-$HT_3$.

**17.** Utilisation d'un composé de la formule (I) tel que défini dans l'une quelconque des revendications 1 à 10 ou d'un solvate ou d'un sel physiologiquement acceptable d'un tel composé en vue de la fabrication d'un médicament pour le traitement des nausées et des vomissements.

**Revendications pour les Etats contractants suivants : ES, GR**

**1.** Procédé de préparation de composés de la formule générale (I)

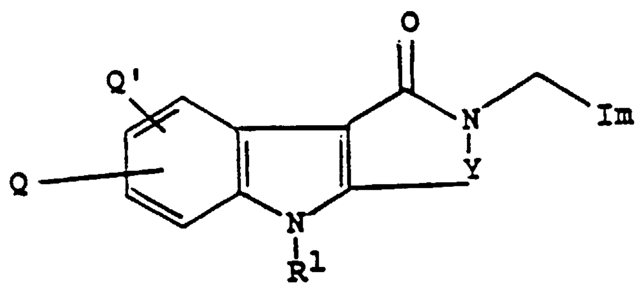

(I)

dans laquelle Im représente un radical imidazolyle de la formule

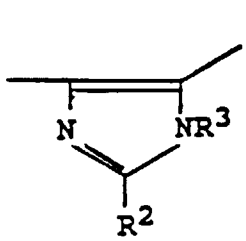

or

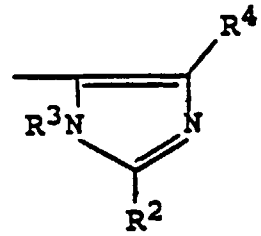

et $R^1$ représente un atome d'hydrogène ou un radical choisi parmi les groupes alkyle en $C_1$ à $C_6$, alcényle en $C_3$ à $C_6$, alcynyle en $C_3$ à $C_{10}$, cycloalkyle en $C_3$ à $C_7$, cycloalkyl($C_3$-$C_7$)alkyle($C_1$-$C_4$), phényle, phénylalkyle ($C_1$-$C_3$), $-CO_2R^5$, $-COR^5$, $-CONR^5R^6$ ou $-SO_2R^5$ (où $R^5$ et $R^6$, qui peuvent avoir des significations identiques ou différentes, représentent chacun un atome d'hydrogène, un radical alkyle en $C_1$ à $C_6$ ou cycloalkyle en $C_3$ à $C_7$, ou un radical phényle ou phénylalkyle($C_1$-$C_4$), où le groupe phényle est éventuellement substitué par un ou plusieurs radicaux alkyle en $C_1$ à $C_4$, alcoxy en $C_1$ à $C_4$, ou hydroxyle, ou un ou plusieurs atomes d'halogènes, avec la condition que $R^5$ ne représente pas d'atome d'hydrogène lorsque $R^1$ représente un groupe $-CO_2R^5$ ou $-SO_2R^5$);
l'un des symboles représentés par $R^2$, $R^3$ et $R^4$ désigne un atome d'hydrogène ou un radical alkyle en $C_1$ à $C_6$, cycloalkyle en $C_3$ à $C_7$, alcényle en $C_3$ à $C_6$, phényle ou phénylalkyle($C_1$-$C_3$) et chacun des deux autres symboles, qui peuvent avoir des significations identiques ou différentes, représente un atome d'hydrogène ou un radical alkyle en $C_1$ à $C_6$;
Y représente un radical CH=CH ou $(CH_2)_n$, où n est égal à 2 ou à 3;
Q représente un atome d'halogène, ou un radical choisi parmi les groupes hydroxyle, alcoxy en $C_1$ à $C_4$, phénylalcoxy($C_1$-$C_3$), alkyle en $C_1$ à $C_6$, cyano, phényle qui peut ne pas être substitué ou être substitué par un ou plusieurs radicaux alkyle en $C_1$ à $C_4$, alcoxy en $C_1$ à $C_4$, ou hydroxyle, ou un ou plusieurs atomes d'halogènes, $-NR^7R^8$, $-CONR^7R^8$ ou $-(CH_2)_pCONR^7R^8$ (où $R^7$ et $R^8$, qui peuvent avoir des significations identiques ou différentes, représentent chacun un atome d'hydrogène ou un radical alkyle en $C_1$ à $C_4$ ou alcényle en $C_3$ à $C_4$, ou bien ils représentent ensemble avec l'atome d'azote auquel ils sont attachés, un noyau saturé pentagonal ou heptagonal et p est égal à 1, 2 ou 3), $-(CH_2)_qNR^9R^{10}$ (où $R^9$ représente un atome d'hydrogène ou un radical alkyle en $C_1$ à $C_4$ et $R^{10}$ représente un groupe $-COR^{11}$ ou $-SO_2R^{11}$, où $R^{11}$ représente un radical alkyle en $C_1$ à $C_4$; et q est égal à 0, 1, 2 ou 3) ou $-(CH_2)_2CO_2R^{11}$ (où $R^{11}$ possède les significations qui lui ont été précédemment attribuées);
Q' représente un atome d'hydrogène ou un atome de fluor;

ainsi que les solvates et les sels physiologiquement acceptables de ces composés, caractérisé en ce que:

(A) on alkyle un composé de la formule (II)

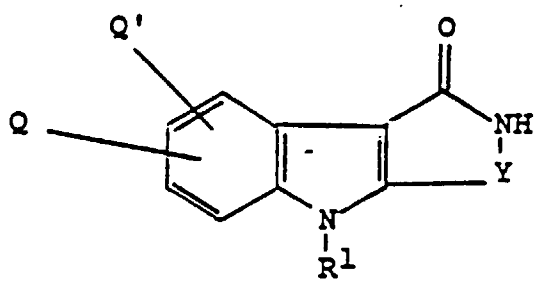

(II)

avec un composé de la formule (III)

X-Im (III)

ou un dérivé protégé de celui-ci, où X représente un radical $-CH_2L$ et L représente un groupe ou atome labile ou sortant et on réalise la réaction en présence d'une base; ou bien X représente le radical $-CH_2OH$ et on réalise la réaction en présence d'un acide, à température élevée, réaction suivie, si cela se révèle nécessaire, de l'élimination de n'importe quels radicaux protecteurs; ou bien

(B) pour la préparation d'un composé de la formule (I) dans laquelle Y représente $(CH_2)_n$, on cyclise un composé de la formule (IV)

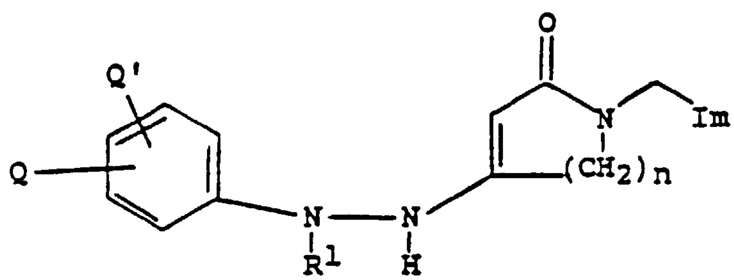

(IV) .

ou un sel ou dérivé protégé de celui-ci, cyclisation suivie, si cela se révèle nécessaire, de l'élimination de n'importe quels radicaux protecteurs présents; ou bien

(C) pour la préparation d'un composé de la formule (I) dans laquelle $R^3$ représente un atome d'hydrogène, on fait réagir un composé de la formule (V)

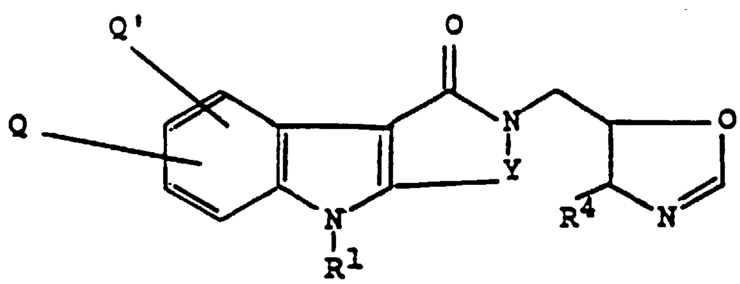

(V)

ou un dérivé protégé de celui-ci, avec le formamide, réaction suivie, si cela se révèle nécessaire, de l'élimination de n'importe quels radicaux protecteurs présents; ou bien

(D) pour la préparation d'un composé de la formule (I) dans laquelle Y représente $(CH_2)_n$, on fait réagir un composé de la formule (VI)

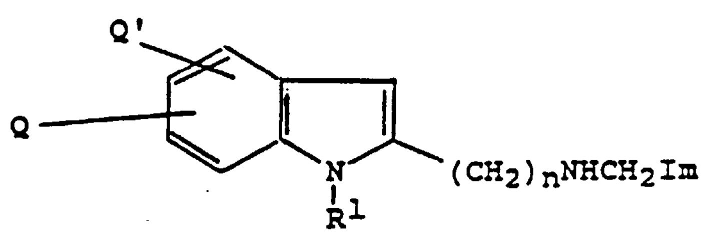

(VI)

ou un dérivé protégé de celui-ci, avec le phosgène, en présence d'un acide de Lewis, réaction suivie, si cela

se révèle nécessaire, de l'élimination de n'importe quels radicaux protecteurs présents; ou bien
(E) pour la préparation d'un composé de la formule (I) dans laquelle Y représente $(CH_2)_n$, on oxyde un composé de la formule (VII)

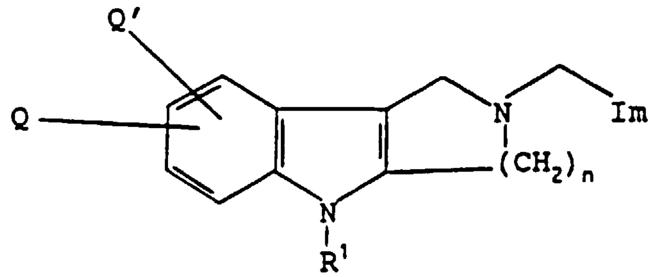

(VII)

ou un dérivé protégé de celui-ci, oxydation suivie, si cela se révèle nécessaire, de l'élimination de n'importe quels radicaux protecteurs présents; ou bien
(F) on convertit un composé de la formule générale (I) en un autre composé de la formule (I) par hydrogénation, alkylation ou acylation; ou bien
(G) on élimine le ou les groupes protecteurs d'une forme protégée d'un composé de la formule (I);

et lorsque le composé de la formule (I) est obtenu sous la forme d'un mélange d'énantiomères, on résout éventuellement le mélange de manière à obtenir l'énantiomère souhaité;
et/ou lorsque le composé de la formule (I) se présente sous la forme d'une base libre, on convertit éventuellement la base libre en un sel.

**2.** Procédé suivant la revendication 1 pour la préparation de composés dans lesquels $R^1$ représente un atome d'hydrogène ou un radical alkyle en $C_1$ à $C_6$, alcynyle en $C_3$ à $C_4$, cycloalkyl($C_4$-$C_6$)méthyle, ou alkylsulfonyle en $C_1$ à $C_3$.

**3.** Procédé suivant la revendication 1 ou 2 pour la préparation de composés dans lesquels $R^2$, $R^3$ et $R^4$ représentent chacun indépendamment un atome d'hydrogène ou un radical alkyle en $C_1$ à $C_4$.

**4.** Procédé suivant l'une quelconque des revendications 1 à 3 pour la préparation de composés dans lesquels Q représente un atome d'halogène ou un radical hydroxyle, alcoxy en $C_1$ à $C_4$, phénylalcoxy($C_1$-$C_3$), alkyle en $C_1$ à $C_6$, cyano, phényle, $-CONH_2$, ou $-(CH_2)_2CO_2CH_3$.

**5.** Procédé suivant l'une quelconque des revendications 1 à 3 pour la préparation de composés dans lesquels Q représente un atome d'halogène, ou un radical hydroxyle, phénylalcoxy($C_1$-$C_3$), alkyle en $C_1$ à $C_3$, ou cyano.

**6.** Procédé suivant l'une quelconque des revendications 1 à 3 pour la préparation de composés dans lesquels Q représente un atome de fluor.

**7.** Procédé suivant la revendication 1 pour la préparation de composés dans lesquels $R^1$ représente un atome d'hydrogène, ou un radical alkyle en $C_1$ à $C_4$, alcynyle en $C_3$ à $C_4$, ou cycloalkyl($C_4$-$C_6$)méthyle; $R^2$ et $R^3$ représentent chacun un atome d'hydrogène; $R^4$ représente un radical alkyle en $C_1$ à $C_4$; et Q représente un atome d'halogène ou un radical hydroxyle, phénylalcoxy($C_1$-$C_3$), alkyle en $C_1$ à $C_3$, ou cyano.

**8.** Procédé suivant l'une quelconque des revendications 1 à 7 pour la préparation de composés dans lesquels Y représente le radical $(CH_2)_2$ et Q' représente un atome d'hydrogène.

**9.** Procédé suivant la revendication 1 pour la préparation du composé ci-dessous :
6-fluoro-2,3,4,5-tétrahydro-5-méthyl-2-[(5-méthyl-1H-imidazole-4-yl)méthyl]-1H-pyrido[4,3-b]indole-1-one et ses solvates et sels physiologiquement acceptables.

**10.** Procédé suivant la revendication 1 pour la préparation d'un, composé choisi parmi les suivants : 2,3,4,5-tétrahydro-5,6-diméthyl-2-[(5-méthyl-1H-imidazole-4-yl)méthyl]-1H-pyrido[4,3-b]indole-1-one; 6,9-difluoro-2,3,4,5-tétrahydro-5-méthyl-2-[(5-méthyl-1H-imidazole-4-yl)méthyl]-1H-pyrido[4,3-b]indole-1-one; 6-fluoro-2,3,4,5-tétrahydro-2-[(5-méthyl-1H-imidazole-4-yl)méthyl]-5-(2-propynyl)-1H-pyrido[4,3-b]indole-1-one; 2,3,4,5-tétrahydro-5-méthyl-2-[(5-méthyl-1H-imidazole-4-yl)méthyl]-1-oxo-1H-pyrido[4,3-b]indole-6-carbonitrile; et de leurs solvates et sels physiologiquement acceptables.